# EUROPEAN PATENT SPECIFICATION

(11) **EP 3 529 269 B1**
(45) Date of publication and mention of the grant of the patent: **26.11.2025**
(21) Application number: 17804323.8
(22) Date of filing: 18.10.2017
(51) Int. Cl.: C07K 16/00

(54) **ANTIBODY CONSTRUCTS**
ANTIKÖRPER KONSTRUKTE
CONSTRUCTIONS D'ANTICORPS

(30) Priority: 19.10.2016 US 201662410054 P; 24.08.2017 US 201762549894 P; 07.09.2017 US 201762555498 P
(43) Date of publication of application: 28.08.2019
(73) Proprietor: Invenra Inc., Madison, WI 53719 (US)
(72) Inventor: BAILEY, Lucas, Madison, WI 53719 (US); GLASER, Bryan, Madison, WI 53719 (US); LI, Qufei, Madison, WI 53719 (US); GREEN, Roland, Madison, WI 53719 (US)
(74) Representative: Mewburn Ellis LLP
(86) International application number: PCT/US2017/057268
(87) International publication number: WO 2018/075692

(56) References cited:
- WO-A1-2013/157953
- WO-A1-2016/087650
- W. SCHAEFER ET AL: "Immunoglobulin domain crossover as a generic approach for the production of bispecific IgG antibodies", PROCEEDINGS OF THE NATIONAL ACADEMY OF SCIENCES, vol. 108, no. 27, 5 July 2011 (2011-07-05), pages 11187 - 11192, XP055003817, ISSN: 0027-8424, DOI: 10.1073/pnas.1019002108
- SPIESS CHRISTOPH ET AL: "Alternative molecular formats and therapeutic applications for bispecific antibodies", MOLECULAR IMMUNOLOGY, PERGAMON, GB, vol. 67, no. 2, 27 January 2015 (2015-01-27), pages 95 - 106, XP029246892, ISSN: 0161-5890, DOI: 10.1016/J.MOLIMM.2015.01.003
- SWINDELLS MARK B ET AL: "abYsis: Integrated Antibody Sequence and Structure-Management, Analysis, and Prediction", JOURNAL OF MOLECULAR BIOLOGY, ACADEMIC PRESS, UNITED KINGDOM, vol. 429, no. 3, 22 August 2016 (2016-08-22), pages 356 - 364, XP029894173, ISSN: 0022-2836, DOI: 10.1016/J.JMB.2016.08.019

## Description

### 1. BACKGROUND

Antibodies are an invaluable tool in the medical field. In particular, the importance of monoclonal antibodies, including their roles in scientific research and medical diagnostics, have been widely recognized for several decades. However, the full potential of antibodies, especially their successful use as therapeutic agents, has only more recently been demonstrated, as demonstrated by the successful therapies adalimumab (Humira), rituximab (Rituxan), infliximab (Remicade), bevacizumab (Avastin), trastuzumab (Herceptin), pembrolizumab (Keytruda), and ipilimumab (Yervoy). Following these clinical successes, interest in antibody therapies will likely only continue to increase. Therefore, a need for efficient generation and manufacturing of antibodies exists in the field, both in the research drug development and downstream clinical settings.

An area of active research in the antibody therapeutic field is the design and use of multispecific antibodies, *i.e.,* a single antibody engineered to recognize multiple targets. These antibodies offer the promise of greater therapeutic control. For example, a need exists to improve target specificity in order to reduce the off-target effects associated with many antibody therapies, particularly in the case of antibody-based immunotherapies. In addition, multispecific antibodies offer new therapeutic strategies, such as synergistic targeting of multiple cell receptors, especially in an immunotherapy context.

WO 2016/087650 describes multispecific antibodies, particularly domain-exchanged antibodies comprising a light chain (LC) composed of VL-CH3, and a heavy chain (HC) comprising VH-CH3-CH2- CH3, wherein the VL-CH3 of the LC is dimerizing with the VH-CH3 of the HC thereby forming a domain-exchanged LC/HC dimer comprising a CH3LC/CH3HC domain pair, and means and method for producing the same.

Despite the promise of multispecific antibodies, their production and use has been plagued by numerous constraints that have limited their practical implementation. In general, all multispecific antibody platforms must solve the problem of ensuring high fidelity pairing between cognate heavy and light chain pairs. However, a multitude of issues exist across the various platforms. For example, antibody chain engineering can result in poor stability of assembled antibodies, poor expression and folding of the antibody chains, and/or generation of immunogenic peptides. Other approaches suffer from impractical manufacturing processes, such as complicated *in vitro* assembly reactions or purification methods. In addition, several platforms suffer from the inability to easily and efficiently plug in different antibody binding domains. These various problems associated with multispecific antibody manufacturing limit the applicability of many platforms, especially their use in high-throughput screens necessary for many therapeutic drug pipelines.

There is, therefore, a need for an antibody platform capable of high-level expression and efficient purification. In particular, there is a need for a multispecific antibody platform that improves the manufacturing capabilities of multispecific antibodies with direct applicability in both research and therapeutic settings.

### 2. SUMMARY

We have designed a variety of novel multivalent antibody constructs. The architecture of these multivalent binding molecules drives high fidelity pairing of the cognate polypeptide chains that together form the antigen binding sites of monospecific, bispecific, trispecific, and tetraspecific constructs. The binding molecules are readily expressed using conventional antibody expression systems, including *in vitro* cell-free translation systems and mammalian transient transfection systems, and can be purified in a single-step with CH1 affinity resins. High fidelity assembly, high level *in vitro* expression, and the ability to purify expression products in a single step make these constructs well-suited to high throughput screening of variable region libraries. These constructs also demonstrate long-term stability, making them well suited as multispecific therapeutic agents.

Accordingly, the present invention relates to an antibody construct that comprises a first and a second polypeptide chain, wherein: (a) the first polypeptide chain comprises a domain A, a domain B, a domain D, and a domain E, wherein the domains are arranged, from N-terminus to C-terminus, in a A-B-D-E orientation, and wherein domain A has a VL amino acid sequence, domain B has a CH3 amino acid sequence, domain D has a CH2 amino acid sequence, domain E has a CH3 amino acid sequence; (b) the second polypeptide chain comprises a domain F and a domain G, wherein the domains are arranged, from N-terminus to C-terminus, in a F-G orientation, and wherein domain F has a VH amino acid sequence and domain G has a CH3 amino acid sequence; and (c) the first and the second polypeptides are associated through an interaction between the A and the F domains and an interaction between the B and the G domains to form the antibody construct; wherein the antibody construct further comprises a third and a fourth polypeptide chain, wherein: (d) the third polypeptide chain comprises a domain H, a domain I, a domain J, and a domain K, wherein the domains are arranged, from N-terminus to C-terminus, in a H-I-J-K orientation, and wherein domain H has a VL amino acid sequence, domain I has a CL amino acid sequence, domain J has a CH2 amino acid sequence, and K has a CH3 amino acid sequence; (e) the fourth polypeptide chain comprises a domain L and a domain M, wherein the domains are arranged, from N-terminus to C-terminus, in a L-M orientation, and wherein domain L has a VH amino acid sequence and domain M has a CH1 amino acid sequence; (f) the third and the fourth polypeptides are associated through an interaction between the H and the L domains and an interaction between the I and the M domains; and (g) the first and the third polypeptides are associated through an interaction between the D and the J domains and an interaction between the E and the K domains to form the binding molecule.

In certain aspects, the amino acid sequences of the B and the G domains are identical, wherein the sequence is an endogenous CH3 sequence.

In certain aspects, the amino acid sequences of the B and the G domains are different and separately comprise respectively orthogonal modifications in an endogenous CH3 sequence, wherein the B domain interacts with the G domain, and wherein neither the B domain nor the G domain significantly interacts with a CH3 domain lacking the orthogonal modification. In certain aspects, the orthogonal modifications comprise mutations that generate engineered disulfide bridges between domain B and G. In certain aspects, the mutations that generate engineered disulfide bridges are a S354C mutation in one of the B domain and G domain, and a 349C in the other domain. In certain aspects, the orthogonal modifications comprise knob-in-hole mutations. In certain aspects, the knob-in hole mutations are a T366W mutation in one of the B domain and G domain, and a T366S, L368A, and aY407V mutation in the other domain. In certain aspects, the orthogonal modifications comprise charge-pair mutations. In certain aspects, the charge-pair mutations are a T366K mutation in one of the B domain and G domain, and a L351D mutation in the other domain.

In certain aspects, the amino acid sequences of the E and K domains are identical, wherein the sequence is an endogenous CH3 sequence.

In certain aspects, the amino acid sequences of the E and K domains are different. In certain aspects, the different sequences separately comprise respectively orthogonal modifications in an endogenous CH3 sequence, wherein the E domain interacts with the K domain, and wherein neither the E domain nor the K domain significantly interacts with a CH3 domain lacking the orthogonal modification. In certain aspects, the orthogonal modifications comprise mutations that generate engineered disulfide bridges between domain E and K. In certain aspects, the mutations that generate engineered disulfide bridges are a S354C mutation in one of the E domain and K domain, and a 349C in the other domain. In certain aspects, the orthogonal modifications in the E and K domains comprise knob-in-hole mutations. In certain aspects, the knob-in hole mutations are a T366W mutation in one of the E domain or K domain and a T366S, L368A, and aY407V mutation in the other domain. In certain aspects, the orthogonal modifications comprise charge-pair mutations. In certain aspects, the charge-pair mutations are a T366K mutation in one of the E domain or K domain and a corresponding L351D mutation in the other domain.

In certain aspects, the interaction between the A domain and the F domain form a first antigen binding site specific for a first antigen, and the interaction between the H domain and the L domain form a second antigen binding site specific for a second antigen.

In certain aspects, the binding molecule further comprises a fifth polypeptide chain, wherein: (a) the first polypeptide chain further comprises a domain N and a domain O, wherein the domains are arranged, from N-terminus to C-terminus, in a N-O-A-B-D-E orientation, and wherein domain N has a VL amino acid sequence, domain O has a CH3 amino acid sequence; (b) the binding molecule further comprises a fifth polypeptide chain, comprising: a domain P and a domain Q, wherein the domains are arranged, from N-terminus to C-terminus, in a P-Q orientation, and wherein domain P has a VH amino acid sequence and domain Q has a CH3 amino acid sequence; and (c) the first and the fifth polypeptides are associated through an interaction between the N and the P domains and an interaction between the O and the Q domains to form the binding molecule.

In certain aspects, (a) the amino acid sequences of domain N and domain A are identical, the amino acid sequences of domain H is different from domains N and A, the amino acid sequences of domain O and domain B are identical, the amino acid sequences of domain I is different from domains O and B, the amino acid sequences of domain P and domain F are identical, the amino acid sequences of domain L is different from domains P and F, the amino acid sequences of domain Q and domain G are identical, the amino acid sequences of domain M is different from domains Q and G; and (b) wherein the interaction between the A domain and the F domain form a first antigen binding site specific for a first antigen, the interaction between the H domain and the L domain form a second antigen binding site specific for a second antigen, and the domain N and domain P form a third antigen binding site specific for the first antigen.

In certain aspects, (a) the amino acid sequences of domain N, domain A, and domain H are different, the amino acid sequences of domain O, domain B, and domain I are different, the amino acid sequences of domain P, domain F, and domain L are different, and the amino acid sequences of domain Q, domain G, and domain M are different; and (b) the interaction between the A domain and the F domain form a first antigen binding site specific for a first antigen, the interaction between the H domain and the L domain form a second antigen binding site specific for a second antigen, and the domain N and domain P form a third antigen binding site specific for a third antigen.

In certain aspects, the binding molecule further comprises a sixth polypeptide chain, wherein: (a) the third polypeptide chain further comprises a domain R and a domain S, wherein the domains are arranged, from N-terminus to C-terminus, in a R-S-H-I-J-K orientation, and wherein domain R has a VL amino acid sequence and domain S has a constant domain amino acid sequence; (b) the binding molecule further comprises a sixth polypeptide chain, comprising: a domain T and a domain U, wherein the domains are arranged, from N-terminus to C-terminus, in a T-U orientation, and wherein domain T has a VH amino acid sequence and domain U has a constant domain amino acid sequence; and (c) the third and the sixth polypeptides are associated through an interaction between the R and the T domains and an interaction between the S and the U domains to form the binding molecule.

In certain aspects, (a) the amino acid sequences of domain R and domain A are identical, the amino acid sequences of domain H is different from domain R and A, the amino acid sequences of domain S and domain B are identical, the amino acid sequences of domain I is different from domain S and B, the amino acid sequences of domain T and domain F are identical, the amino acid sequences of domain L is different from domain T and F, the amino acid sequences of domain U and domain G are identical, the amino acid sequences of domain M is different from domain U and G and (b) the interaction between the A domain and the F domain form a first antigen binding site specific for a first antigen, the interaction between the H domain and the L domain form a second antigen binding site specific for a second antigen, and the domain R and domain T form a third antigen binding site specific for the first antigen.

In certain aspects, (a) the amino acid sequences of domain R and domain H are identical, the amino acid sequences of domain A is different from domain R and H, the amino acid sequences of domain S and domain I are identical, the amino acid sequences of domain B is different from domain S and I, the amino acid sequences of domain T and domain L are identical, the amino acid sequences of domain F is different from domain T and L, the amino acid sequences of domain U and domain M are identical, the amino acid sequences of domain G is different from domain U and M and (b) the interaction between the A domain and the F domain form a first antigen binding site specific for a first antigen, the interaction between the H domain and the L domain form a second antigen binding site specific for a second antigen, and the domain R and domain T form a third antigen binding site specific for the second antigen.

In certain aspects, (a) the amino acid sequences of domain R, domain A, and domain H are different, the amino acid sequences of domain S, domain B, and domain I are different, the amino acid sequences of domain T, domain F, and domain L are different, and the amino acid sequences of domain U, domain G, and domain M are different; and (b) the interaction between the A domain and the F domain form a first antigen binding site specific for a first antigen, the interaction between the H domain and the L domain form a second antigen binding site specific for a second antigen, and the domain R and domain T form a third antigen binding site specific for a third antigen.

In certain aspects, the binding molecule further comprises a fifth and a sixth polypeptide chain, wherein: (a) the first polypeptide chain further comprises a domain N and a domain O, wherein the domains are arranged, from N-terminus to C-terminus, in a N-O-A-B-D-E orientation; (b) the third polypeptide chain further comprises a domain R and a domain S, wherein the domains are arranged, from N-terminus to C-terminus, in a R-S-H-I-J-K orientation; (c) the binding molecule further comprises a fifth and a sixth polypeptide chain, wherein: the fifth polypeptide chain comprises a domain P and a domain Q, wherein the domains are arranged, from N-terminus to C-terminus, in a P-Q orientation, and the sixth polypeptide chain comprises a domain T and a domain U, wherein the domains are arranged, from N-terminus to C-terminus, in a T-U orientation; and (d) the first and the fifth polypeptides are associated through an interaction between the N and the P domains and an interaction between the O and the Q domains, and the third and the sixth polypeptides are associated through an interaction between the R and the T domains and an interaction between the S and the U domains to form the binding molecule.

In certain aspects, (a) the amino acid sequences of domain N and domain A are identical, the amino acid sequences of domain H and domain R are identical, the amino acid sequences of domain O and domain B are identical, the amino acid sequences of domain I and domain S are identical, the amino acid sequences of domain P and domain F are identical, the amino acid sequences of domain L and domain T are identical, the amino acid sequences of domain Q and domain G are identical, the amino acid sequences of domain M and domain U are identical; and (b) wherein the interaction between the A domain and the F domain form a first antigen binding site specific for a first antigen, the domain N and domain P form a second antigen binding site specific for the first antigen, the interaction between the H domain and the L domain form a third antigen binding site specific for a second antigen, and the interaction between the R domain and the T domain form a fourth antigen binding site specific for the second antigen.

In certain aspects, the sequence that forms a junction between the A domain and the B domain is IKRTPREP or IKRTVREP. In certain aspects, the sequence that forms a junction between the F domain and the G domain is SSASPREP.

In certain aspects, at least one CH3 amino acid sequence has a C-terminal tripeptide insertion connecting the CH3 amino acid sequence and a hinge amino acid sequence, wherein the tripeptide insertion is selected from the group consisting of PGK, KSC, and GEC.

In certain aspects, the sequences are human sequences. In certain aspects, at least one CH3 amino acid sequence is an IgG sequence. In certain aspects, the IgG sequences are IgG1 sequences. In certain aspects, at least one CH3 amino acid sequence has one or more isoallotype mutations. In certain aspects, the isoallotype mutations are D356E and L358M. In certain aspects, the CL amino acid sequence is a Cₖₐₚₚₐ sequence.

Also disclosed herein (but not claimed) is a pharmaceutical composition, comprising any of the binding molecules disclosed or described herein, and a pharmaceutically acceptable carrier.

Also disclosed herein (but not claimed) is a method of treatment, comprising administering to a subject in need of treatment the pharmaceutical composition comprising any of the binding molecules disclosed or described herein and any of the pharmaceutically acceptable carriers disclosed or described herein.

### 3. BRIEF DESCRIPTION OF THE DRAWINGS

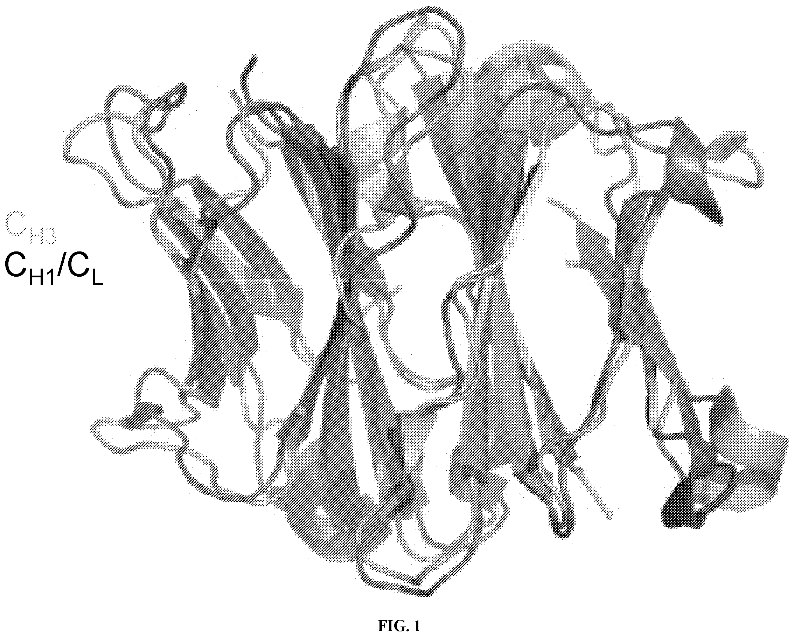

**FIG. 1** shows an alignment of the CH3-CH3 IgG1 dimer pair with CH1-C_{L}. The quaternary structures align with an RMSD of ~1.6 Å².
**FIG. 2** presents schematic architectures, with respective naming conventions, for various binding molecules (also called antibody constructs) described herein.
**FIG. 3** presents a higher resolution schematic of polypeptide chains and their domains, with respective naming conventions, for the bivalent 1x1 antibody constructs described herein.
**FIG. 4** shows the architecture of an exemplary bivalent, monospecific, construct.

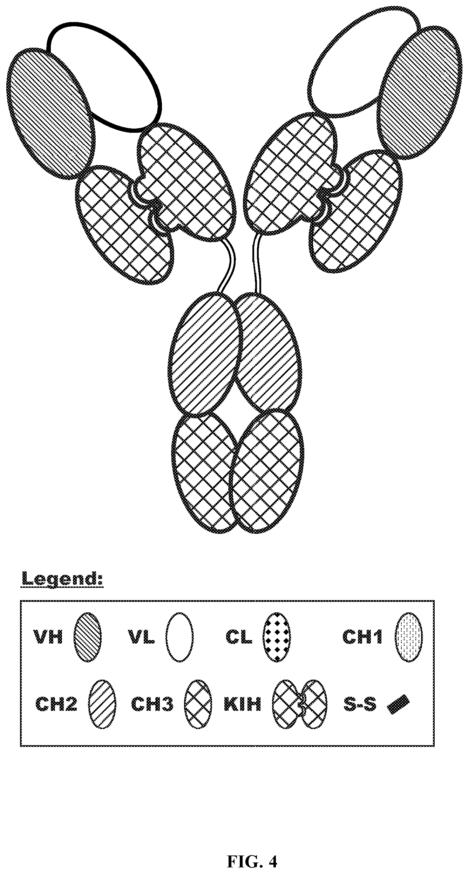

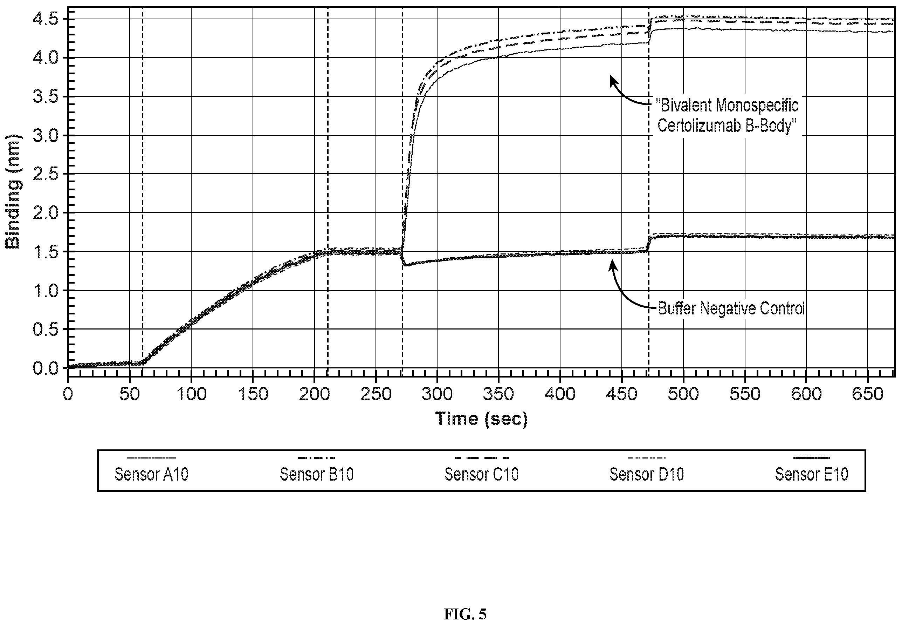

**FIG. 5** shows data from a biolayer interferometry (BLI) experiment, described in Example 1, in which a bivalent monospecific binding molecule having the architecture illustrated in FIG. 4 [polypeptide 1: VL-CH3(Knob)-CH2-CH3 / polypeptide 2: VH-CH3(Hole)] was assayed. The antigen binding site was specific for TNFα. The BLI response from binding molecule immobilization and TNFα binding to the immobilized construct demonstrates robust, specific, bivalent binding to the antigen. The data are consistent with a molecule having a high percentage of intended pairing of polypeptide 1 and polypeptide 2.
**FIG. 6** illustrates features of an exemplary bivalent 1x1 bispecific binding molecule, "BC1".
**FIG. 7A** shows size exclusion chromatography (SEC) analysis of "BC1", demonstrating that a single-step CH1 affinity purification step (CaptureSelect^{™} CH1 affinity resin) yields a single, monodisperse peak via gel filtration in which >98% is unaggregated bivalent protein. **FIG. 7B** shows comparative literature data of SEC analysis of a CrossMab bivalent antibody construct [data from Schaefer et al. (Proc Natl Acad Sci USA. 2011 Jul 5;108(27):11187-92)].
**FIG. 8A** is a cation exchange chromatography elution profile of "BC1" following one-step purification using the CaptureSelect^{™} CH1 affinity resin, showing a single tight peak. **FIG. 8B** is a cation exchange chromatography elution profile of "BC1" following purification using standard Protein A purification.
**FIG. 9** shows nonreducing SDS-PAGE gels of "BC1" at various stages of purification.
**FIGS. 10A** and **10B** compare SDS-PAGE gels of "BC1" after single-step CH1-affinity purification under both non-reducing and reducing conditions (FIG. 10A) with SDS-PAGE gels of a CrossMab bispecific antibody under non-reducing and reducing conditions as published in the referenced literature (FIG. 10B).
**FIGS. 11A** and **11B** show mass spec analysis of "BC1", demonstrating two distinct heavy chains (FIG. 11A) and two distinct light chains (FIG. 11B) under reducing conditions.
**FIG. 12** presents a mass spectrometry analysis of purified "BC1" under non-reducing conditions, confirming the absence of incomplete pairing after purification.
**FIG. 13** presents accelerated stability testing data demonstrating stability of "BC1" over 8 weeks at 40°C, compared to two IgG control antibodies.
**FIG. 14** illustrates features of an exemplary bivalent 1x1 bispecific binding molecule, "BC6", further described in Example 3.
**FIG. 15A** presents size exclusion chromatography (SEC) analysis of "BC6" following one-step purification using the CaptureSelect^{™} CH1 affinity resin, demonstrating that the single step CH1 affinity purification yields a single monodisperse peak and the absence of non-covalent aggregates. **FIG. 15B** shows an SDS-PAGE gel of "BC6" under non-reducing conditions.
**FIG. 16** illustrates features of an exemplary bivalent bispecific binding molecule, "BC28", further described in Example 4.
**FIG. 17** shows SDS-PAGE analysis under non-reducing conditions following single-step CH1 affinity purification of "BC28", "BC29", "BC30", "BC31", and "BC32".
**FIG. 18** shows SEC analysis of "BC28" and "BC30", each following one-step purification using the CaptureSelect^{™} CH1 affinity resin.
**FIG. 19** illustrates features of an exemplary bivalent bispecific binding molecule, "BC44", further described in Example 5.
**FIGS. 20A and 20B** show size exclusion chromatography data of two bivalent binding molecules, "BC15" and "BC16", respectively, under accelerated stability testing conditions. "BC15" and "BC16" have different variable region-CH3 junctions.
**FIG. 21** presents a schematic of polypeptide chains and their domains, with respective naming conventions, for the trivalent 2x1 antibody constructs described herein.
**FIG. 22** illustrates features of an exemplary trivalent 2x1 bispecific binding molecule, "BC1-2x1", further described in Example 7.
**FIG. 23** shows non-reducing SDS-PAGE of "BC1" and "BC1-2x1" protein expressed using the ThermoFisher Expi293 transient transfection system, at various stages of purification.
**FIG. 24** compares the avidity of the bivalent 1x1 construct "BC1" to the avidity of the trivalent 2x1 construct "BC1-2x1" using an Octet (Pall ForteBio) biolayer interferometry analysis.
**FIG. 25** illustrates salient features of a trivalent 2x1 construct, "TB 111."
**FIG. 26** presents a schematic of polypeptide chains and their domains, with respective naming conventions, for the trivalent 1x2 antibody constructs described herein.
**FIG. 27** illustrates features of an exemplary trivalent 1x2 construct "CTLA4-4 x Nivo x CTLA4-4", further described in Example 10.
**FIG. 28** is an SDS-PAGE gel in which the lanes showing the trivalent 1x2 construct "CTLA4-4 x Nivo x CTLA4-4" construct under non-reducing ("-DTT") and reducing ("+DTT") conditions have been boxed.
**FIG. 29** shows a comparison of antigen binding between two antibodies: bivalent 1x1 construct "CTLA4-4 x OX40-8" and the trivalent 1x2 construct "CTLA4-4 x Nivo x CTLA4-4." "CTLA4-4 x OX40-8" binds to CTLA4 monovalently, while "CTLA4-4 x Nivo x CTLA4-4" binds to CTLA4 bivalently.
**FIG. 30** illustrates features of an exemplary trivalent 1x2 trispecific construct, "BC28-1x1x1a", further described in Example 11.
**FIG. 31** shows size exclusion chromatography of "BC28-1x1x1a" following transient expression and single step CH1 affinity resin purification, demonstrating a single well-defined peak.
**FIG. 32** shows SDS-PAGE results with bivalent and trivalent constructs, each after transient expression and one-step purification using the CaptureSelect^{™} CH1 affinity resin, under non-reducing and reducing conditions, as further described in Example 12.
**FIGS. 33A-33C** show Octet binding analyses to 3 antigens: PD1, Antigen "A", and CTLA4. As further described in Example 13, **FIG. 33A** shows binding of "BC1" to PD1 and Antigen "A"; **FIG. 33B** shows binding of a bivalent bispecific construct "CTLA4-4 x OX40-8" to CTLA4, Antigen "A", and PD1; **FIG. 33C** shows binding of trivalent trispecific "BC28-1x1x1a" to PD1, Antigen "A", and CTLA4.
**FIG. 34** presents a schematic of polypeptide chains and their domains, with respective naming conventions, for certain tetravalent 2x2 constructs described herein.
**FIG. 35** illustrates certain salient features of the exemplary tetravalent 2x2 construct, "BC22-2x2" further described in Example 14.
**FIG. 36** is a non-reducing SDS-PAGE gel comparing the 2x2 tetravalent "BC22-2x2" construct to a 1x2 trivalent construct "BC12-1x2" and a 2x1 trivalent construct "BC21-2x1" at different stages of purification.
**FIG. 37** provides architecture for an exemplary tetravalent 2x2 construct.
**FIG. 38** presents a schematic of polypeptide chains and their domains, with respective naming conventions, for certain tetravalent constructs described herein.
**FIG. 39** provides exemplary architecture of a bispecific tetravalent construct.
**FIG. 40** provides exemplary architecture for a trispecific tetravalent construct utilizing a common light chain strategy.
**FIG. 41** shows bispecific antigen engagement by the tetravalent construct schematized in FIG. 39, demonstrating that this construct was capable of simultaneous engagement. The biolayer interferometry (BLI) response from B-Body immobilization and TNFα binding to the immobilized construct are consistent with a molecule with a high percentage of intended chain pairing.
**FIG. 42** provides flow cytometry analysis of B-Body binding to cell-surface antigen. Cross-hatched signal indicates cells without antigen; dotted signal indicates transiently transfected cells with surface antigen.

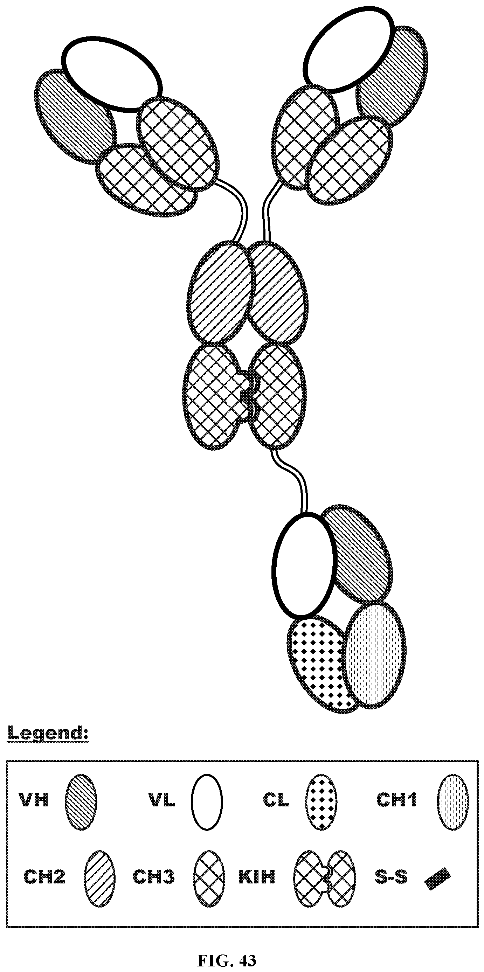

**FIG. 43** provides exemplary architecture of a trivalent construct.

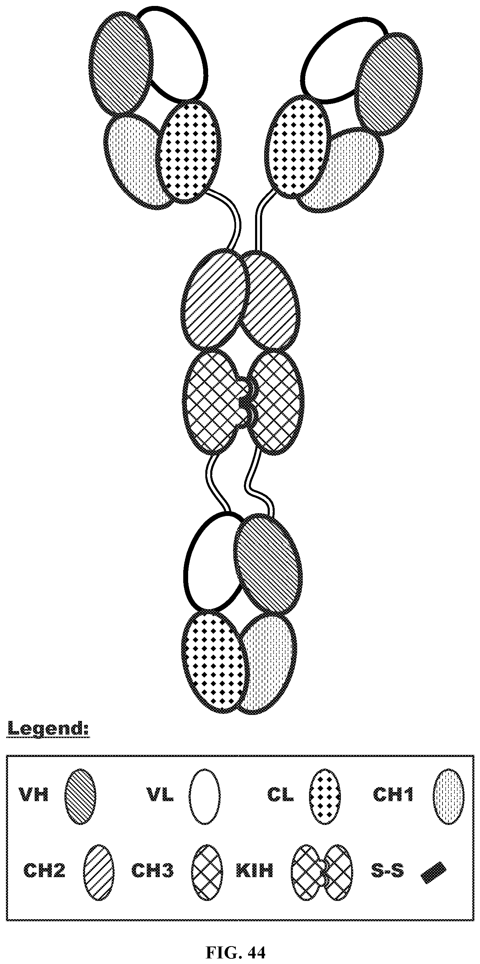

**FIG. 44** provides exemplary architecture of a trivalent construct.
**FIG. 45** shows SDS-PAGE results with bivalent and trivalent constructs, each after transient expression and one-step purification using the CaptureSelect^{™} CH1 affinity resin, under non-reducing and reducing conditions, as further described in Example 17.
**FIG. 46** shows differences in the thermal transitions for "BC24jv", "BC26jv", and "BC28jv" measured to assess pairing stability of junctional variants.

The figures depict various embodiments of the present invention for purposes of illustration only. One skilled in the art will readily recognize from the following discussion that alternative embodiments of the structures and methods illustrated herein may be employed without departing from the principles of the invention described herein.

### 4. DETAILED DESCRIPTION

### 4.1. Definitions

Unless defined otherwise, all technical and scientific terms used herein have the meaning commonly understood by a person skilled in the art to which this invention belongs. As used herein, the following terms have the meanings ascribed to them below.

By **"antigen binding site"** is meant a region of a binding molecule that specifically recognizes or binds to a given antigen or epitope.

**"B-Body"** means any of the binding molecule constructs described herein.

As used herein, the terms **"treat"** or **"treatment"** refer to both therapeutic treatment and prophylactic or preventative measures, wherein the object is to prevent or slow down (lessen) an undesired physiological change or disorder, such as the progression of multiple sclerosis, arthritis, or cancer. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

By **"subject"** or **"individual"** or **"animal"** or **"patient"** or **"mammal,"** is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, and so on.

The term **"sufficient amount"** means an amount sufficient to produce a desired effect, e.g., an amount sufficient to modulate protein aggregation in a cell.

The term **"therapeutically effective amount"** is an amount that is effective to ameliorate a symptom of a disease. A therapeutically effective amount can be a **"prophylactically effective amount"** as prophylaxis can be considered therapy.

### 4.2. Other interpretational conventions

Unless otherwise specified, all references to sequences herein are to amino acid sequences.

Unless otherwise specified, antibody constant region residue numbering is according to the Eu index as described at
www.imgt.org/IMGTScientificChart/Numbering/Hu_IGHGnber.html#refs (accessed Aug. 22, 2017) and in Edelman et al., Proc. Natl. Acad. USA, 63:78-85 (1969) and identifies the residue according to its location in an endogenous constant region sequence regardless of the residue's physical location within a chain of the binding molecules described herein. By "endogenous sequence" or "native sequence" is meant any sequence, including both nucleic acid and amino acid sequences, which originates from an organism, tissue, or cell and has not been artificially modified or mutated.

In this disclosure, "comprises," "comprising," "containing," "having," "includes," "including," and linguistic variants thereof have the meaning ascribed to them in U.S. Patent law, permitting the presence of additional components beyond those explicitly recited.

Ranges provided herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

Unless specifically stated or apparent from context, as used herein the term "or" is understood to be inclusive. Unless specifically stated or apparent from context, as used herein, the terms "a", "an", and "the" are understood to be singular or plural.

Unless specifically stated or otherwise apparent from context, as used herein the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

### 4.3. Binding molecules

In a first aspect, binding molecules are provided.

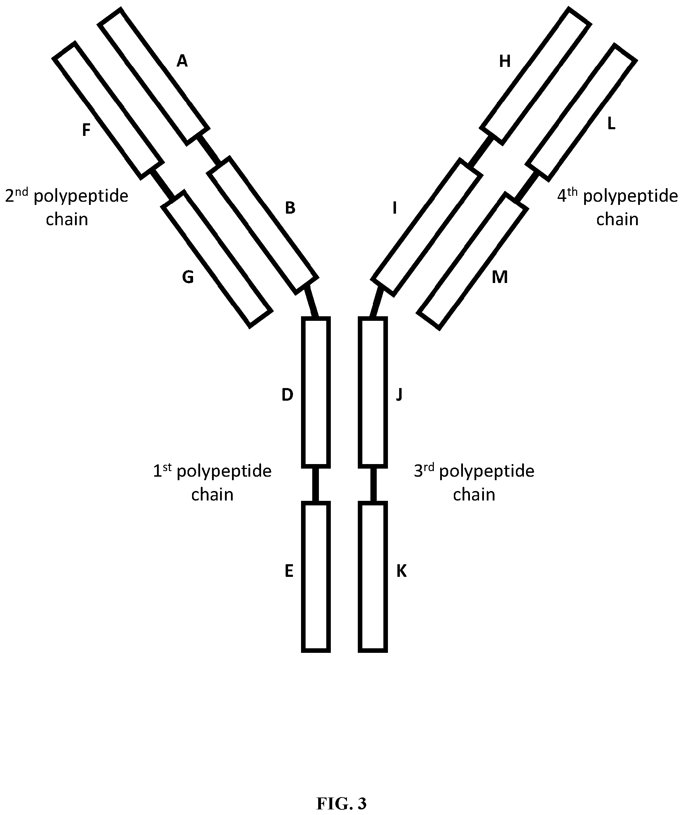

With reference to **FIG. 3****,** the binding molecules comprise a first and a second polypeptide chain, wherein: (a) the first polypeptide chain comprises a domain A, a domain B, a domain D, and a domain E, wherein the domains are arranged, from N-terminus to C-terminus, in a A-B-D-E orientation, and wherein domain A has a VL amino acid sequence, domain B has a CH3 amino acid sequence, domain D has a CH2 amino acid sequence, and domain E has a constant region domain CH3 amino acid sequence; (b) the second polypeptide chain comprises a domain F and a domain G, wherein the domains are arranged, from N-terminus to C-terminus, in a F-G orientation, and wherein domain F has a VH amino acid sequence and domain G has a CH3 amino acid sequence; and (c) the first and the second polypeptides are associated through an interaction between the A and the F domains and an interaction between the B and the G domains to form the binding molecule; and the binding molecules further comprise a third and a fourth polypeptide chain, wherein: (a) the third polypeptide chain comprises a domain H, a domain I, a domain J, and a domain K, wherein the domains are arranged, from N-terminus to C-terminus, in a H-I-J-K orientation, and wherein domain H has a VL amino acid sequence, domain I has a CL amino acid sequence, domain J has a CH2 amino acid sequence, and K has a CH3 amino acid sequence; (b) the fourth polypeptide chain comprises a domain L and a domain M, wherein the domains are arranged, from N-terminus to C-terminus, in a L-M orientation, and wherein domain L has a VH amino acid sequence and domain M has a CH1 amino acid sequence; (c) the third and the fourth polypeptides are associated through an interaction between the H and the L domains and an interaction between the I and the M domains; and (d) the first and the third polypeptides are associated through an interaction between the D and the J domains and an interaction between the E and the K domains to form the binding molecule.

In various embodiments, the first and third polypeptide chains are non-identical in sequence to one another, and the second and fourth polypeptide are non-identical in sequence to one another. In these embodiments, association of the first and third polypeptide chains through interactions between domains E & K (see Section 4.3.15 below) is capable of forming a bivalent, bispecific, antibody construct.

### 4.3.1. Domain A (VL)

In the binding molecules described herein, domain A has a VL amino acid sequence. The VL amino acid sequences useful in the binding molecules described herein are antibody light chain variable domain sequences. In a typical arrangement in both natural antibodies and the antibody constructs described herein, a specific VL amino acid sequence associates with a specific VH amino acid sequence to form an antigen-binding site. In various embodiments, the VL amino acid sequences are mammalian sequences, including human sequences, synthesized sequences, or combinations of human, non-human mammalian, mammalian, and/or synthesized sequences, as described in further detail below in Sections 4.3.1.1 and 4.3.1.2.

In various embodiments, VL amino acid sequences are mutated sequences of naturally occurring sequences. In certain embodiments, the VL amino acid sequences are lambda (λ) light chain variable domain sequences. In certain embodiments, the VL amino acid sequences are kappa (κ) light chain variable domain sequences. In a preferred embodiment, the VL amino acid sequences are kappa (κ) light chain variable domain sequences.

In the binding molecules described herein, the C-terminus of domain A is connected to the N-terminus of domain B. In certain embodiments, domain A has a VL amino acid sequence that is mutated at its C-terminus at the junction between domain A and domain B, as described in greater detail below in Section 4.3.19.1 and in Example 6.

### 4.3.1.1. Complementarity Determining Regions

The VL amino acid sequences comprise highly variable sequences termed **"complementarity determining regions"** (CDRs), typically three CDRs (CDR1, CD2, and CDR3). In a variety of embodiments, the CDRs are mammalian sequences, including, but not limited to, mouse, rat, hamster, rabbit, camel, donkey, goat, and human sequences. In a preferred embodiment, the CDRs are human sequences. In various embodiments, the CDRs are naturally occurring sequences. In various embodiments, the CDRs are naturally occurring sequences that have been mutated to alter the binding affinity of the antigen-binding site for a particular antigen or epitope. In certain embodiments, the naturally occurring CDRs have been mutated in an *in vivo* host through affinity maturation and somatic hypermutation. In certain embodiments, the CDRs have been mutated *in vitro* through methods including, but not limited to, PCR-mutagenesis and chemical mutagenesis. In various embodiments, the CDRs are synthesized sequences including, but not limited to, CDRs obtained from random sequence CDR libraries and rationally designed CDR libraries.

### 4.3.1.2. Framework Regions and CDR Grafting

The VL amino acid sequences comprise **"framework region"** (FR) sequences. FRs are generally conserved sequence regions that act as a scaffold for interspersed CDRs (see Section 4.3.1.1.), typically in a FR1-CDR1-FR2-CDR2-FR3-CDR3-FR4 arrangement (from N-terminus to C-terminus). In a variety of embodiments, the FRs are mammalian sequences, including, but not limited to mouse, rat, hamster, rabbit, camel, donkey, goat, and human sequences. In a preferred embodiment, the FRs are human sequences. In various embodiments, the FRs are naturally occurring sequences. In various embodiments, the FRs are synthesized sequences including, but not limited, rationally designed sequences.

In a variety of embodiments, the FRs and the CDRs are both from the same naturally occurring variable domain sequence. In a variety of embodiments, the FRs and the CDRs are from different variable domain sequences, wherein the CDRs are grafted onto the FR scaffold with the CDRs providing specificity for a particular antigen. In certain embodiments, the grafted CDRs are all derived from the same naturally occurring variable domain sequence. In certain embodiments, the grafted CDRs are derived from different variable domain sequences. In certain embodiments, the grafted CDRs are synthesized sequences including, but not limited to, CDRs obtained from random sequence CDR libraries and rationally designed CDR libraries. In certain embodiments, the grafted CDRs and the FRs are from the same species. In certain embodiments, the grafted CDRs and the FRs are from different species. In a preferred grafted CDR embodiment, an antibody is **"humanized",** wherein the grafted CDRs are non-human mammalian sequences including, but not limited to, mouse, rat, hamster, rabbit, camel, donkey, and goat sequences, and the FRs are human sequences. Humanized antibodies are discussed in more detail in U.S. Pat. No. 6,407,213. In various embodiments, portions or specific sequences of FRs from one species are used to replace portions or specific sequences of another species' FRs.

### 4.3.2. Domain B (CH3)

In the binding molecules, domain B has a CH3 amino acid sequence. CH3 amino acid sequences, as described herein, are sequences of the C-terminal domain of an antibody heavy chain.

In a variety of embodiments, the CH3 sequences are mammalian sequences, including, but not limited to, mouse, rat, hamster, rabbit, camel, donkey, goat, and human sequences. In a preferred embodiment, the CH3 sequences are human sequences. In certain embodiments, the CH3 sequences are from an IgA1, IgA2, IgD, IgE, IgM, IgG1, IgG2, IgG3, IgG4 isotype or CH4 sequences from an IgE or IgM isotype. In a preferred embodiment, the CH3 sequences are from an IgG1 isotype.

In certain embodiments, the CH3 sequences are endogenous sequences. In particular embodiments, the CH3 sequence is UniProt accession number P01857 amino acids 224-330. In various embodiments, a CH3 sequence is a segment of an endogenous CH3 sequence. In particular embodiments, a CH3 sequence has an endogenous CH3 sequence that lacks the N-terminal amino acids G224 and Q225. In particular embodiments, a CH3 sequence has an endogenous CH3 sequence that lacks the C-terminal amino acids P328, G329, and K330. In particular embodiments, a CH3 sequence has an endogenous CH3 sequence that lacks both the N-terminal amino acids G224 and Q225 and the C-terminal amino acids P328, G329, and K330. In preferred embodiments, a binding molecule has multiple domains that have CH3 sequences, wherein a CH3 sequence can refer to both a full endogenous CH3 sequence as well as a CH3 sequence that lacks N-terminal amino acids, C-terminal amino acids, or both.

In certain embodiments, the CH3 sequences are endogenous sequences that have one or more mutations. In particular embodiments, the mutations are one or more orthogonal mutations that are introduced into an endogenous CH3 sequence to guide specific pairing of specific CH3 sequences, as described in more detail below in Sections 4.3.14.1-4.3.14.3.

In certain embodiments, the CH3 sequences are engineered to reduce immunogenicity of the antibody by replacing specific amino acids of one allotype with those of another allotype and referred to herein as isoallotype mutations, as described in more detail in Stickler et al. (Genes Immun. 2011 Apr; 12(3): 213-221). In particular embodiments, specific amino acids of the G1m1 allotype are replaced. In a preferred embodiment, isoallotype mutations D356E and L358M are made in the CH3 sequence.

In a preferred embodiment, domain B has a human IgG1 CH3 amino acid sequence with the following mutational changes: P343V; Y349C; and a tripeptide insertion, 445P, 446G, 447K. In other preferred embodiments, domain B has a human IgG1 CH3 sequence with the following mutational changes: T366K; and a tripeptide insertion, 445K, 446S, 447C. In still other preferred embodiments, domain B has a human IgG1 CH3 sequence with the following mutational changes: Y349C and a tripeptide insertion, 445P, 446G, 447K.

In certain embodiments, domain B has a human IgG1 CH3 sequence with a 447C mutation incorporated into an otherwise endogenous CH3 sequence.

In the binding molecules described herein, the N-terminus of domain B is connected to the C-terminus of domain A. In certain embodiments, domain B has a CH3 amino acid sequence that is mutated at its N-terminus at the junction between domain A and domain B, as described in greater detail below in Section 4.3.19.1 and Example 6.

In the binding molecules, the C-terminus of domain B is connected to the N-terminus of domain D. In certain embodiments, domain B has a CH3 amino acid sequence that is extended at the C-terminus at the junction between domain B and domain D, as described in greater detail below in Section 4.3.19.3.

### 4.3.3. Domain D (CH2)

In the binding molecules described herein, domain D has a CH2 amino acid sequence. CH2 amino acid sequences, as described herein, are CH2 amino acid sequences of the third domain of a native antibody heavy chain, with reference from the N-terminus to C-terminus. In a variety of embodiments, the CH2 sequences are mammalian sequences, including but not limited to mouse, rat, hamster, rabbit, camel, donkey, goat, and human sequences. In a preferred embodiment, the CH2 sequences are human sequences. In certain embodiments, the CH2 sequences are from a IgA1, IgA2, IgD, IgE, IgG1, IgG2, IgG3, IgG4, or IgM isotype. In a preferred embodiment, the CH2 sequences are from an IgG1 isotype.

In certain embodiments, the CH2 sequences are endogenous sequences. In particular embodiments, the sequence is UniProt accession number P01857 amino acids 111-223. In a preferred embodiment, the CH2 sequences have a N-terminal hinge region peptide that connects the N-terminal variable domain-constant domain segment to the CH2 domain, as discussed in more detail below in Section 4.3.19.3.

In the binding molecules, the N-terminus of domain D is connected to the C-terminus of domain B. In certain embodiments, domain B has a CH3 amino acid sequence that is extended at the C-terminus at the junction between domain D and domain B, as described in greater detail below in Section 4.3.19.3.

In the binding molecules, the C-terminus of domain D is connected to the N-terminus of domain E. In particular embodiments, domain D is connected to the N-terminus of domain E that has a CH1 amino acid sequence or CL amino acid sequence, as described in further detail below in Section 4.3.19.5.

### 4.3.4. Domain E (CH3)

In the binding molecules, domain E has a CH3 amino acid sequence. CH3 sequences are described in greater detail above in Section 4.3.2.

In particular embodiments, the CH3 sequence has been mutated to include one or more orthogonal mutations. In a preferred embodiment, domain E has a a CH3 sequence comprising knob-hole (synonymously, "knob-in-hole," "KIH") orthogonal mutations, as described in greater detail below in Section 4.3.14.2, and either a S354C or a Y349C mutation that forms an engineered disulfide bridge with a CH3 domain containing an orthogonal mutation, as described in in greater detail below in Section 4.3.14.1. In some preferred embodiments, the knob-hole orthogonal mutation is a T366W mutation.

### 4.3.4.1. CH1 Domains

CH1 amino acid sequences, as described herein, are sequences of the second domain of an antibody heavy chain, with reference from the N-terminus to C-terminus. In certain embodiments, the CH1 sequences are endogenous sequences. In a variety of embodiments, the CH1 sequences are mammalian sequences, including, but not limited to mouse, rat, hamster, rabbit, camel, donkey, goat, and human sequences. In a preferred embodiment, the CH1 sequences are human sequences. In certain embodiments, the CH1 sequences are from an IgA1, IgA2, IgD, IgE, IgG1, IgG2, IgG3, IgG4, or IgM isotype. In a preferred embodiment, the CH1 sequences are from an IgG1 isotype. In preferred embodiments, the CH1 sequence is UniProt accession number P01857 amino acids 1-98.

### 4.3.4.2. CL Domains

The CL amino acid sequences useful in the binding molecules described herein are antibody light chain constant domain sequences. In certain embodiments, the CL sequences are endogenous sequences. In a variety of embodiments, the CL sequences are mammalian sequences, including, but not limited to mouse, rat, hamster, rabbit, camel, donkey, goat, and human sequences. In a preferred embodiment, CL sequences are human sequences.

In certain embodiments, the CL amino acid sequences are lambda (λ) light chain constant domain sequences. In particular embodiments, the CL amino acid sequences are human lambda light chain constant domain sequences. In preferred embodiments, the lambda (λ) light chain sequence is UniProt accession number P0CG04.

In certain embodiments, the CL amino acid sequences are kappa (κ) light chain constant domain sequences. In a preferred embodiment, the CL amino acid sequences are human kappa (κ) light chain constant domain sequences. In a preferred embodiment, the kappa light chain sequence is UniProt accession number P01834.

### 4.3.5. Domain F (VH)

In the binding molecules, domain F has a VH amino acid sequence. The VH amino acid sequences in the binding molecules described herein are antibody heavy chain variable domain sequences. In a typical antibody arrangement in both nature and in the binding molecules described herein, a specific VH amino acid sequence associates with a specific VL amino acid sequence to form an antigen-binding site. In various embodiments, VH amino acid sequences are mammalian sequences, including human sequences, synthesized sequences, or combinations of non-human mammalian, mammalian, and/or synthesized sequences, as described in further detail above in Sections 4.3.1.1 and 4.3.1.2. In various embodiments, VH amino acid sequences are mutated sequences of naturally occurring sequences.

### 4.3.6. Domain G (CH3)

In the binding molecules, domain G has a CH3 amino acid sequence. CH3 sequences are described in greater detail above in Section 4.3.2.

In certain preferred embodiments, domain G has a human IgG1 CH3 sequence with the following mutational changes: S354C; and a tripeptide insertion, 445P, 446G, 447K. In some preferred embodiments, domain G has a human IgG1 CH3 sequence with the following mutational changes: S354C; and 445P, 446G, 447K tripeptide insertion. In some preferred embodiments, domain G has a human IgG1 CH3 sequence with the following changes: L351D, and a tripeptide insertion of 445G, 446E, 447C.

### 4.3.7. Domain H (VL)

In the binding molecules, domain H has a VL amino acid sequence. VL sequences are described in greater detail above in Section 4.3.1.

### 4.3.8. Domain I (CL)

In the binding molecules described herein, domain I has a CL amino acid sequence. CL domain amino acid sequences are described in greater detail above in Section 4.3.4.2. In a preferred embodiment, domain I has a constant region sequence that is a CL from a kappa light chain, as discussed in more detail in Section 4.3.4.2.

### 4.3.9. Domain J (CH2)

In the binding molecules, domain J has a CH2 amino acid sequence. CH2 amino acid sequences are described in greater detail above in Section 4.3.3. In a preferred embodiment, the CH2 amino acid sequence has a N-terminal hinge region that connects domain J to domain I, as described in more detail below in Section 4.3.19.4.

In the binding molecules, the C-terminus of domain J is connected to the N-terminus of domain K. In particular embodiments, domain J is connected to the N-terminus of domain K that has a CH1 amino acid sequence or CL amino acid sequence, as described in further detail below in Section 4.3.19.5.

### 4.3.10. Domain K (CH3)

In the binding molecules, domain K has a CH3 amino acid sequence. CH3 amino acid sequences are described in greater detail above in Section 4.3.4. In a preferred embodiment, domain K has a CH3 sequence comprising knob-hole orthogonal mutations, as described in greater detail below in Section 4.3.14.2; isoallotype mutations, as described in more detail above in 4.3.2.; and either a S354C or a Y349C mutation that forms an engineered disulfide bridge with a CH3 domain containing an orthogonal mutation, as described in in greater detail below in Section 4.3.14.1. In some preferred embodiments, the knob-hole orthogonal mutations combined with isoallotype mutations are the following mutational changes: D356E, L358M, T366S, L368A, and Y407V.

### 4.3.11. Domain L (VH)

In the binding molecules, domain L has a VH amino acid sequence. VH sequences are described in greater detail above in Section 4.3.5.

### 4.3.12. Domain M (CH1)

In the binding molecules, domain M has a CH1 amino acid sequence. CH1 amino acid sequences are described in greater detail above in Section 4.3.4.1. In a preferred embodiment, domain M has a sequence that is a CH1 from an IgG1 isotype, as discussed in more detail in Section 4.3.4.1.

### 4.3.13. Pairing of Domains A & F

In the binding molecules, a Domain A VL amino acid sequence and a Domain F VH amino acid sequence are associated and form an antigen binding site (ABS). The A:F antigen binding site (ABS) is capable of specifically binding an epitope of an antigen. Antigen binding by an ABS is described in greater detail below in Section 4.3.13.1.

In a variety of multivalent embodiments, the ABS formed by domains A and F (A:F) is identical in sequence to one or more other ABSs within the binding molecule and therefore has the same recognition specificity as the one or more other sequence-identical ABSs within the binding molecule.

In a variety of multivalent embodiments, the A:F ABS is non-identical in sequence to one or more other ABSs within the binding molecule. In certain embodiments, the A:F ABS has a recognition specificity different from that of one or more other sequence-non-identical ABSs in the binding molecule. In particular embodiments, the A:F ABS recognizes a different antigen from that recognized by at least one other sequence-non-identical ABS in the binding molecule. In particular embodiments, the A:F ABS recognizes a different epitope of an antigen that is also recognized by at least one other sequence-non-identical ABS in the binding molecule. In these embodiments, the ABS formed by domains A and F recognizes an epitope of antigen, wherein one or more other ABSs within the binding molecule recognizes the same antigen but not the same epitope.

### 4.3.13.1. Binding of Antigen by ABS

An ABS, and the binding molecule comprising such ABS, is said to "recognize" the epitope (or more generally, the antigen) to which the ABS specifically binds, and the epitope (or more generally, the antigen) is said to be the **"recognition specificity" or "binding specificity"** of the ABS.

The ABS is said to bind to its specific antigen or epitope with a particular affinity.

As described herein, **"affinity"** refers to the strength of interaction of non-covalent intermolecular forces between one molecule and another. The affinity, i.e., the strength of the interaction, can be expressed as a dissociation equilibrium constant (K_{D}), wherein a lower K_{D} value refers to a stronger interaction between molecules. K_{D} values of antibody constructs are measured by methods well known in the art including, but not limited to, bio-layer interferometry (e.g., Octet/FORTEBIO^{®}), surface plasmon resonance (SPR) technology (e.g., Biacore^{®}), and cell binding assays. For purposes herein, affinities are dissociation equilibrium constants measured by bio-layer interferometry using Octet/FORTEBIO^{®}.

**"Specific binding,"** as used herein, refers to an affinity between an ABS and its cognate antigen or epitope in which the K_{D} value is below 10⁻⁶M, 10⁻⁷M, 10⁻⁸M, 10⁻⁹M, or 10⁻¹⁰M.

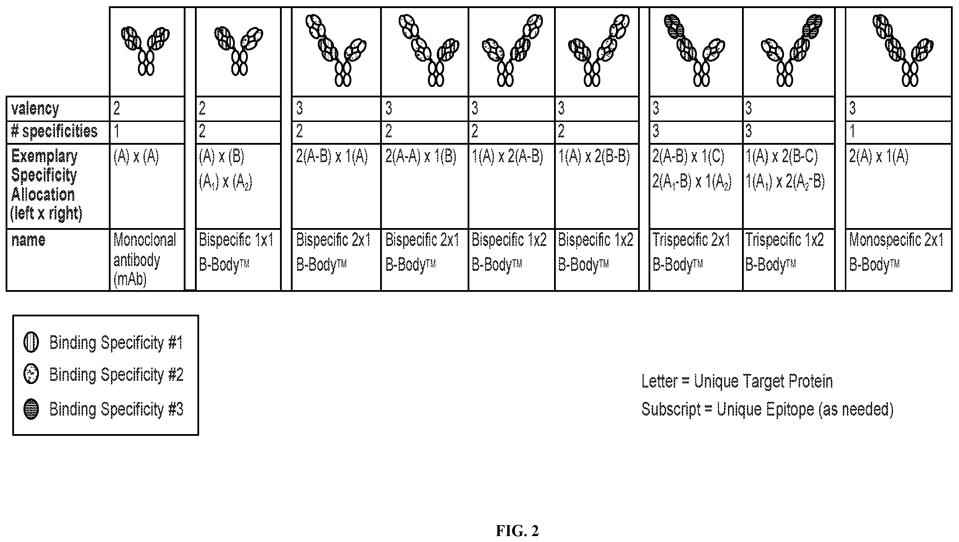

The number of ABSs in a binding molecule as described herein defines the "valency" of the binding molecule. As schematized in **FIG. 2****,** a binding molecule having a single ABS is **"monovalent".** A binding molecule having a plurality of ABSs is said to be **"multivalent".** A multivalent binding molecule having two ABSs is **"bivalent."** A multivalent binding molecule having three ABSs is **"trivalent."** A multivalent binding molecule having four ABSs is **"tetravalent."**

In various multivalent embodiments, all of the plurality of ABSs have the same recognition specificity. As schematized in **FIG. 2****,** such a binding molecule is a **"monospecific"** "multivalent" binding construct. In other multivalent embodiments, at least two of the plurality of ABSs have different recognition specificities. Such binding molecules are multivalent and **"multispecific".** In multivalent embodiments in which the ABSs collectively have two recognition specificities, the binding molecule is **"bispecific."** In multivalent embodiments in which the ABSs collectively have three recognition specificities, the binding molecule is **"trispecific."**

In multivalent embodiments in which the ABSs collectively have a plurality of recognition specificities for different epitopes present on the same antigen, the binding molecule is **"multiparatopic."** Multivalent embodiments in which the ABSs collectively recognize two epitopes on the same antigen are **"biparatopic."**

In various multivalent embodiments, multivalency of the binding molecule improves the avidity of the binding molecule for a specific target. As described herein, **"avidity"** refers to the overall strength of interaction between two or more molecules, e.g., a multivalent binding molecule for a specific target, wherein the avidity is the cumulative strength of interaction provided by the affinities of multiple ABSs. Avidity can be measured by the same methods as those used to determine affinity, as described above. In certain embodiments, the avidity of a binding molecule for a specific target is such that the interaction is a specific binding interaction, wherein the avidity between two molecules has a K_{D} value below 10⁻⁶M, 10⁻⁷M, 10⁻⁸M, 10⁻⁹M, or 10⁻¹⁰M. In certain embodiments, the avidity of a binding molecule for a specific target has a K_{D} value such that the interaction is a specific binding interaction, wherein the one or more affinities of individual ABSs do not have has a K_{D} value that qualifies as specifically binding their respective antigens or epitopes on their own. In certain embodiments, the avidity is the cumulative strength of interaction provided by the affinities of multiple ABSs for separate antigens on a shared specific target or complex, such as separate antigens found on an individual cell. In certain embodiments, the avidity is the cumulative strength of interaction provided by the affinities of multiple ABSs for separate epitopes on a shared individual antigen.

### 4.3.14. Pairing of Domains B & G

In the binding molecules described herein, a domain B CH3 amino acid sequence and a domain G CH3 amino acid sequence are associated. CH3 sequences are described in greater detail above in Section 4.3.2.

In various embodiments, the amino acid sequences of the B and the G domains are identical. In certain of these embodiments, the sequence is an endogenous CH3 sequence.

In a variety of embodiments, the amino acid sequences of the B and the G domains are different, and separately comprise respectively orthogonal modifications in an endogenous CH3 sequence, wherein the B domain interacts with the G domain, and wherein neither the B domain nor the G domain significantly interacts with a CH3 domain lacking the orthogonal modification.

**"Orthogonal modifications"** or synonymously **"orthogonal mutations"** as described herein are one or more engineered mutations in an amino acid sequence of an antibody domain that increase the affinity of binding of a first domain having orthogonal modification for a second domain having a complementary orthogonal modification. In certain embodiments, the orthogonal modifications decrease the affinity of a domain having the orthogonal modifications for a domain lacking the complementary orthogonal modifications. In certain embodiments, orthogonal modifications are mutations in an endogenous antibody domain sequence. In a variety of embodiments, orthogonal modifications are modifications of the N-terminus or C-terminus of an endogenous antibody domain sequence including, but not limited to, amino acid additions or deletions. In particular embodiments, orthogonal modifications include, but are not limited to, engineered disulfide bridges, knob-in-hole mutations, and charge-pair mutations, as described in greater detail below in Sections 4.3.14.1-4.3.14.3. In particular embodiments, orthogonal modifications include a combination of orthogonal modifications selected from, but not limited to, engineered disulfide bridges, knob-in-hole mutations, and charge-pair mutations. In particular embodiments, the orthogonal modifications can be combined with amino acid substitutions that reduce immunogenicity, such as isoallotype mutations, as described in greater detail above in Section 4.3.2.

### 4.3.14.1. Orthogonal Engineered Disulfide Bridges

In a variety of embodiments, the orthogonal modifications comprise mutations that generate engineered disulfide bridges between a first and a second domain. As described herein, **"engineered disulfide bridges"** are mutations that provide non-endogenous cysteine amino acids in two or more domains such that a non-native disulfide bond forms when the two or more domains associate. Engineered disulfide bridges are described in greater detail in Merchant et al. (Nature Biotech (1998) 16:677-681). In certain embodiments, engineered disulfide bridges improve orthogonal association between specific domains. In a particular embodiment, the mutations that generate engineered disulfide bridges are a K392C mutation in one of a first or second CH3 domains, and a D399C in the other CH3 domain. In a preferred embodiment, the mutations that generate engineered disulfide bridges are a S354C mutation in one of a first or second CH3 domains, and a Y349C in the other CH3 domain. In another preferred embodiment, the mutations that generate engineered disulfide bridges are a 447C mutation in both the first and second CH3 domains that are provided by extension of the C-terminus of a CH3 domain incorporating a KSC tripeptide sequence.

### 4.3.14.2. Orthogonal Knob-Hole Mutations

In a variety of embodiments, orthogonal modifications comprise knob-hole (synonymously, knob-in-hole) mutations. As described herein, knob-hole mutations are mutations that change the steric features of a first domain's surface such that the first domain will preferentially associate with a second domain having complementary steric mutations relative to association with domains without the complementary steric mutations. Knob-hole mutations are described in greater detail in U.S. Pat. No. 5,821,333 and U.S. Pat. No. 8,216,805. In various embodiments, knob-hole mutations are combined with engineered disulfide bridges, as described in greater detail in Merchant et al. (Nature Biotech (1998) 16:677-681)). In various embodiments, knob-hole mutations, isoallotype mutations, and engineered disulfide mutations are combined.

In certain embodiments, the knob-in-hole mutations are a T366Y mutation in a first domain, and a Y407T mutation in a second domain. In certain embodiments, the knob-in-hole mutations are a F405A in a first domain, and a T394W in a second domain. In certain embodiments, the knob-in-hole mutations are a T366Y mutation and a F405A in a first domain, and a T394W and a Y407T in a second domain. In certain embodiments, the knob-in-hole mutations are a T366W mutation in a first domain, and a Y407A in a second domain. In certain embodiments, the combined knob-in-hole mutations and engineered disulfide mutations are a S354C and T366W mutations in a first domain, and a Y349C, T366S, L368A, and aY407V mutation in a second domain. In a preferred embodiment, the combined knob-in-hole mutations, isoallotype mutations, and engineered disulfide mutations are a S354C and T366W mutations in a first domain, and a Y349C, D356E, L358M, T366S, L368A, and aY407V mutation in a second domain.

### 4.3.14.3. Orthogonal Charge-pair Mutations

In a variety of embodiments, orthogonal modifications are charge-pair mutations. As used herein, charge-pair mutations are mutations that affect the charge of an amino acid in a domain's surface such that the domain will preferentially associate with a second domain having complementary charge-pair mutations relative to association with domains without the complementary charge-pair mutations. In certain embodiments, charge-pair mutations improve orthogonal association between specific domains. Charge-pair mutations are described in greater detail in U.S. Pat. No. 8,592,562, U.S. Pat. No. 9,248,182, and U.S. Pat. No. 9,358,286. In certain embodiments, charge-pair mutations improve stability between specific domains. In a preferred embodiment, the charge-pair mutations are a T366K mutation in a first domain, and a L351D mutation in the other domain.

### 4.3.15. Pairing of Domains E & K

In the binding molecules described herein, the E domain has a CH3 amino acid sequence and the K domain has a CH3 amino acid sequence.

In a variety of embodiments, the amino acid sequences of the E and K domains are identical, wherein the sequence is an endogenous CH3 sequence.

In a variety of embodiments, the sequences of the E and K domains are different. In a variety of embodiments, the different sequences separately comprise respectively orthogonal modifications in an endogenous CH3 sequence, wherein the E domain interacts with the K domain, and wherein neither the E domain nor the K domain significantly interacts with a CH3 domain lacking the orthogonal modification. In certain embodiments, the orthogonal modifications include, but are not limited to, engineered disulfide bridges, knob-in-hole mutations, and charge-pair mutations, as described in greater detail above in sections 4.3.14.1-4.3.14.3. In particular embodiments, orthogonal modifications include a combination of orthogonal modifications selected from, but not limited to, engineered disulfide bridges, knob-in-hole mutations, and charge-pair mutations. In particular embodiments, the orthogonal modifications can be combined with amino acid substitutions that reduce immunogenicity, such as isoallotype mutations.

### 4.3.16. Pairing of Domains I & M and Pairing of Domains H & L

In the binding molecules described herein, domain H has a VL amino acid sequence, domain I has a CL amino acid sequence, domain L has a VH amino acid sequence, domain M has a CH1 amino acid sequence, and domain K has a CH3 amino acid sequence.

In a variety of embodiments, the amino acid sequences of the I domain and the M domain separately comprise respectively orthogonal modifications in an endogenous sequence, wherein the I domain interacts with the M domain, and wherein neither the I domain nor the M domain significantly interacts with a domain lacking the orthogonal modification. Orthogonal mutations are described in more detail above in Sections 4.3.14.1-4.3.14.3. In a variety of embodiments, the orthogonal mutations in the CL sequence and the CH1 sequence are charge-pair mutations. In specific embodiments the charge-pair mutations are a F118S, F118A or F118V mutation in the CL sequence with a corresponding A141L in the CH1 sequence, or a T129R mutation in the CL sequence with a corresponding K147D in the CH1 sequence, as numbered by the Eu index and described in greater detail in Bonisch et al. (Protein Engineering, Design & Selection, 2017, pp. 1-12) In a series of preferred embodiments the charge-pair mutations are a N138K mutation in the CL sequence with a corresponding G166D in the CH1 sequence, or a N138D mutation in the CL sequence with a corresponding G166K in the CH1 sequence, as numbered by the Eu index.

In a variety of embodiments, the orthogonal mutations in the CL sequence and the CH1 sequence generate an engineered disulfide bridge. In a series of preferred embodiments, the mutations that provide non-endogenous cysteine amino acids are a F118C mutation in the CL sequence with a corresponding A141C in the CH1 sequence, or a F118C mutation in the CL sequence with a corresponding L128C in the CH1 sequence, or a S162C mutations in the CL sequence with a corresponding P171C mutation in the CH1 sequence, as numbered by the Eu index.

In a variety of embodiments, the amino acid sequences of the H domain and the L domain separately comprise respectively orthogonal modifications in an endogenous sequence, wherein the H domain interacts with the L domain, and wherein neither the H domain nor the L domain significantly interacts with a domain lacking the orthogonal modification. In specific embodiments, the orthogonal mutations are charge-pair mutations at the VH/VL interface. In preferred embodiments, the charge-pair mutations at the VH/VL interface are a Q39E in VH with a corresponding Q38K in VL, or a Q39K in VH with a corresponding Q38E in VL, as described in greater detail in Igawa et al. (Protein Eng. Des. Sel., 2010, vol. 23, 667-677)

In certain embodiments, the interaction between the A domain and the F domain form a first antigen binding site specific for a first antigen, and the interaction between the H domain and the L domain form a second antigen binding site specific for a second antigen. In certain embodiments, the interaction between the A domain and the F domain form a first antigen binding site specific for a first antigen, and the interaction between the H domain and the L domain form a second antigen binding site specific for the first antigen.

### 4.3.17. Trivalent Binding Molecules

In another series of embodiments, the binding molecules have three antigen binding sites and are therefore termed **"trivalent."**

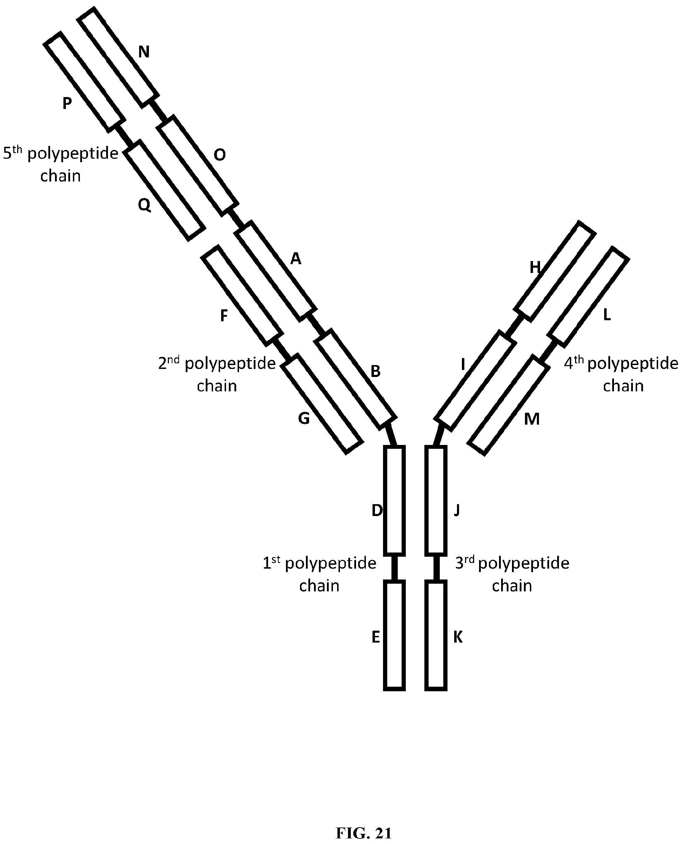

With reference to **FIG. 21****,** in various trivalent embodiments the binding molecules further comprise a fifth polypeptide chain, wherein (a) the first polypeptide chain further comprises a domain N and a domain O, wherein the domains are arranged, from N-terminus to C-terminus, in a N-O-A-B-D-E orientation, and wherein domain N has a VL amino acid sequence, domain O has a CH3 amino acid sequence; (b) the binding molecule further comprises a fifth polypeptide chain, comprising: a domain P and a domain Q, wherein the domains are arranged, from N-terminus to C-terminus, in a P-Q orientation, and wherein domain P has a VH amino acid sequence and domain Q has a CH3 amino acid sequence; and (c) the first and the fifth polypeptides are associated through an interaction between the N and the P domains and an interaction between the O and the Q domains to form the binding molecule. As schematized in **FIG. 2****,** these trivalent embodiments are termed "2x1" trivalent constructs.

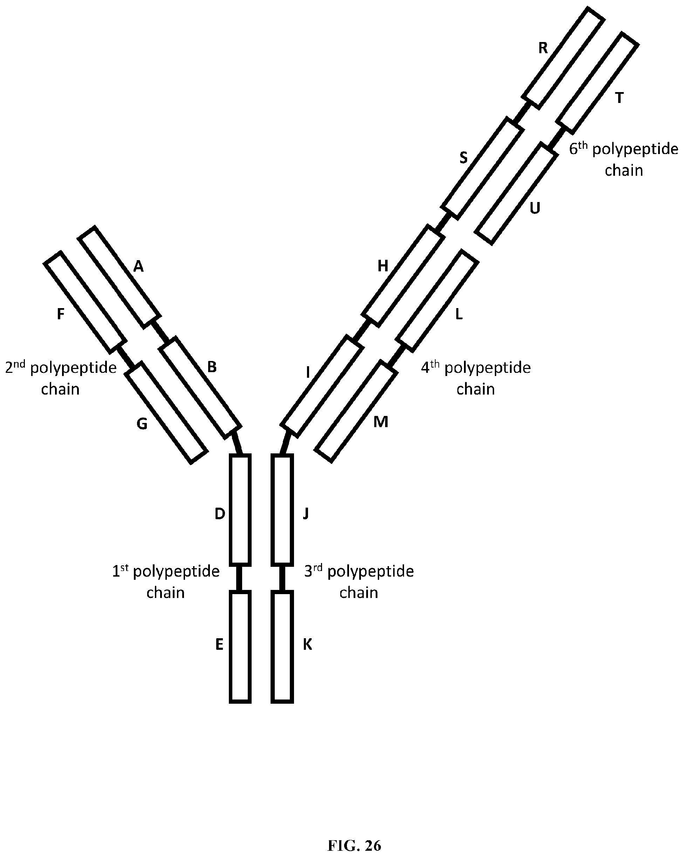

With reference to **FIG. 26****,** in a further series of trivalent embodiments, the binding molecules further comprise a sixth polypeptide chain, wherein (a) the third polypeptide chain further comprises a domain R and a domain S, wherein the domains are arranged, from N-terminus to C-terminus, in a R-S-H-I-J-K orientation, and wherein domain R has a VL amino acid sequence and domain S has a constant domain amino acid sequence; (b) the binding molecule further comprises a sixth polypeptide chain, comprising: a domain T and a domain U, wherein the domains are arranged, from N-terminus to C-terminus, in a T-U orientation, and wherein domain T has a VH amino acid sequence and domain U has a constant domain amino acid sequence; and (c) the third and the sixth polypeptides are associated through an interaction between the R and the T domains and an interaction between the S and the U domains to form the binding molecule. As schematized in FIG. 2, these trivalent embodiments are termed "1x2" trivalent constructs.

In a variety of embodiments, the domain O is connected to domain A through a peptide linker. In a variety of embodiments, the domain S is connected to domain H through a peptide linker. In a preferred embodiment, the peptide linker connecting either domain O to domain A or connecting domain S to domain H is a 6 amino acid GSGSGS peptide sequence, as described in more detail in Section 4.3.19.6.

### 4.3.17.1. Trivalent 2x1 Bispecific Constructs [2(A-A)x1(B)]

With reference to **FIG. 21****,** in a variety of embodiments the amino acid sequences of domain N and domain A are identical, the amino acid sequences of domain H is different from domains N and A, the amino acid sequences of domain O and domain B are identical, the amino acid sequences of domain I is different from domains O and B, the amino acid sequences of domain P and domain F are identical, the amino acid sequences of domain L is different from domains P and F, the amino acid sequences of domain Q and domain G are identical, the amino acid sequences of domain M is different from domains Q and G; and the interaction between the A domain and the F domain form a first antigen binding site specific for a first antigen, the interaction between the H domain and the L domain form a second antigen binding site specific for a second antigen, and the domain N and domain P form a third antigen binding site specific for the first antigen.

### 4.3.17.2. Trivalent 2x1 Bispecific Constructs [2(A-B)x1(A)]

With reference to **FIG. 21****,** in a variety of constructs disclosed herein (but not claimed), the amino acid sequences of domain N and domain H are identical, the amino acid sequences of domain A is different from domains N and H, the amino acid sequences of domain O and domain I are identical, the amino acid sequences of domain B is different from domains O and I, the amino acid sequences of domain P and domain L are identical, the amino acid sequences of domain F is different from domains P and L, the amino acid sequences of domain Q and domain M are identical, the amino acid sequences of domain G is different from domains Q and M; and the interaction between the A domain and the F domain form a first antigen binding site specific for a first antigen, the interaction between the H domain and the L domain form a second antigen binding site specific for a second antigen, and the domain N and domain P form a third antigen binding site specific for the second antigen.

### 4.3.17.3. Trivalent 2x1 Trispecific Constructs [2(A-B)x1(C)]

With reference to **FIG. 21****,** in a variety of embodiments, the amino acid sequences of domain N, domain A, and domain H are different, the amino acid sequences of domain O, domain B, and domain I are different, the amino acid sequences of domain P, domain F, and domain L are different, and the amino acid sequences of domain Q, domain G, and domain M are different; and the interaction between the A domain and the F domain form a first antigen binding site specific for a first antigen, the interaction between the H domain and the L domain form a second antigen binding site specific for a second antigen, and the domain N and domain P form a third antigen binding site specific for a third antigen.

In certain embodiments, domain O has a constant region sequence that is a CL from a kappa light chain and domain Q has a constant region sequence that is a CH1 from an IgG1 isotype, as discussed in more detail in Sections 4.3.4.1 and 4.3.4.2. In a preferred embodiment, domain O and domain Q have CH3 sequences such that they specifically associate with each other, as discussed in more detail above in Section 4.3.14.

### 4.3.17.4. Trivalent 2x1 Monospecific Constructs

With reference to **FIG. 21****,** in a variety of embodiments, the amino acid sequences of domain N, domain A, and domain H are identical, the amino acid sequences of domain O and domain B are identical, the amino acid sequences of domain P, domain F, and domain L are identical, and the amino acid sequences of domain Q and domain G are identical; and the interaction between the A domain and the F domain form a first antigen binding site specific for a first antigen, the interaction between the H domain and the L domain form a second antigen binding site specific for the first antigen, and the domain N and domain P form a third antigen binding site specific for the first antigen.

With reference to **FIG. 21****,** in another series of constructs disclosed herein (but not claimed), the amino acid sequences of domain N, domain A, and domain H are identical, the amino acid sequences of domain O, domain B, and domain I are identical, the amino acid sequences of domain P, domain F, and domain L are identical, and the amino acid sequences of domain Q, domain G, and domain M are identical; and the interaction between the A domain and the F domain form a first antigen binding site specific for a first antigen, the interaction between the H domain and the L domain form a second antigen binding site specific for the first antigen, and the domain N and domain P form a third antigen binding site specific for the first antigen.

### 4.3.17.5. Trivalent 1x2 Bispecific Constructs [1(A)x2(B-A)]

With reference to **FIG. 26****,** in a variety of embodiments, the amino acid sequences of domain R and domain A are identical, the amino acid sequences of domain H is different from domain R and A, the amino acid sequences of domain S and domain B are identical, the amino acid sequences of domain I is different from domain S and B, the amino acid sequences of domain T and domain F are identical, the amino acid sequences of domain L is different from domain T and F, the amino acid sequences of domain U and domain G are identical, the amino acid sequences of domain M is different from domain U and G and the interaction between the A domain and the F domain form a first antigen binding site specific for a first antigen, the interaction between the H domain and the L domain form a second antigen binding site specific for a second antigen, and the domain R and domain T form a third antigen binding site specific for the first antigen.

### 4.3.17.6. Trivalent 1x2 Bispecific Constructs [1(A)x2(B-B)]

With reference to **FIG. 26****,** in a variety of embodiments, the amino acid sequences of domain R and domain H are identical, the amino acid sequences of domain A is different from domain R and H, the amino acid sequences of domain S and domain I are identical, the amino acid sequences of domain B is different from domain S and I, the amino acid sequences of domain T and domain L are identical, the amino acid sequences of domain F is different from domain T and L, the amino acid sequences of domain U and domain M are identical, the amino acid sequences of domain G is different from domain U and M and the interaction between the A domain and the F domain form a first antigen binding site specific for a first antigen, the interaction between the H domain and the L domain form a second antigen binding site specific for a second antigen, and the domain R and domain T form a third antigen binding site specific for the second antigen.

### 4.3.17.7. Trivalent 1x2 Trispecific Constructs [1(A)x2(B-C)]

With reference to **FIG. 26****,** in a variety of embodiments, the amino acid sequences of domain R, domain A, and domain H are different, the amino acid sequences of domain S, domain B, and domain I are different, the amino acid sequences of domain T, domain F, and domain L are different, and the amino acid sequences of domain U, domain G, and domain M are different; and the interaction between the A domain and the F domain form a first antigen binding site specific for a first antigen, the interaction between the H domain and the L domain form a second antigen binding site specific for a second antigen, and the domain R and domain T form a third antigen binding site specific for a third antigen.

In particular embodiments, domain S has a constant region sequence that is a CL from a kappa light chain and domain U has a constant region sequence that is a CH1 from an IgG1 isotype, as discussed in more detail in Sections 4.3.4.1 and 4.3.4.2. In a preferred embodiment, domain S and domain U have CH3 sequences such that they specifically associate with each other, as discussed in more detail above in Section 4.3.14.

### 4.3.17.8. Trivalent 1x2 Monospecific Constructs

With reference to **FIG. 26****,** in a variety of embodiments, the amino acid sequences of domain R, domain A, and domain H are identical, the amino acid sequences of domain S and domain B are identical, the amino acid sequences of domain T, domain F, and domain L are identical, and the amino acid sequences of domain U and domain G are identical; and the interaction between the A domain and the F domain form a first antigen binding site specific for a first antigen, the interaction between the H domain and the L domain form a second antigen binding site specific for the first antigen, and the domain R and domain T form a third antigen binding site specific for the first antigen.

### 4.3.18. Tetravalent 2x2 Binding Molecules

In a variety of embodiments, the binding molecules have 4 antigen binding sites and are therefore termed **"tetravalent."**

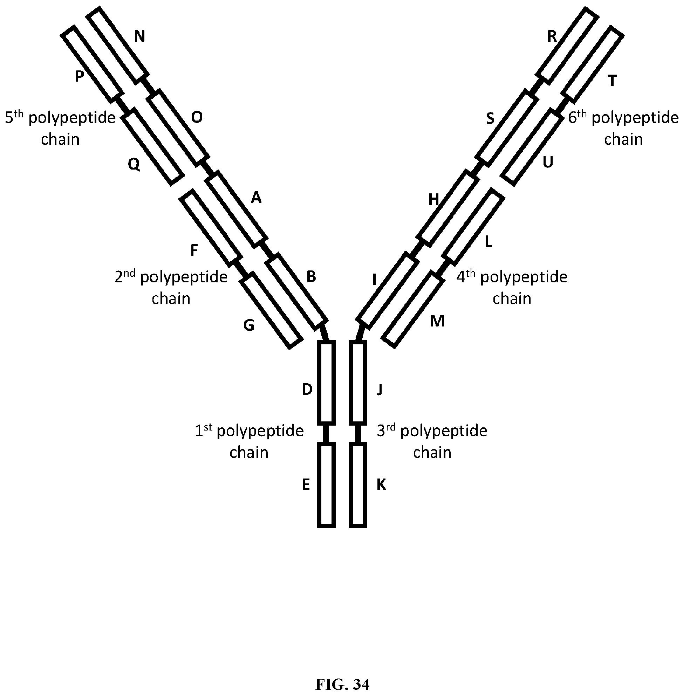

With reference to **FIG. 34****,** in a further series of embodiments, the binding molecules further comprise a fifth and a sixth polypeptide chain, wherein (a) the first polypeptide chain further comprises a domain N and a domain O, wherein the domains are arranged, from N-terminus to C-terminus, in a N-O-A-B-D-E orientation; (b) the third polypeptide chain further comprises a domain R and a domain S, wherein the domains are arranged, from N-terminus to C-terminus, in a R-S-H-I-J-K orientation; (c) the binding molecule further comprises a fifth and a sixth polypeptide chain, wherein the fifth polypeptide chain comprises a domain P and a domain Q, wherein the domains are arranged, from N-terminus to C-terminus, in a P-Q orientation, and the sixth polypeptide chain comprises a domain T and a domain U, wherein the domains are arranged, from N-terminus to C-terminus, in a T-U orientation; and (d) the first and the fifth polypeptides are associated through an interaction between the N and the P domains and an interaction between the O and the Q domains, and the third and the sixth polypeptides are associated through an interaction between the R and the T domains and an interaction between the S and the U domains to form the binding molecule.

In a variety of embodiments, the domain O is connected to domain A through a peptide linker and the domain S is connected to domain H through a peptide linker. In a preferred embodiment, the peptide linker connecting domain O to domain A and connecting domain S to domain H is a 6 amino acid GSGSGS peptide sequence, as described in more detail in Section 4.3.19.6.

### 4.3.18.1. Tetravalent 2x2 Bispecific Constructs

With reference to **FIG. 34****,** in a series of tetravalent 2x2 bispecific binding molecules, the amino acid sequences of domain N and domain A are identical, the amino acid sequences of domain H and domain R are identical, the amino acid sequences of domain O and domain B are identical, the amino acid sequences of domain I and domain S are identical, the amino acid sequences of domain P and domain F are identical, the amino acid sequences of domain L and domain T are identical, the amino acid sequences of domain Q and domain G are identical, the amino acid sequences of domain M and domain U are identical; and wherein the interaction between the A domain and the F domain form a first antigen binding site specific for a first antigen, the domain N and domain P form a second antigen binding site specific for the first antigen, the interaction between the H domain and the L domain form a third antigen binding site specific for a second antigen, and the interaction between the R domain and the T domain form a fourth antigen binding site specific for the second antigen.

With reference to **FIG. 34****,** in a series of tetravalent 2x2 bispecific binding molecules disclosed herein (but not claimed), the amino acid sequences of domain H and domain A are identical, the amino acid sequences of domain N and domain R are identical, the amino acid sequences of domain I and domain B are identical, the amino acid sequences of domain O and domain S are identical, the amino acid sequences of domain L and domain F are identical, the amino acid sequences of domain P and domain T are identical, the amino acid sequences of domain M and domain G are identical, the amino acid sequences of domain Q and domain U are identical; and wherein the interaction between the A domain and the F domain form a first antigen binding site specific for a first antigen, the domain N and domain P form a second antigen binding site specific for a second antigen, the interaction between the H domain and the L domain form a third antigen binding site specific for the first antigen, and the interaction between the R domain and the T domain form a fourth antigen binding site specific for the second antigen.

### 4.3.18.2. Tetravalent 2x2 Monospecific Constructs

With reference to **FIG. 34****,** in a variety of embodiments, the amino acid sequences of domain N, domain A, domain H and domain R are identical, the amino acid sequences of domain O and domain B are identical, the amino acid sequences of domain I and domain S are identical, the amino acid sequences of domain P, domain F, domain L, and domain T are identical, the amino acid sequences of domain Q and domain G are identical, the amino acid sequences of domain M and domain U are identical; and wherein the interaction between the A domain and the F domain form a first antigen binding site specific for a first antigen, the domain N and domain P form a second antigen binding site specific for the first antigen, the interaction between the H domain and the L domain form a third antigen binding site specific for the first antigen, and the interaction between the R domain and the T domain form a fourth antigen binding site specific for the first antigen.

With reference to **FIG. 34****,** in a series of tetravalent 2x2 monospecific binding molecules disclosed herein (but not claimed), the amino acid sequences of domain N, domain A, domain H and domain R are identical, the amino acid sequences of domain I and domain B are identical, the amino acid sequences of domain O and domain S are identical, the amino acid sequences of domain P, domain F, domain L, and domain T are identical, the amino acid sequences of domain M and domain G are identical, the amino acid sequences of domain Q and domain U are identical; and wherein the interaction between the A domain and the F domain form a first antigen binding site specific for a first antigen, the domain N and domain P form a second antigen binding site specific for the first antigen, the interaction between the H domain and the L domain form a third antigen binding site specific for the first antigen, and the interaction between the R domain and the T domain form a fourth antigen binding site specific for the first antigen.

### 4.3.19. Domain Junctions

### 4.3.19.1. Junctions Connecting VL and CH3 Domains

In a variety of embodiments, the amino acid sequence that forms a junction between the C-terminus of a VL domain and the N-terminus of a CH3 domain is an engineered sequence. In certain embodiments, one or more amino acids are deleted or added in the C-terminus of the VL domain. In certain embodiments, the junction connecting the C-terminus of a VL domain and the N-terminus of a CH3 domain is one of the sequences described in Table 2 below in Section 4.11.6. In particular embodiments, A111 is deleted in the C-terminus of the VL domain. In certain embodiments, one or more amino acids are deleted or added in the N-terminus of the CH3 domain. In particular embodiments, P343 is deleted in the N-terminus of the CH3 domain. In particular embodiments, P343 and R344 are deleted in the N-terminus of the CH3 domain. In certain embodiments, one or more amino acids are deleted or added to both the C-terminus of the VL domain and the N-terminus of the CH3 domain. In particular embodiments, A111 is deleted in the C-terminus of the VL domain and P343 is deleted in the N-terminus of the CH3 domain. In a preferred embodiment, A111 and V110 are deleted in the C-terminus of the VL domain. In another preferred embodiment, A111 and V110 are deleted in the C-terminus of the VL domain and the N-terminus of the CH3 domain has a P343V mutation.

### 4.3.19.2. Junctions Connecting VH and CH3 Domains

In a variety of embodiments, the amino acid sequence that forms a junction between the C-terminus of a VH domain and the N-terminus of a CH3 domain is an engineered sequence. In certain embodiments, one or more amino acids are deleted or added in the C-terminus of the VH domain. In certain embodiments, the junction connecting the C-terminus of a VH domain and the N-terminus of the CH3 domain is one of the sequences described in Table 3 below in Section 4.11.6. In particular embodiments, K177 and G118 are deleted in the C-terminus of the VH domain. In certain embodiments, one or more amino acids are deleted or added in the N-terminus of the CH3 domain. In particular embodiments, P343 is deleted in the N-terminus of the CH3 domain. In particular embodiments, P343 and R344 are deleted in the N-terminus of the CH3 domain. In particular embodiments, P343, R344, and E345 are deleted in the N-terminus of the CH3 domain. In certain embodiments, one or more amino acids are deleted or added to both the C-terminus of the VH domain and the N-terminus of the CH3 domain. In a preferred embodiment, T166, K177, and G118 are deleted in the C-terminus of the VH domain.

### 4.3.19.3. Junctions Connecting CH3 C-terminus to CH2 N-terminus (Hinge)

In the binding molecules described herein, the N-terminus of the CH2 domain has a "hinge" region amino acid sequence. As used herein, hinge regions are sequences of an antibody heavy chain that link the N-terminal variable domain-constant domain segment of an antibody and a CH2 domain of an antibody. In addition, the hinge region typically provides both flexibility between the N-terminal variable domain-constant domain segment and CH2 domain, as well as amino acid sequence motifs that form disulfide bridges between heavy chains (e.g., the first and the third polypeptide chains). As used herein, the hinge region amino acid sequence is SEQ ID NO: 56.

In a variety of embodiments, a CH3 amino acid sequence is extended at the C-terminus at the junction between the C-terminus of the CH3 domain and the N-terminus of a CH2 domain. In certain embodiments, a CH3 amino acid sequence is extended at the C-terminus at the junction between the C-terminus of the CH3 domain and a hinge region, which in turn is connected to the N-terminus of a CH2 domain. In a preferred embodiment, the CH3 amino acid sequence is extended by inserting a PGK tripeptide sequence followed by the DKTHT motif of an IgG1 hinge region.

In a particular embodiment, the extension at the C-terminus of the CH3 domain incorporates amino acid sequences that can form a disulfide bond with orthogonal C-terminal extension of another CH3 domain. In a preferred embodiment, the extension at the C-terminus of the CH3 domain incorporates a KSC tripeptide sequence that is followed by the DKTHT motif of an IgG1 hinge region that forms a disulfide bond with orthogonal C-terminal extension of another CH3 domain that incorporates a GEC motif of a kappa light chain.

### 4.3.19.4. Junctions Connecting CL C-Terminus and CH2 N-Terminus (Hinge)

In a variety of embodiments, a CL amino acid sequence is connected through its C-terminus to a hinge region, which in turn is connected to the N-terminus of a CH2 domain. Hinge region sequences are described in more detail above in Section 4.3.19.3. In a preferred embodiment, the hinge region amino acid sequence is SEQ ID NO:56.

### 4.3.19.5. Junctions Connecting CH2 C-terminus to Constant Region Domain

In a variety of embodiments, a CH2 amino acid sequence is connected through its C-terminus to the N-terminus of a constant region domain. Constant regions are described in more detail above in Section 4.3.4. In a preferred embodiment, the CH2 sequence is connected to a CH3 sequence via its endogenous sequence. In other embodiments, the CH2 sequence is connected to a CH1 or CL sequence. Examples discussing connecting a CH2 sequence to a CH1 or CL sequence are described in more detail in U.S. Pat. No. 8,242,247.

### 4.3.19.6. Junctions Connecting Domain O to Domain A or Domain S to Domain H on Trivalent and Tetravalent Molecules

In a variety of embodiments, heavy chains of antibodies (e.g., the first and third polypeptide chains) are extended at their N-terminus to include additional domains that provide additional ABSs. With reference to **Fig. 21****,** **Fig. 26****,** and **Fig. 34****,** in certain embodiments, the C-terminus of the constant region domain amino acid sequence of a domain O and/or a domain S is connected to the N-terminus of the VL amino acid sequence of a domain A and/or a domain H, respectively. In some preferred embodiments, the constant region domain is a CH3 amino acid sequence. In some preferred embodiments, the constant region domain is a CL amino acid sequence. In certain embodiments, the constant region domain is connected to the VL amino acid sequence through a peptide linker. In a preferred embodiment, the peptide linker is a 6 amino acid GSGSGS peptide sequence.

In a variety of embodiments, light chains of antibodies (e.g., the second and fourth polypeptide chains) are extended at their N-terminus to include additional variable domain-constant domain segments of an antibody. In certain embodiments, the constant region domain is a CH1 amino acid sequence.

### 4.4. Specific Bivalent Binding Molecules

With reference to **FIG. 3****,** the binding molecules comprise a first, second, third, and fourth polypeptide chain, wherein (a) the first polypeptide chain comprises a domain A, a domain B, a domain D, and a domain E, wherein the domains are arranged, from N-terminus to C-terminus, in a A-B-D-E orientation, and domain A has a VL amino acid sequence, domain B has a CH3 amino acid sequence, domain D has a CH2 amino acid sequence, and domain E has a CH3 amino acid sequence; (b) the second polypeptide chain comprises a domain F and a domain G, wherein the domains are arranged, from N-terminus to C-terminus, in a F-G orientation, and wherein domain F has a VH amino acid sequence and domain G has a CH3 amino acid sequence; (c) the third polypeptide chain comprises a domain H, a domain I, a domain J, and a domain K, wherein the domains are arranged, from N-terminus to C-terminus, in a H-I-J-K orientation, and wherein domain H has a VL amino acid sequence, domain I has a CL amino acid sequence, domain J has a CH2 amino acid sequence, and domain K has a CH3 amino acid sequence; (d) the fourth polypeptide chain comprises a domain L and a domain M, wherein the domains are arranged, from N-terminus to C-terminus, in a L-M orientation, and wherein domain L has a VH amino acid sequence and domain M has a CH1 amino acid sequence; (e) the first and the second polypeptides are associated through an interaction between the A and the F domains and an interaction between the B and the G domains; (f) the third and the fourth polypeptides are associated through an interaction between the H and the L domains and an interaction between the I and the M domains; and (g) the first and the third polypeptides are associated through an interaction between the D and the J domains and an interaction between the E and the K domains to form the binding molecule.

In certain embodiments, the interaction between the A domain and the F domain form a first antigen binding site specific for a first antigen, and the interaction between the H domain and the L domain form a second antigen binding site specific for a second antigen, and the binding molecule is a bispecific bivalent binding molecule. In certain embodiments, the interaction between the A domain and the F domain form a first antigen binding site specific for a first antigen, and the interaction between the H domain and the L domain form a second antigen binding site specific for the first antigen, and the binding molecule is a monospecific bivalent binding molecule.

### 4.4.1. Bivalent Bispecific B-Body "BC1"

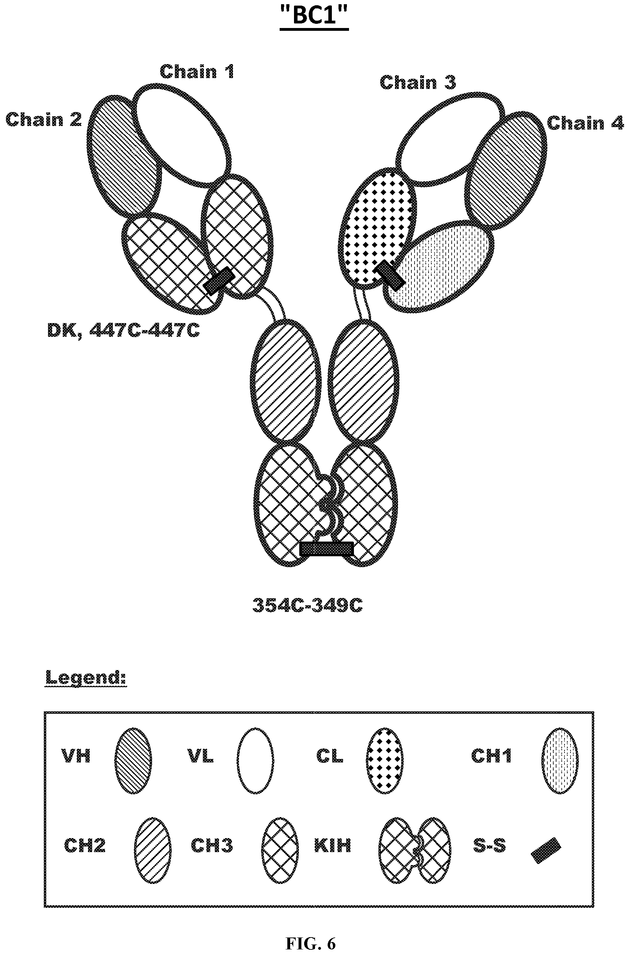

With reference to **FIG. 3** and **FIG. 6****,** in a series of embodiments, the binding molecule has a first, second, third, and fourth polypeptide chain, wherein (a) the first polypeptide chain comprises a domain A, a domain B, a domain D, and a domain E, wherein the domains are arranged, from N-terminus to C-terminus, in a A-B-D-E orientation, and domain A has a first VL amino acid sequence, domain B has a human IgG1 CH3 amino acid sequence with a T366K mutation and a C-terminal extension incorporating a KSC tripeptide sequence that is followed by the DKTHT motif of an IgG1 hinge region, domain D has a human IgG1 CH2 amino acid sequence, and domain E has human IgG1 CH3 amino acid with a S354C and T366W mutation; (b) the second polypeptide chain has a domain F and a domain G, wherein the domains are arranged, from N-terminus to C-terminus, in a F-G orientation, and wherein domain F has a first VH amino acid sequence and domain G has a human IgG1 CH3 amino acid sequence with a L351D mutation and a C-terminal extension incorporating a GEC amino acid disulfide motif; (c) the third polypeptide chain has a domain H, a domain I, a domain J, and a domain K, wherein the domains are arranged, from N-terminus to C-terminus, in a H-I-J-K orientation, and wherein domain H has a second VL amino acid sequence, domain I has a human CL kappa amino acid sequence, domain J has a human IgG1 CH2 amino acid sequence, and K has a human IgG1 CH3 amino acid sequence with a Y349C, a D356E, a L358M, a T366S, a L368A, and a Y407V mutation; (d) the fourth polypeptide chain has a domain L and a domain M, wherein the domains are arranged, from N-terminus to C-terminus, in a L-M orientation, and wherein domain L has a second VH amino acid sequence and domain M has a human IgG1 CH1 amino acid sequence; (e) the first and the second polypeptides are associated through an interaction between the A and the F domains and an interaction between the B and the G domains; (f) the third and the fourth polypeptides are associated through an interaction between the H and the L domains and an interaction between the I and the M domains; (g) the first and the third polypeptides are associated through an interaction between the D and the J domains and an interaction between the E and the K domains to form the binding molecule; (h) domain A and domain F form a first antigen binding site specific for a first antigen; and (i) domain H and domain L form a second antigen binding site specific for a second antigen.

In preferred embodiments, the first polypeptide chain has the sequence SEQ ID NO:8, the second polypeptide chain has the sequence SEQ ID NO:9, the third polypeptide chain has the sequence SEQ ID NO:10, and the fourth polypeptide chain has the sequence SEQ ID NO:11.

### 4.4.2. Bivalent Bispecific B-Body "BC6"

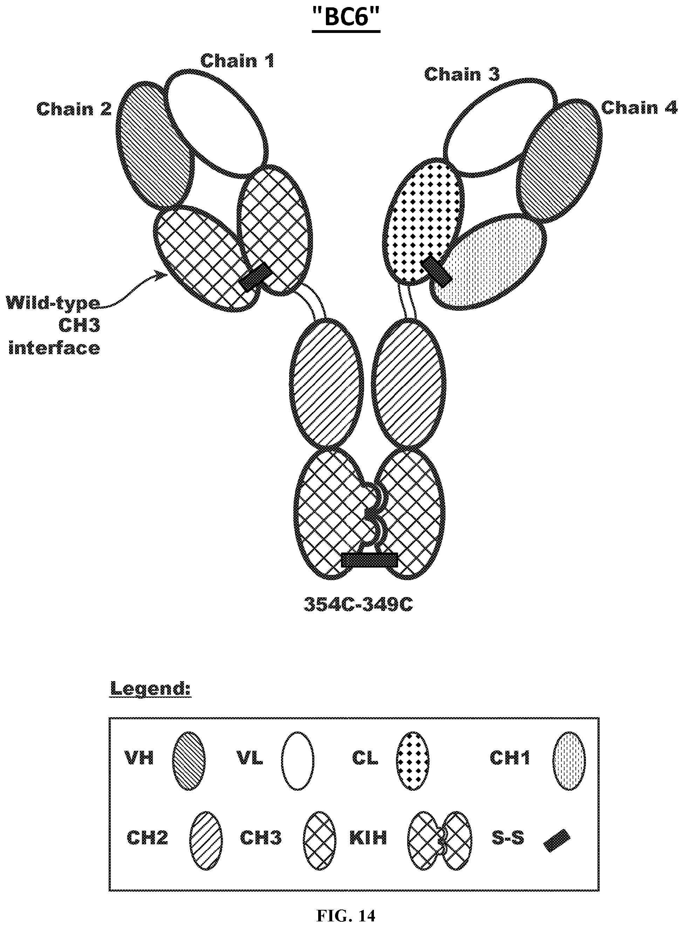

With reference to **FIG. 3** and **FIG. 14****,** in a series of embodiments, the binding molecule has a first, second, third, and fourth polypeptide chain, wherein (a) the first polypeptide chain comprises a domain A, a domain B, a domain D, and a domain E, wherein the domains are arranged, from N-terminus to C-terminus, in a A-B-D-E orientation, and domain A has a first VL amino acid sequence, domain B has a human IgG1 CH3 amino acid sequence with a C-terminal extension incorporating a KSC tripeptide sequence that is followed by the DKTHT motif of an IgG1 hinge region, domain D has a human IgG1 CH2 amino acid sequence, and domain E has human IgG1 CH3 amino acid with a S354C and a T366W mutation; (b) the second polypeptide chain has a domain F and a domain G, wherein the domains are arranged, from N-terminus to C-terminus, in a F-G orientation, and wherein domain F has a first VH amino acid sequence and domain G has a human IgG1 CH3 amino acid sequence with a C-terminal extension incorporating a GEC amino acid disulfide motif; (c) the third polypeptide chain has a domain H, a domain I, a domain J, and a domain K, wherein the domains are arranged, from N-terminus to C-terminus, in a H-I-J-K orientation, and wherein domain H has a second VL amino acid sequence, domain I has a human CL kappa amino acid sequence, domain J has a human IgG1 CH2 amino acid sequence, and K has a human IgG1 CH3 amino acid sequence with a Y349C, a D356E, a L358M, a T366S, a L368A, and a Y407V mutation; (d) the fourth polypeptide chain has a domain L and a domain M, wherein the domains are arranged, from N-terminus to C-terminus, in a L-M orientation, and wherein domain L has a second VH amino acid sequence and domain M has a human IgG1 amino acid sequence; (e) the first and the second polypeptides are associated through an interaction between the A and the F domains and an interaction between the B and the G domains; (f) the third and the fourth polypeptides are associated through an interaction between the H and the L domains and an interaction between the I and the M domains; (g) the first and the third polypeptides are associated through an interaction between the D and the J domains and an interaction between the E and the K domains to form the binding molecule; (h) domain A and domain F form a first antigen binding site specific for a first antigen; and (i) domain H and domain L form a second antigen binding site specific for a second antigen.

### 4.4.3. Bivalent Bispecific B-Body "BC28"

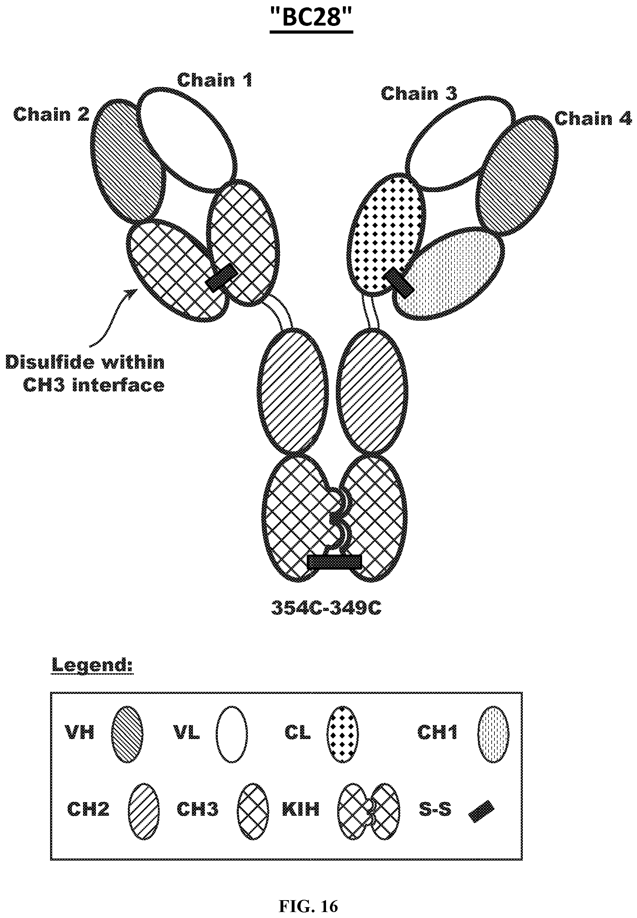

With reference to **FIG. 3** and **FIG. 16****,** in a series of embodiments, the binding molecule has a first, second, third, and fourth polypeptide chain, wherein (a) the first polypeptide chain comprises a domain A, a domain B, a domain D, and a domain E, wherein the domains are arranged, from N-terminus to C-terminus, in a A-B-D-E orientation, and domain A has a first VL amino acid sequence, domain B has a human IgG1 CH3 amino acid sequence with a Y349C mutation and a C-terminal extension incorporating a PGK tripeptide sequence that is followed by the DKTHT motif of an IgG1 hinge region, domain D has a human IgG1 CH2 amino acid sequence, and domain E has a human IgG1 CH3 amino acid with a S354C and a T366W mutation; (b) the second polypeptide chain has a domain F and a domain G, wherein the domains are arranged, from N-terminus to C-terminus, in a F-G orientation, and wherein domain F has a first VH amino acid sequence and domain G has a human IgG1 CH3 amino acid sequence with a S354C mutation and a C-terminal extension incorporating a PGK tripeptide sequence; (c) the third polypeptide chain has a domain H, a domain I, a domain J, and a domain K, wherein the domains are arranged, from N-terminus to C-terminus, in a H-I-J-K orientation, and wherein domain H has a second VL amino acid sequence, domain I has a human CL kappa amino acid sequence, domain J has a human IgG1 CH2 amino acid sequence, and K has a human IgG1 CH3 amino acid sequence with a - Y349C, a D356E, a L358M, a T366S, a L368A, and a Y407V; (d) the fourth polypeptide chain has a domain L and a domain M, wherein the domains are arranged, from N-terminus to C-terminus, in a L-M orientation, and wherein domain L has a second VH amino acid sequence and domain M has a human IgG1 CH1 amino acid sequence; (e) the first and the second polypeptides are associated through an interaction between the A and the F domains and an interaction between the B and the G domains; (f) the third and the fourth polypeptides are associated through an interaction between the H and the L domains and an interaction between the I and the M domains; (g) the first and the third polypeptides are associated through an interaction between the D and the J domains and an interaction between the E and the K domains to form the binding molecule; (h) domain A and domain F form a first antigen binding site specific for a first antigen; and (i) domain H and domain L form a second antigen binding site specific for a second antigen.

In preferred embodiments, the first polypeptide chain has the sequence SEQ ID NO:24, the second polypeptide chain has the sequence SEQ ID NO:25, the third polypeptide chain has the sequence SEQ ID NO:10, and the fourth polypeptide chain has the sequence SEQ ID NO:11.

### 4.4.4. Bivalent Bispecific B-Body "BC44"

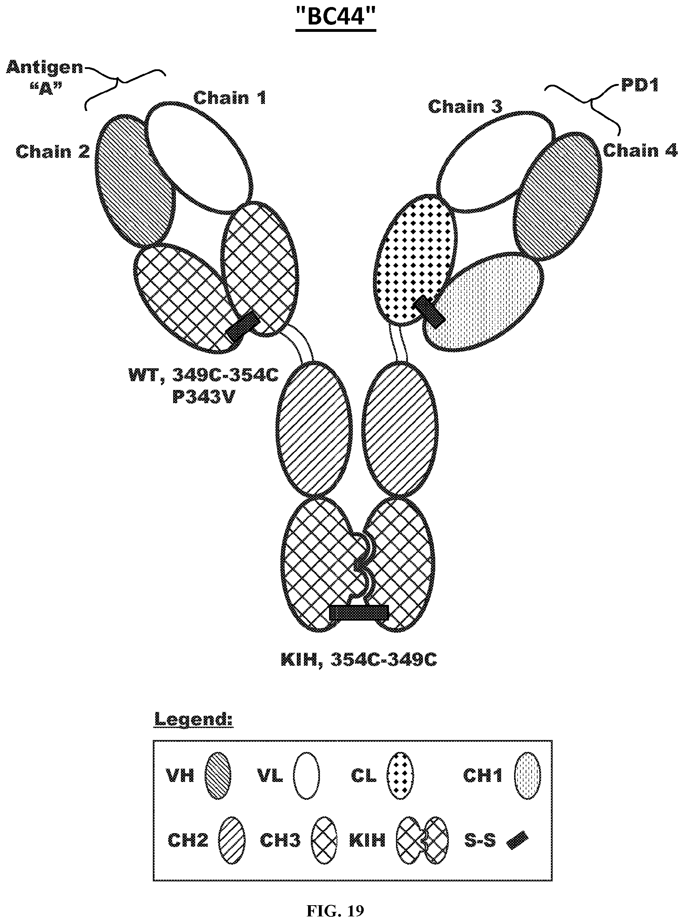

With reference to **FIG. 3** and **FIG. 19****,** in a series of embodiments, the binding molecule has a first, second, third, and fourth polypeptide chain, wherein (a) the first polypeptide chain comprises a domain A, a domain B, a domain D, and a domain E, wherein the domains are arranged, from N-terminus to C-terminus, in a A-B-D-E orientation, and domain A has a first VL amino acid sequence, domain B has a human IgG1 CH3 amino acid sequence with a Y349C mutation, a P343V mutation, and a C-terminal extension incorporating a PGK tripeptide sequence that is followed by the DKTHT motif of an IgG1 hinge region, domain D has a human IgG1 CH2 amino acid sequence, and domain E has human IgG1 CH3 amino acid with a S354C mutation and a T366W mutation; (b) the second polypeptide chain has a domain F and a domain G, wherein the domains are arranged, from N-terminus to C-terminus, in a F-G orientation, and wherein domain F has a first VH amino acid sequence and domain G has a human IgG1 CH3 amino acid sequence with a S354C mutation and a C-terminal extension incorporating a PGK tripeptide sequence; (c) the third polypeptide chain has a domain H, a domain I, a domain J, and a domain K, wherein the domains are arranged, from N-terminus to C-terminus, in a H-I-J-K orientation, and wherein domain H has a second VL amino acid sequence, domain I has a human CL kappa amino acid sequence, domain J has a human IgG1 CH2 amino acid sequence, and K has a human IgG1 CH3 amino acid sequence with aY349C, T366S, L368A, and aY407V; (d) the fourth polypeptide chain has a domain L and a domain M, wherein the domains are arranged, from N-terminus to C-terminus, in a L-M orientation, and wherein domain L has a second VH amino acid sequence and domain M has a human IgG1 amino acid sequence; (e) the first and the second polypeptides are associated through an interaction between the A and the F domains and an interaction between the B and the G domains; (f) the third and the fourth polypeptides are associated through an interaction between the H and the L domains and an interaction between the I and the M domains; and (g) the first and the third polypeptides are associated through an interaction between the D and the J domains and an interaction between the E and the K domains to form the binding molecule; (h) domain A and domain F form a first antigen binding site specific for a first antigen; and (i) domain H and domain L form a second antigen binding site specific for a second antigen.

In preferred embodiments, the first polypeptide chain has the sequence SEQ ID NO:32, the second polypeptide chain has the sequence SEQ ID NO:25, the third polypeptide chain has the sequence SEQ ID NO:10, and the fourth polypeptide chain has the sequence SEQ ID NO:11.

### 4.5. Specific Trivalent Binding Molecules

### 4.5.1. Trivalent 1x2 Bispecific B-Body "BC28-1x2"

With reference to Section 4.4.3. and **FIG. 26****,** in a series of embodiments, the binding molecules further comprise a sixth polypeptide chain, wherein (a) the third polypeptide chain further comprises a domain R and a domain S, wherein the domains are arranged, from N-terminus to C-terminus, in a R-S-H-I-J-K orientation, and wherein domain R has the first VL amino acid sequence and domain S has a human IgG1 CH3 amino acid sequence with a Y349C mutation and a C-terminal extension incorporating a PGK tripeptide sequence that is followed by GSGSGS linker peptide connecting domain S to domain H; (b) the binding molecule further comprises a sixth polypeptide chain, comprising: a domain T and a domain U, wherein the domains are arranged, from N-terminus to C-terminus, in a T-U orientation, and wherein domain T has the first VH amino acid sequence and domain U has a human IgG1 CH3 amino acid sequence with a S354C mutation and a C-terminal extension incorporating a PGK tripeptide sequence; (c) the third and the sixth polypeptides are associated through an interaction between the R and the T domains and an interaction between the S and the U domains to form the binding molecule, and (d) domain R and domain T form a third antigen binding site specific for the first antigen.

In preferred embodiments, the first polypeptide chain has the sequence SEQ ID NO:24, the second polypeptide chain has the sequence SEQ ID NO:25, the third polypeptide chain has the sequence SEQ ID NO:37, the fourth polypeptide chain has the sequence SEQ ID NO:11, and the sixth polypeptide chain has the sequence SEQ ID NO:25.

### 4.5.2. Trivalent 1x2 Trispecific B-Body "BC28-1x1x1a"

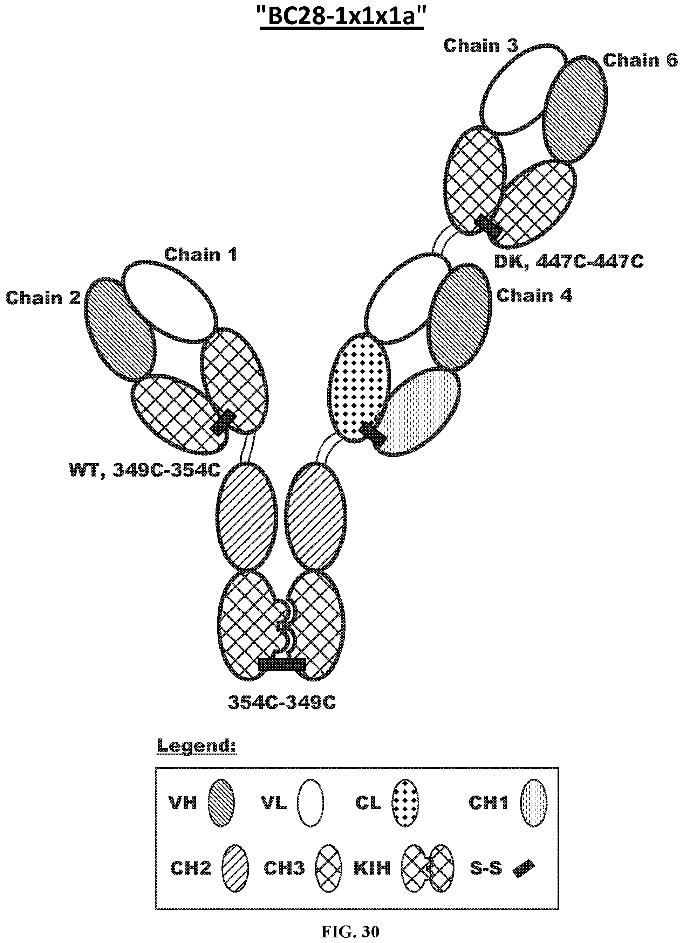

With reference to Section 4.4.3. and **FIG. 26** and **FIG. 30****,** in a series of embodiments, the binding molecules further comprise a sixth polypeptide chain, wherein (a) the third polypeptide chain further comprises a domain R and a domain S, wherein the domains are arranged, from N-terminus to C-terminus, in a R-S-H-I-J-K orientation, and wherein domain R has a third VL amino acid sequence and domain S has a human IgG1 CH3 amino acid sequence with a T366K mutation and a C-terminal extension incorporating a KSC tripeptide sequence that is followed by GSGSGS linker peptide connecting domain S to domain H; (b) the binding molecule further comprises a sixth polypeptide chain, comprising: a domain T and a domain U, wherein the domains are arranged, from N-terminus to C-terminus, in a T-U orientation, and wherein domain T has a third VH amino acid sequence and domain U has a human IgG1 CH3 amino acid sequence with a L351D mutation and a C-terminal extension incorporating a GEC amino acid disulfide motif; and (c) the third and the sixth polypeptides are associated through an interaction between the R and the T domains and an interaction between the S and the U domains to form the binding molecule, and (d) domain R and domain T form a third antigen binding site specific for a third antigen.

In preferred embodiments, the first polypeptide chain has the sequence SEQ ID NO:24, the second polypeptide chain has the sequence SEQ ID NO:25, the third polypeptide chain has the sequence SEQ ID NO:45, the fourth polypeptide chain has the sequence SEQ ID NO:11, and the sixth polypeptide chain has the sequence SEQ ID NO: 53.

### 4.6. Antigen specificities

The antigen binding sites of the binding molecules described herein may be chosen to specifically bind a wide variety of molecular targets. For example, an antigen binding site or sites may specifically bind E-Cad, CLDN7, FGFR2b, N-Cad, Cad-11, FGFR2c, ERBB2, ERBB3, FGFR1, FOLR1, IGF-Ira, GLP1R, PDGFRa, PDGFRb, EPHB6, ABCG2, CXCR4, CXCR7, Integrin-avb3, SPARC, VCAM, ICAM, Annexin, ROR1, ROR2, TNFα, CD137, angiopoietin 2, angiopoietin 3, BAFF, beta amyloid, C5, CA-125, CD147, CD125, CD147, CD152, CD19, CD20, CD22, CD23, CD24, CD25, CD274, CD28, CD3, CD30, CD33, CD37, CD4, CD40, CD44, CD44v4, CD44v6, CD44v7, CD50, CD51, CD52, CEA, CSF1R, CTLA-2, DLL4, EGFR, EPCAM, HER3, GD2 ganglioside, GDF-8, Her2/neu, CD2221, IL-17A, IL-12, IL-23, IL-13, IL-6, IL-23, an integrin, CD11a, MUC1, Notch, TAG-72, TGFβ, TRAIL-R2, VEGF-A, VEGFR-1, VEGFR2, VEGFc, hematopoietins (four-helix bundles) (such as EPO (erythropoietin), IL-2 (T-cell growth factor), IL-3 (multicolony CSF), IL-4 (BCGF-1, BSF-1), IL-5 (BCGF-2), IL-6 IL-4 (IFN-β2, BSF-2, BCDF), IL-7, IL-8, IL-9, IL-11, IL-13 (P600), G-CSF, IL-15 (T-cell growth factor), GM-CSF (granulocyte macrophage colony stimulating factor), OSM (OM, oncostatin M), and LIF (leukemia inhibitory factor)); interferons (such as IFN-y, IFN-α, and IFN-β); immunoglobin superfamily (such as B7.1 (CD80), and B7.2 (B70, CD86)); TNF family (such as TNF-α (cachectin), TNF-β (lymphotoxin, LT, LT-α), LT-β, Fas, CD27, CD30, and 4-1BBL); and those unassigned to a particular family (such as TGF-β, IL 1α, IL-1β, IL-1 RA, IL-10 (cytokine synthesis inhibitor F), IL-12 (NK cell stimulatory factor), MIF, IL-16, IL-17 (mCTLA-8), and/or IL-18 (IGIF, interferon-y inducing factor)); in embodiments relating to bispecific antibodies, the antibody may for example bind two of these targets. Furthermore, the Fc portion of the heavy chain of an antibody may be used to target Fc receptor-expressing cells such as the use of the Fc portion of an IgE antibody to target mast cells and basophils.

An antigen binding site or sites may be chosen that specifically binds the TNF family of receptors including, but not limited to, TNFR1 (also known as CD120a and TNFRSF1A), TNFR2 (also known as CD120b and TNFRSF1B), TNFRSF3 (also known as LTβR), TNFRSF4 (also known as OX40 and CD134), TNFRSF5 (also known as CD40), TNFRSF6 (also known as FAS and CD95), TNFRSF6B (also known as DCR3), TNFRSF7 (also known as CD27), TNFRSF8 (also known as CD30), TNFRSF9 (also known as 4-1BB), TNFRSF10A (also known as TRAILR1, DR4, and CD26), TNFRSF10B (also known as TRAILR2, DR5, and CD262), TNFRSF10C (also known as TRAILR3, DCR1, CD263), TNFRSF10D (also known as TRAILR4, DCR2, and CD264), TNFRSF11A (also known as RANK and CD265), TNFRSF11B (also known as OPG), TNFRSF12A (also known as FN14, TWEAKR, and CD266), TNFRSF13B (also known as TACI and CD267), TNFRSF13C (also known as BAFFR, BR3, and CD268), TNFRSF14 (also known as HVEM and CD270), TNFRSF16 (also known as NGFR, p75NTR, and CD271), or TNFRSF17 (also known as BCMA and CD269), TNFRSF18 (also known as GITR and CD357), TNFRSF19 (also known as TROY, TAJ, and TRADE), TNFRSF21 (also known as CD358), TNFRSF25 (also known as Apo-3, TRAMP, LARD, or WS-1), EDA2R (also known as XEDAR).

An antigen binding site or sites may be chosen that specifically binds immune-oncology targets including, but not limited to, checkpoint inhibitor targets such as PD1, PDL1, CTLA-4, PDL2, B7-H3, B7-H4, BTLA, TIM3, GAL9, LAG3, VISTA, KIR, 2B4, BY55, and CGEN-15049.

In a series of embodiments, an antigen binding site or sites may be chosen that specifically target tumor-associated cells. In various embodiments, the antigen binding site or sites specifically target tumor associated immune cells. In certain embodiments, the antigen binding site or sites specifically target tumor associated regulatory T cells (Tregs). In specific embodiments, a binding molecule has antigen binding sites specific for antigens selected from one or more of CD25, OX40, CTLA-4, and NRP1 such that the binding molecule specifically targets tumor associated regulatory T cells. In specific embodiments, a binding molecule has antigen binding sites that specifically bind CD25 and OX40, CD25 and CTLA-4, CD25 and NRP1, OX40 and CTLA-4, OX40 and NRP1, or CTLA-4 and NRP1 such that the binding molecule specifically targets tumor associated regulatory T cells. In preferred embodiments, a bispecific bivalent binding molecule has antigen binding sites that specifically bind CD25 and OX40, CD25 and CTLA-4, CD25 and NRP1, OX40 and CTLA-4, OX40 and NRP1, or CTLA-4 and NRP1 such that the binding molecule specifically targets tumor associated regulatory T cells. In specific embodiments, the specific targeting of the tumor associated regulatory T cells results in depletion (e.g., killing) of the regulatory T cells. In preferred embodiments, the depletion of the regulatory T cells is mediated by an antibody-drug conjugate (ADC) modification, such as an antibody conjugated to a toxin, as discussed in more detail below in Section 4.7.1.

In a series of embodiments, a binding molecule has antigen binding sites selected from one or more of CD3, ROR1, and ROR2. In a specific embodiment, a bispecific bivalent has antigen binding sites that specifically bind CD3 and ROR1. In a specific embodiment, a bispecific bivalent has antigen binding sites that specifically bind CD3 and ROR2. In a specific embodiment, a trispecific trivalent has antigen binding sites that specifically bind CD3, ROR1, and ROR2.

### 4.7. Further modifications

In a further series of embodiments, the binding molecule has additional modifications.

### 4.7.1. Binding Molecule-Drug Conjugates

In various embodiments, the binding molecule is conjugated to a therapeutic agent (i.e., drug) to form a binding molecule-drug conjugate. Therapeutic agents include, but are not limited to, chemotherapeutic agents, imaging agents (e.g., radioisotopes), immune modulators (e.g., cytokines, chemokines, or checkpoint inhibitors), and toxins (e.g., cytotoxic agents). In certain embodiments, the therapeutic agents are attached to the binding molecule through a linker peptide, as discussed in more detail below in Section 4.7.3. Methods of preparing antibody-drug conjugates (ADCs) that can be adapted to conjugate drugs to the binding molecules disclosed herein are described, e.g., in U.S. Patent No. 8,624,003 (pot method), U.S. Patent No. 8,163,888 (one-step), U.S. Patent No. 5,208,020 (two-step method), U.S. Patent No. 8,337,856, U.S. Patent No. 5,773,001, U.S. Patent No. 7,829,531, U.S. Patent No. 5,208,020, U.S. Patent No. 7,745,394, WO 2017/136623, WO 2017/015502, WO 2017/015496, WO 2017/015495, WO 2004/010957, WO 2005/077090, WO 2005/082023, WO 2006/065533, WO 2007/030642, WO 2007/103288, WO 2013/173337, WO 2015/057699, WO 2015/095755, WO 2015/123679, WO 2015/157286, WO 2017/165851, WO 2009/073445, WO 2010/068759, WO 2010/138719 , WO 2012/171020, WO 2014/008375, WO 2014/093394, WO 2014/093640, WO 2014/160360, WO 2015/054659, WO 2015/195925, WO 2017/160754, Storz (MAbs. 2015 Nov-Dec; 7(6): 989-1009), Lambert et al. (Adv Ther, 2017 34: 1015), Diamantis et al. (British Journal of Cancer, 2016, 114, 362-367), Carrico et al. (Nat Chem Biol, 2007. 3: 321-2), We et al. (Proc Natl Acad Sci USA, 2009. 106: 3000-5), Rabuka et al. (Curr Opin Chem Biol., 2011 14: 790-6), Hudak et al. (Angew Chem Int Ed Engl., 2012: 4161-5), Rabuka et al. (Nat Protoc., 2012 7:1052-67), Agarwal et al. (Proc Natl Acad Sci USA., 2013, 110: 46-51), Agarwal et al. (Bioconjugate Chem., 2013, 24: 846-851), Barfield et al. (Drug Dev. and D., 2014, 14:34-41), Drake et al. (Bioconjugate Chem., 2014, 25:1331-41), Liang et al. (J Am Chem Soc., 2014, 136:10850-3), Drake et al. (Curr Opin Chem Biol., 2015, 28:174-80), and York et al. (BMC Biotechnology, 2016, 16(1):23).

### 4.7.2. Additional Binding Moieties

In various embodiments, the binding molecule has modifications that comprise one or more additional binding moieties. In certain embodiments the binding moieties are antibody fragments or antibody formats including, but not limited to, full-length antibodies, Fab fragments, Fvs, scFvs, tandem scFvs, Diabodies, scDiabodies, DARTs, tandAbs, minibodies, camelid VHH, and other antibody fragments or formats known to those skilled in the art. Exemplary antibody and antibody fragment formats are described in detail in Brinkmann et al. (MABS, 2017, Vol. 9, No. 2, 182-212).

In particular embodiments, the one or more additional binding moieties are attached to the C-terminus of the first or third polypeptide chain. In particular embodiments, the one or more additional binding moieties are attached to the C-terminus of both the first and third polypeptide chain. In particular embodiments, the one or more additional binding moieties are attached to the C-terminus of both the first and third polypeptide chains. In certain embodiments, individual portions of the one or more additional binding moieties are separately attached to the C-terminus of the first and third polypeptide chains such that the portions form the functional binding moiety.

In particular embodiments, the one or more additional binding moieties are attached to the N-terminus of any of the polypeptide chains (e.g., the first, second, third, fourth, fifth, or sixth polypeptide chains). In certain embodiments, individual portions of the additional binding moieties are separately attached to the N-terminus of different polypeptide chains such that the portions form the functional binding moiety.

In certain embodiments, the one or more additional binding moieties are specific for a different antigen or epitope of the ABSs within the binding molecule. In certain embodiments, the one or more additional binding moieties are specific for the same antigen or epitope of the ABSs within the binding molecule. In certain embodiments, wherein the modification is two or more additional binding moieties, the additional binding moieties are specific for the same antigen or epitope. In certain embodiments, wherein the modification is two or more additional binding moieties, the additional binding moieties are specific for different antigens or epitopes.

In certain embodiments, the one or more additional binding moieties are attached to the binding molecule using *in vitro* methods including, but not limited to, reactive chemistry and affinity tagging systems, as discussed in more detail below in Section 4.7.3. In certain embodiments, the one or more additional binding moieties are attached to the binding molecule through Fc-mediated binding (e.g., Protein A/G). In certain embodiments, the one or more additional binding moieties are attached to the binding molecule using recombinant DNA techniques, such as encoding the nucleotide sequence of the fusion product between the binding molecule and the additional binding moieties on the same expression vector (e.g., plasmid).

### 4.7.3. Functional/Reactive Groups

In various embodiments, the binding molecule has modifications that comprise functional groups or chemically reactive groups that can be used in downstream processes, such as linking to additional moieties (e.g. drug conjugates and additional binding moieties, as discussed in more detail above in Sections 4.7.1. and 4.7.2.) and downstream purification processes.

In certain embodiments, the modifications are chemically reactive groups including, but not limited to, reactive thiols (e.g., maleimide based reactive groups), reactive amines (e.g., N-hydroxysuccinimide based reactive groups), "click chemistry" groups (e.g., reactive alkyne groups), and aldehydes bearing formylglycine (FGly). In certain embodiments, the modifications are functional groups including, but not limited to, affinity peptide sequences (e.g., HA, HIS, FLAG, GST, MBP, and Strep systems etc.). In certain embodiments, the functional groups or chemically reactive groups have a cleavable peptide sequence. In particular embodiments, the cleavable peptide is cleaved by means including, but not limited to, photocleavage, chemical cleavage, protease cleavage, reducing conditions, and pH conditions. In particular embodiments, protease cleavage is carried out by intracellular proteases. In particular embodiments, protease cleavage is carried out by extracellular or membrane associated proteases. ADC therapies adopting protease cleavage are described in more detail in Choi et al. (Theranostics, 2012; 2(2): 156-178.).

### 4.8. Pharmaceutical compositions

In another (non-claimed) aspect, pharmaceutical compositions are provided that comprise a binding molecule as described herein and a pharmaceutically acceptable carrier or diluent. In typical embodiments, the pharmaceutical composition is sterile.

In various embodiments, the pharmaceutical composition comprises the binding molecule at a concentration of 0.1 mg/mL - 100 mg/mL. In specific embodiments, the pharmaceutical composition comprises the binding molecule at a concentration of 0.5 mg/mL, 1 mg/mL, 1.5 mg/mL, 2 mg/mL, 2.5 mg/mL, 5 mg/mL, 7.5 mg/mL, or 10 mg/mL. In some embodiments, the pharmaceutical composition comprises the binding molecule at a concentration of more than 10 mg/mL. In certain embodiments, the binding molecule is present at a concentration of 20 mg/mL, 25 mg/mL, 30 mg/mL, 35 mg/mL, 40 mg/mL, 45 mg/mL, or even 50 mg/mL or higher. In particular embodiments, the binding molecule is present at a concentration of more than 50 mg/mL.

In various embodiments, the pharmaceutical compositions are described in more detail in U.S. Pat No. 8,961,964, U.S. Pat No. 8,945,865, U.S. Pat No. 8,420,081, U.S. Pat No. 6,685,940, U.S. Pat No. 6,171,586, U.S. Pat No. 8,821,865, U.S. Pat No. 9,216,219, US application 10/813,483, WO 2014/066468, WO 2011/104381, and WO 2016/180941.

### 4.9. Methods of Manufacturing

The binding molecules described herein can readily be manufactured by expression using standard cell free translation, transient transfection, and stable transfection approaches currently used for antibody manufacture. In specific embodiments, Expi293 cells (ThermoFisher) can be used for production of the binding molecules using protocols and reagents from ThermoFisher, such as ExpiFectamine, or other reagents known to those skilled in the art, such as polyethylenimine as described in detail in Fang et al. (Biological Procedures Online, 2017, 19:11).

As further described in the Examples below, the expressed proteins can be readily purified using a CH1 affinity resin, such as the CaptureSelect CH1 resin and provided protocol from ThermoFisher. Further purification can be effected using ion exchange chromatography as is routinely used in the art.

### 4.10. Methods of Treatment

In another (non-claimed) aspect, methods of treatment are provided, the methods comprising administering a binding molecule as described herein to a patient in an amount effective to treat the patient.

In some embodiments, an antibody of the present disclosure may be used to treat a cancer. The cancer may be a cancer from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In some embodiments, the cancer may be a neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; bronchioloalveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; non encapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; Paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; Sertoli cell carcinoma; Leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malig melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; Brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; Kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; Ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

An antibody of the present disclosure may be administered to a subject per se or in the form of a pharmaceutical composition for the treatment of, e.g., cancer, autoimmunity, transplantation rejection, post-traumatic immune responses, graft-versus-host disease, ischemia, stroke, and infectious diseases, for example by targeting viral antigens, such as gp120 of HIV.

### 4.11. Examples

The following examples are provided by way of illustration.

### 4.11.1. Example 1: Bivalent monospecific construct and bivalent bispecific construct

A bivalent monospecific B-Body recognizing TNFa was constructed with the following architecture (VL(Certolizumab)-CH3(Knob)-CH2-CH3/VH(Certolizumab)-CH3(Hole)) using standard molecular biology procedures. In this construct,
1^{st} polypeptide chain (SEQ ID NO: 1)
   Domain A = VL (certolizumab)
   Domain B = CH3 (IgG1) (knob: S354C+T366W)
   Domain D = CH2 (IgG1)
   Domain E =CH3 (IgG1)
2^{nd} polypeptide chain (SEQ ID NO:2)
   Domain F = VH (certolizumab)
   Domain G = CH3 (IgG1) (hole:Y349C, T366S, L368A, Y407V)
3^{rd} polypeptide chain:
   identical to the 1^{st} polypeptide chain
4^{th} polypeptide chain:
   identical to the 2^{nd} polypeptide chain.

Domain and polypeptide chain references are in accordance with FIG. 3. The overall construct architecture is illustrated in FIG. 4. The sequence of the first polypeptide chain, with domain A identified in shorthand as "(VL)", is provided in SEQ ID NO:1. The sequence of the second polypeptide chain, with domain F identified in shorthand as "(VH)", is provided in SEQ ID NO:2.

The full-length construct was expressed in an E. coli cell free protein synthesis expression system for ~18 hours at 26 °C with gentle agitation. Following expression, the cell-free extract was centrifuged to pellet insoluble material and the supernatant was diluted 2x with 10x Kinetic Buffer (Forte Bio) and used as analyte for biolayer interferometry.

Biotinylated TNFα was immobilized on a streptavidin sensor to give a wave shift response of ~1.5 nm. After establishing a baseline with 10x kinetic buffer, the sensor was dipped into the antibody construct analyte solution. The construct gave a response of ~3 nm, comparable to the traditional IgG format of certolizumab, demonstrating the ability of the bivalent monospecific construct to assemble into a functional, full-length antibody. Results are shown in FIG. 5.

We also constructed a bivalent bispecific antibody with the following domain architecture:
1^{st} polypeptide chain: VL-CH3-CH2-CH3(Knob)
2^{nd} polypeptide chain: VH-CH3
3^{rd} polypeptide chain: VL-CL-CH2-CH3(Hole)
4^{th} polypeptide chain VH-CH1.

The sequences (except for the variable region sequences) are provided respectively in SEQ ID NO:3 (1st polypeptide chain), SEQ ID NO:4 (2nd polypeptide chain), SEQ ID NO:5 (3rd polypeptide chain), SEQ ID NO:6 (4th polypeptide chain).

### 4.11.2. Example 2: Bivalent bispecific B-Body "BC1"

We constructed a bivalent bispecific construct, termed "BC1", specific for PD1 and a second antigen, "Antigen A"). Salient features of the "BC1" architecture are illustrated in **FIG. 6****.**

In greater detail, with domain and polypeptide chain references in accordance with **FIG. 3** and modifications from native sequence indicated in parentheses, the architecture was:
1^{st} polypeptide chain (SEQ ID NO:8)
   Domain A = VL ("Antigen A")
   Domain B = CH3 (T366K; 445K, 446S, 447C tripeptide insertion)
   Domain D = CH2
   Domain E = CH3 (T366W, S354C)
2^{nd} polypeptide chain (SEQ ID NO:9):
   Domain F = VH ("Antigen A")
   Domain G = CH3 (L351D; 445G, 446E, 447C tripeptide insertion)
3^{rd} polypeptide chain (SEQ ID NO: 10):
   Domain H = VL ("Nivo")
   Domain I = CL (Kappa)
   Domain J = CH2
   Domain K = CH3 (Y349C, D356E, L358M, T366S, L368A, Y407V)
4^{th} polypeptide chain (SEQ ID NO:11):
   Domain L = VH ("Nivo")
   Domain M = CH1.

The A domain (SEQ ID NO: 12) and F domain (SEQ ID NO: 16) form an antigen binding site (A:F) specific for "Antigen A". The H domain has the VH sequence from nivolumab and the L domain has the VL sequence from nivolumab; H and L associate to form an antigen binding site (H:L) specific for human PD1.

The B domain (SEQ ID NO:13) has the sequence of human IgG1 CH3 with several mutations: T366K, 445K, 446S, and 447C insertion. The T366K mutation is a charge pair cognate of the L351D residue in Domain G. The "447C" residue on domain B comes from the C-terminal KSC tripeptide insertion.
Domain D (SEQ ID NO: 14) has the sequence of human IgG1 CH2
Domain E (SEQ ID NO: 15) has the sequence of human IgG1 CH3 with the mutations T366W and S354C. The 366W is the "knob" mutation. The 354C introduces a cysteine that is able to form a disulfide bond with the cognate 349C mutation in Domain K.
Domain G (SEQ ID NO:17) has the sequence of human IgG1 CH3 with the following mutations: L351D, and 445G, 446E, 447C tripeptide insertion. The L351D mutation introduces a charge pair cognate to the Domain B T366K mutation. The "447C" residue on domain G comes from the C-terminal GEC tripeptide insertion.
Domain I (SEQ ID NO: 19) has the sequence of human C kappa light chain (Cκ)
Domain J [SEQ ID NO: 20] has the sequence of human IgG1 CH2 domain, and is identical to the sequence of domain D.
Domain K [SEQ ID NO: 21] has the sequence of human IgG1 CH3 with the following changes: Y349C, D356E, L358M, T366S, L368A, Y407V. The 349C mutation introduces a cysteine that is able to form a disulfide bond with the cognate 354C mutation in Domain E. The 356E and L358M introduce isoallotype amino acids that reduce immunogenicity. The 366S, 368A, and 407V are "hole" mutations.
Domain M [SEQ ID NO: 23] has the sequence of the human IgG1 CH1 region.

"BC1" could readily be expressed at high levels using mammalian expression at concentrations greater than 100 µg/mL.

We found that the bivalent bispecific "BC1" protein could easily be purified in a single step using a CH1-specific CaptureSelect^{™} affinity resin from ThermoFisher.

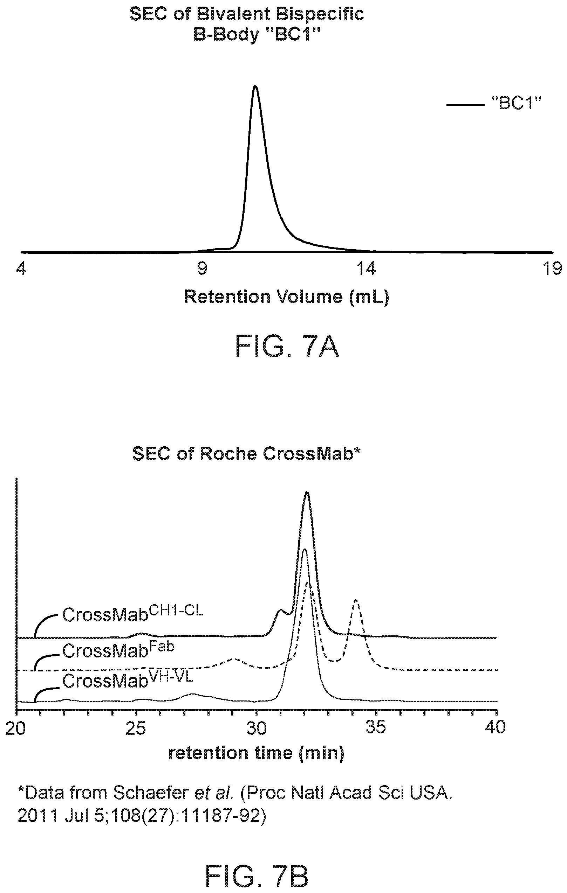

As shown in FIG. 7A, SEC analysis demonstrates that a single-step CH1 affinity purification step yields a single, monodisperse peak via gel filtration in which >98% is monomer. FIG. 7B shows comparative literature data of SEC analysis of a CrossMab bivalent antibody construct.

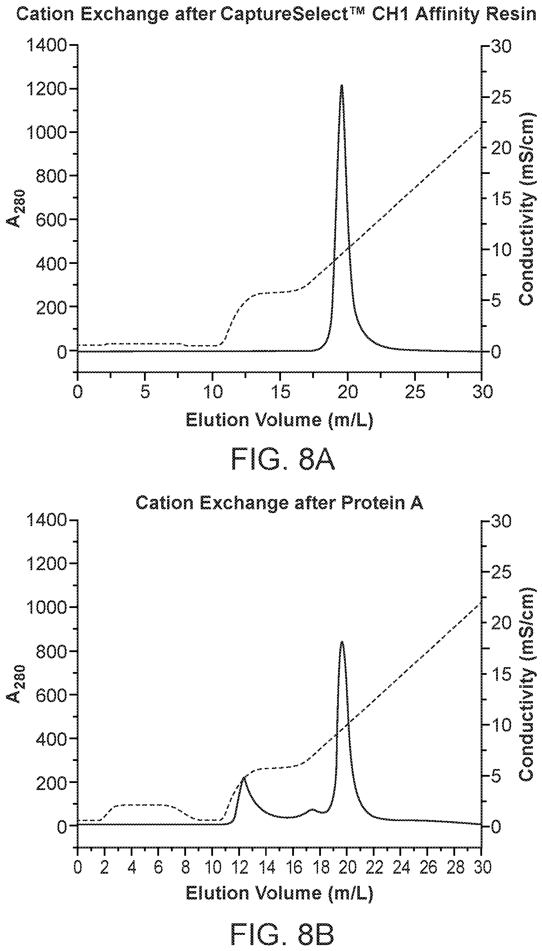

FIG. 8A is a cation exchange chromatography elution profile of "BC1" following one-step purification using the CaptureSelect^{™} CH1 affinity resin, showing a single tight peak. FIG. 8B is a cation exchange chromatography elution profile of "BC1" following purification using standard Protein A purification, showing additional elution peaks consistent with the co-purification of incomplete assembly products.

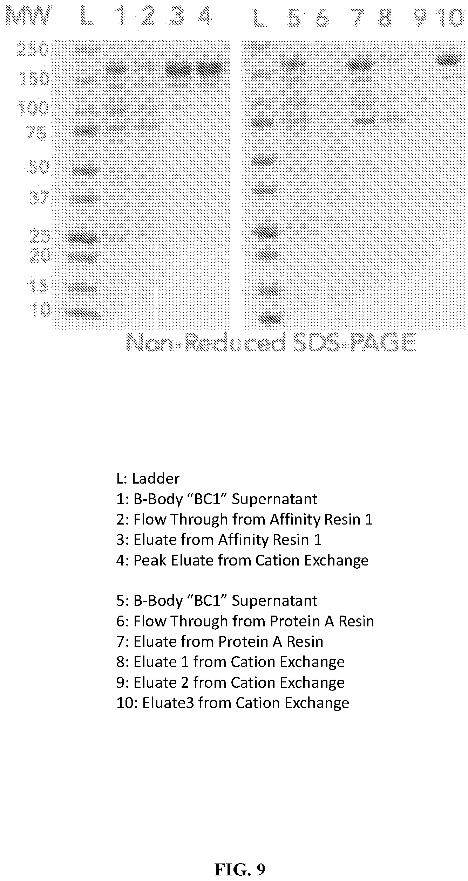

FIG. 9 shows SDS-PAGE gels under non-reducing conditions. As seen in lane 3, single-step purification of "BC1" with CH1 affinity resin provides a nearly homogeneous single band, with lane 4 showing minimal additional purification with a subsequent cation exchange polishing step. Lane 7, by comparison, shows less substantial purification using standard Protein A purification, with lanes 8-10 demonstrating further purification of the Protein A purified material using cation exchange chromatography.

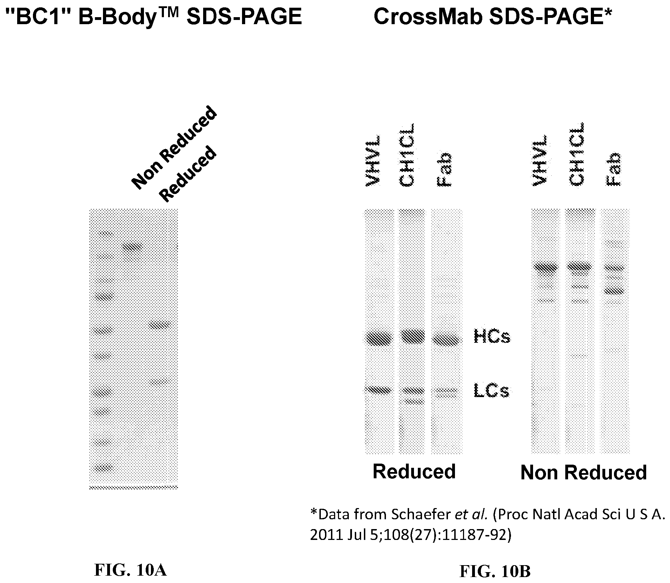

FIG. 10 compares SDS-PAGE gels of "BC1" after single-step CH1-affinity purification, under both non-reducing and reducing conditions (Panel A) with SDS-PAGE gels of a CrossMab bispecific antibody under non-reducing and reducing conditions as published in the referenced literature (Panel B).

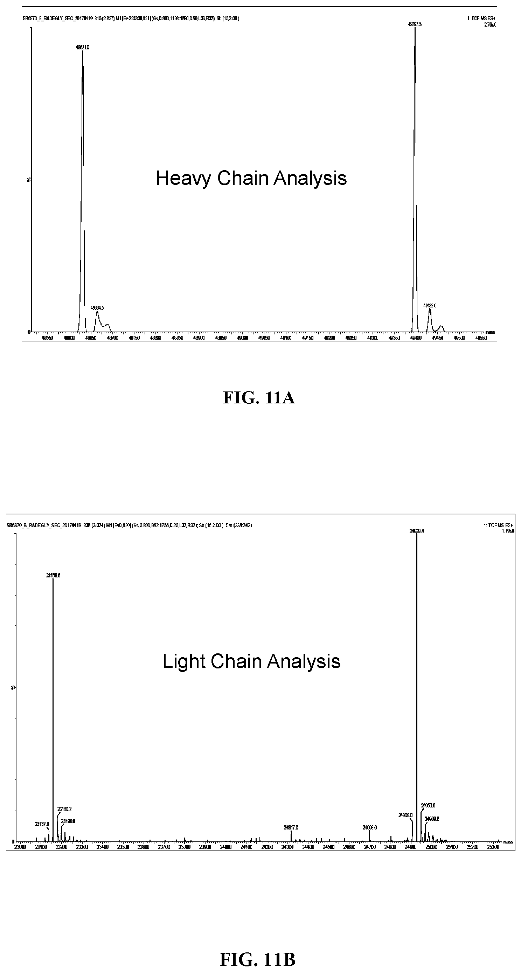

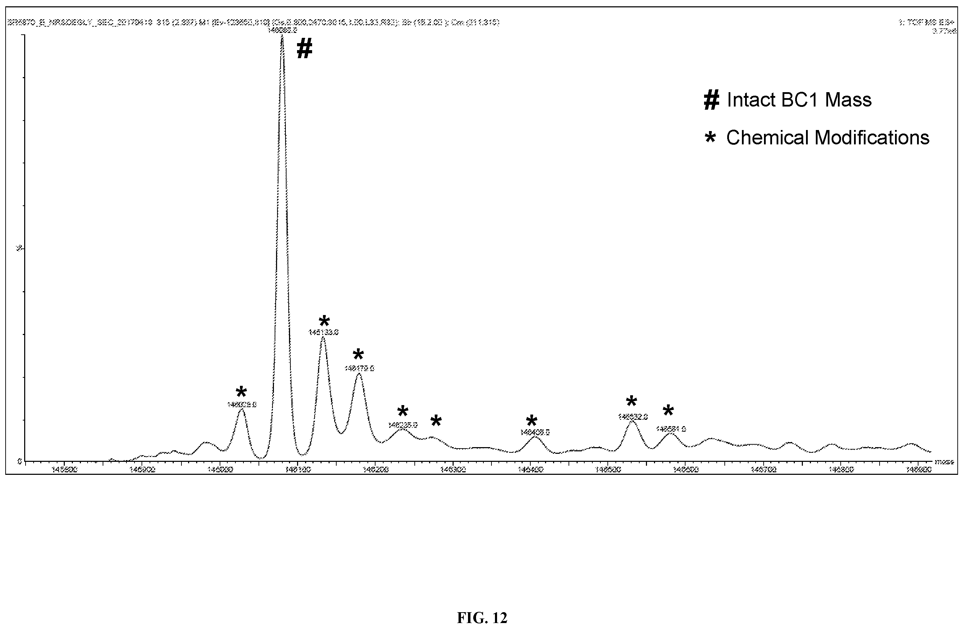

FIG. 11 shows mass spec analysis of "BC1", demonstrating two distinct heavy chains (FIG. 11A) and two distinct light chains (FIG. 11B) under reducing conditions. The mass spectrometry data in FIG. 12 confirms the absence of incomplete pairing after purification.

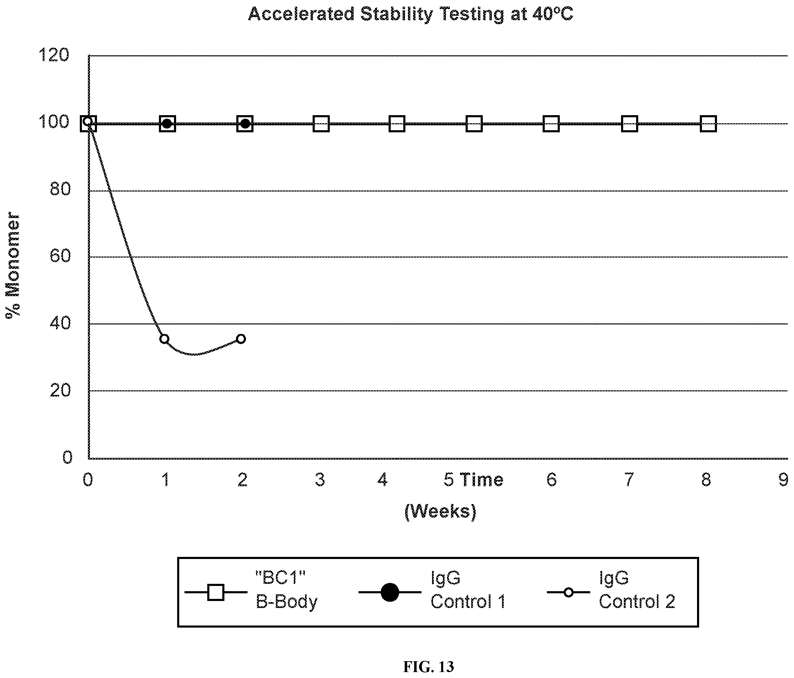

Accelerated stability testing was performed to evaluate the long-term stability of the "BC1" B-Body design. The purified B-Body was concentrated to 8.6 mg/mL in PBS buffer and incubated at 40°C. The structural integrity was measured weekly using analytical size exclusion chromatography (SEC) with a Shodex KW-803 column. The structural integrity was determined by measuring the percentage of intact monomer (% Monomer) in relation to the formation of aggregates. Data are shown in FIG. 13. The IgG Control 1 is a positive control with good stability properties. IgG Control 2 is a negative control that is known to aggregate under the incubation conditions. The "BC1" B-Body has been incubated for 8 weeks without any loss of structural integrity as determined by the analytical SEC.

We have also determined that "BC1" has high thermostability, with a TM of the bivalent construct of ~ 72 °C.

Table 1 compares "BC1" to CrossMab in key developability characteristics:

| **Table 1** | | | |
|---|---|---|---|
| **Parameter** | **Unit** | **Roche CrossMab*** | **"BC1"** |
| Purification yield after protein A/SEC | mg/L | 58.5 | 300 |
| Homogeneity After purification | % SEC Area | 50-85 | 98 |
| Denaturation Temp (Tm) | degrees C | 69.2 | 72 |

| | | | |
|---|---|---|---|
| *Data from Schaefer et al. (Proc Natl Acad Sci USA. 2011 Jul 5;108(27):11187-92) | | | |

### 4.11.3. Example 3: Bivalent bispecific B-Body "BC6"

We constructed a bivalent bispecific B-Body, termed "BC6", that is identical to "BC1" but for retaining wild type residues in Domain B at residue 366 and Domain G at residue 351. "BC6" thus lacks the charge-pair cognates T366K and L351D that had been designed to facilitate correct pairing of domain B and domain G in "BC1". Salient features of the "BC6" architecture are illustrated in **FIG. 14****.**

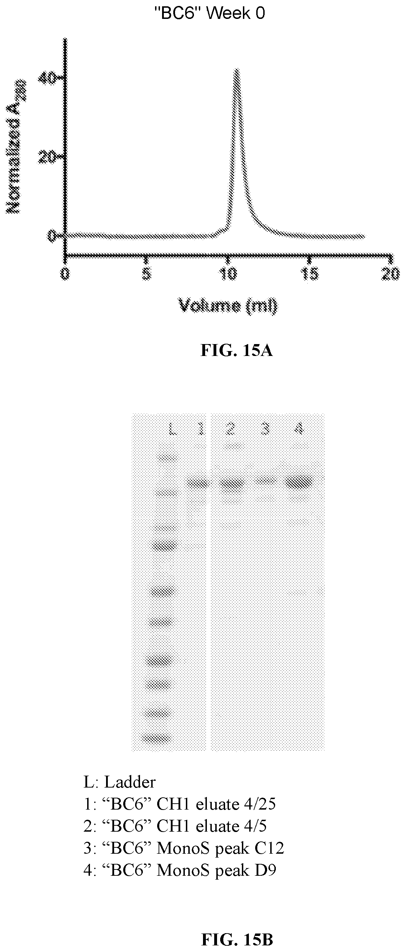

Notwithstanding the absence of the charge-pair residues present in "BC1", we found that a single step purification of "BC6" using CH1 affinity resin resulted in a highly homogeneous sample. FIG. 15A shows SEC analysis of "BC6" following one-step purification using the CaptureSelect^{™} CH1 affinity resin. The data demonstrate that the single step CH1 affinity purification yields a single monodisperse peak, similar to what we observed with "BC1", demonstrating that the disulfide bonds between polypeptide chains 1 and 2 and between polypeptide chains 3 and 4 are intact. The chromatogram also shows the absence of non-covalent aggregates.

FIG. 15B shows an SDS-PAGE gel under non-reducing conditions, with lane 1 loaded with a first lot of "BC6" after a single-step CH1 affinity purification, lane 2 loaded with a second lot of "BC6" after a single-step CH1 affinity purification. Lanes 3 and 4 demonstrate further purification can be achieved with ion exchange chromatography subsequent to CH1 affinity purification.

### 4.11.4. Example 4: Bivalent bispecific B-Bodies "BC28", "BC29" , "BC30", "BC31"

We constructed bivalent 1x1 bispecific B-Bodies "BC28", "BC29", "BC30" and "BC31" having an engineered disulfide within the CH3 interface in Domains B and G as an alternative S-S linkage to the C-terminal disulfide present in "BC1" and "BC6". Literature indicates that CH3 interface disulfide bonding is insufficient to enforce orthogonality in the context of Fc CH3 domains. The general architecture of these B-Body constructs is schematized in FIG. 16 with salient features of "BC28" summarized below:
Polypeptide chain 1: "BC28" chain 1 (SEQ ID NO:24)
   Domain A = VL (Antigen "A")
   Domain B = CH3 (Y349C; 445P, 446G, 447K insertion)
   Domain D = CH2
   Domain E= CH3 (S354C, K366W)
Polypeptide chain 2: "BC28" chain 2 (SEQ ID NO:25)
   Domain F = VH (Antigen "A")
   Domain G = CH3 (S354C; 445P, 446G, 447K insertion)
Polypeptide chain 3: "BC1" chain 3 (SEQ ID NO:10)
   Domain H = VL ("Nivo")
   Domain I = CL (Kappa)
   Domain J = CH2
   Domain K = CH3 (Y349C, D356E, L358M, T366S, L368A, Y407V)
Polypeptide chain 4: "BC1" chain 4 (SEQ ID NO:11)
   Domain L = VH ("Nivo")
   Domain M = CH1.

The "BC28" A:F antigen binding site is specific for "Antigen A". The "BC28" H:L antigen binding site is specific for PD1 (nivolumab sequences). "BC28" domain B has the following changes as compared to wild type CH3: Y349C; 445P, 446G, 447K insertion. "BC28" domain E has the following changes as compared to wild type CH3: S354C, K366W. "BC28" domain G has the following changes as compared to wild type: S354C; 445P, 446G, 447K insertion.

"BC28" thus has an engineered cysteine at residue 349C of Domain B and engineered cysteine at residue 354C of domain G ("349C-354C").

"BC29" has engineered cysteines at residue 351C of Domain B and 351C of Domain G ("351C-351C"). "BC30" has an engineered cysteine at residue 354C of Domain B and 349C of Domain G ("354C-349C"). BC31 has an engineered cysteine at residue 394C and engineered cysteine at 394C of Domain G ("394C-394C"). BC32 has engineered cysteines at residue 407C of Domain B and 407C of Domain G ("407C-407C").

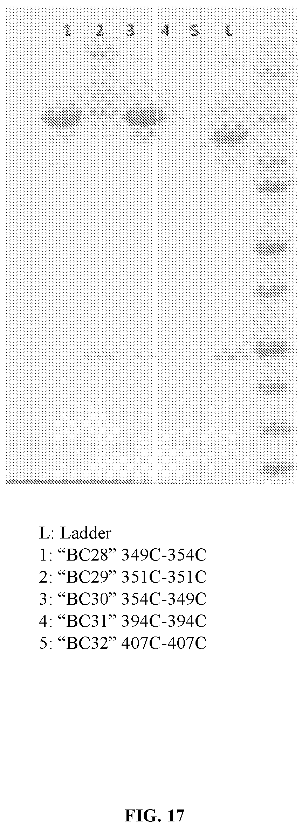

FIG. 17 shows SDS-PAGE analysis under non-reducing conditions following one-step purification using the CaptureSelect^{™} CH1 affinity resin. Lanes 1 and 3 show high levels of expression and substantial homogeneity of intact "BC28" (lane 1) and "BC30" (lane 3). Lane 2 shows oligomerization of BC29. Lanes 4 and 5 show poor expression of BC31 and BC32, respectively, and insufficient linkage in BC32. Another construct, BC9, which had cysteines introduced at residue 392 in domain B and 399 in Domain G ("392C-399C"), a disulfide pairing reported by Genentech, demonstrated oligomerization on SDS PAGE (data not shown).

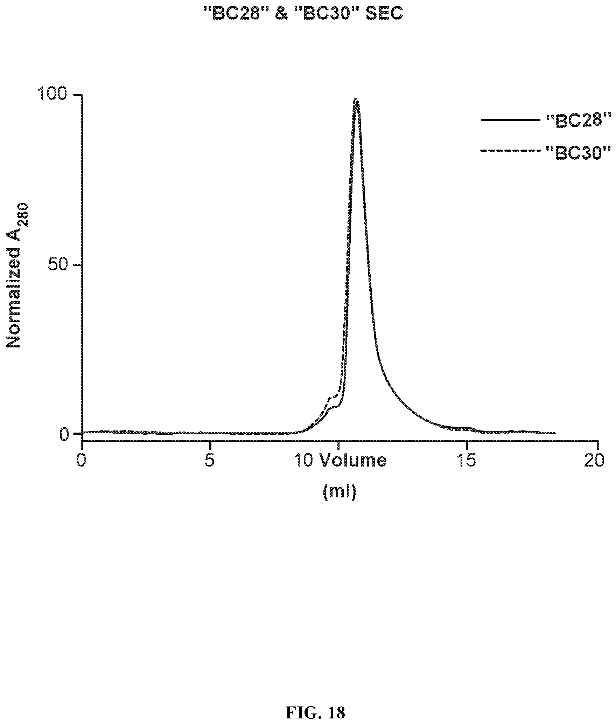

FIG. 18 shows SEC analysis of "BC28" and "BC30" following one-step purification using the CaptureSelect^{™} CH1 affinity resin. We have also demonstrated that "BC28" can readily be purified using a single step purification using Protein A resin (results not shown).

### 4.11.5. Example 5: Bivalent bispecific B-Body "BC44"

**FIG. 19** shows the general architecture of the bivalent bispecific 1x1 B-Body "BC44", our currently preferred bivalent bispecific 1x1 construct.
first polypeptide chain ("BC44" chain 1) (SEQ ID NO:32)
   Domain A = VL (Antigen "A")
   Domain B = CH3 (P343V; Y349C; 445P, 446G, 447K insertion)
   Domain E = CH2
   Domain E = CH3 (S354C, K366W)
second polypeptide chain (= "BC28" polypeptide chain 2) (SEQ NO:25)
   Domain F= VH (Antigen "A")
   Domain G = CH3 (S354C; 445P, 446G, 447K insertion)
third polypeptide chain (="BC1" polypeptide chain 3) (SEQ ID NO:10)
   Domain H = VL ("Nivo")
   Domain I = CL (Kappa)
   Domain J = CH2
   Domain K = CH3 (Y349C, D356E, L358M, T366S, L368A, Y407V)
fourth polypeptide chain (="BC1" polypeptide chain 4) (SEQ ID NO:11)
   Domain L = VH ("Nivo")
   Domain M = CH1.

### 4.11.6. Example 6: Variable-CH3 junction engineering

We produced a series of variants in which we mutated the VL-CH3 junction between Domains A and B and the VH-CH3 junction between domains F and G to assess the expression level, assembly and stability of bivalent 1x1 B-Body constructs. Although there are likely many solutions, to reduce introduction of T cell epitopes we chose to only use residues found naturally within the VL, VH and CH3 domains. Structural assessment of the domain architecture further limits desirable sequence combinations. Table 2 and Table 3 below show junctions for several junctional variants based on "BC1" and other bivalent constructs.

| Table 2 - Variants of Variable Domain/Constant Domain Junctions for 1^{st} Polypeptide Chain | | | | | | | | | | | |
|---|---|---|---|---|---|---|---|---|---|---|---|
| | VL | | | | | | CH3 | | | | Sequence |
| Variant | 106 | 107 | 108 | 109 | 110 | 111 | 343 | 344 | 345 | 346 | |
| BC1 | I | K | R | T | | | P | R | E | P | IKRTPREP (SEQ ID NO: 57) |
| BC13 | I | K | R | T | | | P | R | E | P | IKRTPREP (SEQ ID NO: 57) |
| BC14 | I | K | R | T | | | P | R | E | P | IKRTPREP (SEQ ID NO: 57) |
| BC15 | I | K | R | T | V | | | R | E | P | IKRTVREP (SEQ ID NO: 58) |
| BC16 | I | K | R | T | | | | R | E | P | IKRTREP (SEQ ID NO: 59) |
| BC17 | I | K | R | T | V | | P | R | E | P | IKRTVPREP (SEQ ID NO: 60) |
| BC24 | I | K | R | T | | | P | R | E | P | IKRTPREP (SEQ ID NO: 57) |
| BC25 | I | K | R | T | | | P | R | E | P | IKRTPREP (SEQ ID NO: 57) |
| BC26 | I | K | R | T | V | A | | | E | P | IKRTVAEP (SEQ ID NO: 61) |
| BC27 | I | K | R | T | V | A | P | R | E | P | IKRTVAPREP (SEQ ID NO: 62) |
| BC44 | I | K | R | T | | | | R | E | P | IKRTREP (SEQ ID NO: 59) |
| BC45 | I | K | R | T | | | P | R | E | P | IKRTPREP (SEQ ID NO: 57) |
| BC5 | I | K | R | T | | | P | R | E | P | IKRTPREP (SEQ ID NO: 57) |
| BC6 | I | K | R | T | | | P | R | E | P | IKRTPREP (SEQ ID NO: 57) |
| BC28 | I | K | R | T | | | P | R | E | P | IKRTPREP (SEQ ID NO: 57) |
| BC30 | I | K | R | T | | | P | R | E | P | IKRTPREP (SEQ ID NO: 57) |

| Table 3 - Variants of Variable Domain/Constant Domain Junctions for 2^{nd} Polypeptide Chain | | | | | | | | | | | | |
|---|---|---|---|---|---|---|---|---|---|---|---|---|
| | VH | | | | | | | CH3 | | | | Sequence |
| Variant | 112 | 113 | 114 | 115 | 116 | 117 | 118 | 343 | 344 | 345 | 346 | |
| BC1 | S | S | A | S | | | | P | R | E | P | SSASPREP (SEQ ID NO: 63) |
| BC13 | S | S | A | S | T | | | | R | E | P | SSASTREP (SEQ ID NO: 64) |
| BC14 | S | S | A | S | T | | | P | R | E | P | SSASTPREP (SEQ ID NO: 65) |
| BC15 | S | S | A | S | | | | P | R | E | P | SSASPREP (SEQ ID NO: 63) |
| BC16 | S | S | A | S | | | | P | R | E | P | SSASPREP (SEQ ID NO: 63) |
| BC17 | S | S | A | S | | | | P | R | E | P | SSASPREP (SEQ ID NO: 63) |
| BC24 | S | S | A | S | T | K | G | | | E | P | SSASTKGEP (SEQ ID NO: 66) |
| BC25 | S | S | A | S | T | K | G | | R | E | P | SSASTKGREP (SEQ ID NO: 67) |
| BC26 | S | S | A | S | | | | P | R | E | P | SSASPREP (SEQ ID NO: 63) |
| BC27 | S | S | A | S | | | | P | R | E | P | SSASPREP (SEQ ID NO: 63) |
| BC44 | S | S | A | S | | | | P | R | E | P | SSASPREP (SEQ ID NO: 63) |
| BC45 | S | S | A | S | | | | P | R | E | P | SSASPREP (SEQ ID NO: 63) |
| BC5 | S | S | A | S | | | | P | R | E | P | SSASPREP (SEQ ID NO: 63) |
| BC6 | S | S | A | S | | | | P | R | E | P | SSASPREP (SEQ ID NO: 63) |
| BC28 | S | S | A | S | | | | P | R | E | P | SSASPREP (SEQ ID NO: 63) |
| BC30 | S | S | A | S | | | | P | R | E | P | SSASPREP (SEQ ID NO: 63) |

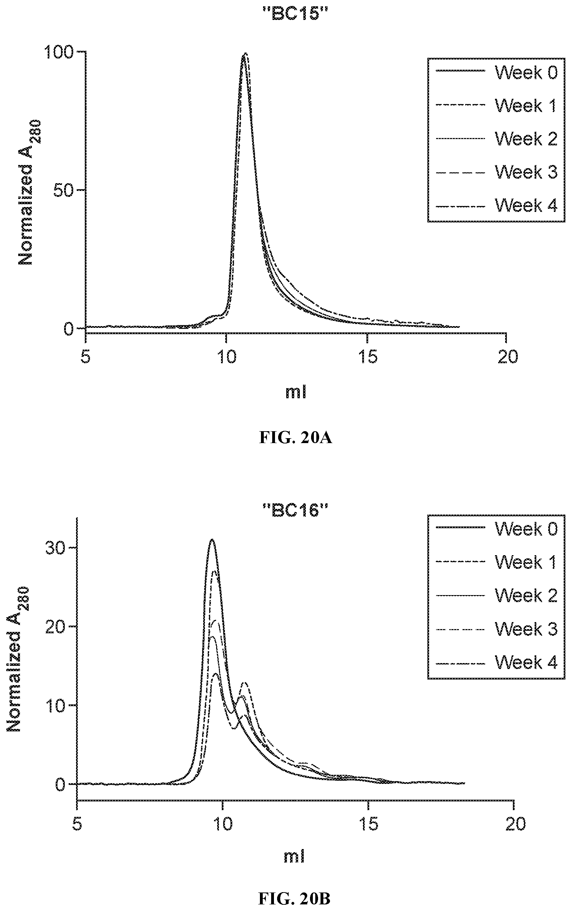

FIG. 20 shows size exclusion chromatography of "BC15" and "BC16" samples at the indicated week of an accelerated stability testing protocol at 40° C. "BC15" remained stable; "BC16" proved to be unstable over time.

### 4.11.7. Example 7: Trivalent 2x1 Bispecific B-Body construct ("BC1-2x1")

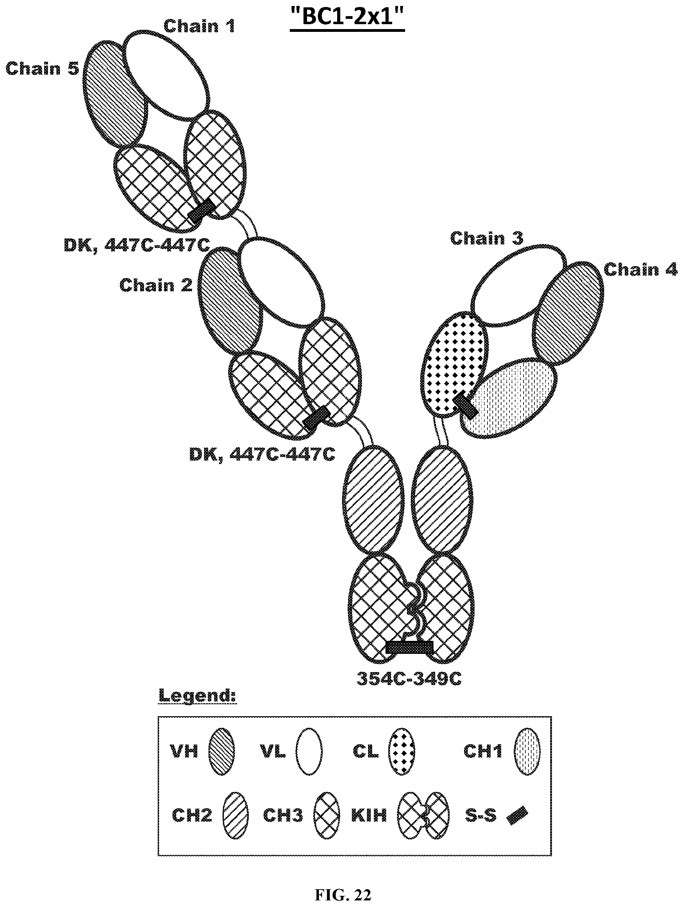

We constructed a trivalent 2x1 bispecific B-Body "BC1-2x1" based on "BC1". Salient features of the architecture are illustrated in **FIG. 22****.**

In greater detail, using the domain and polypeptide chain references summarized in FIG. 21,
1^{st} polypeptide chain
   Domain N = VL ("Antigen A")
   Domain O = CH3 (T366K, 447C)
   Domain A = VL ("Antigen A")
   Domain B = CH3 (T366K, 477C)
   Domain D = CH2
   Domain E = CH3 (Knob, 354C)
5^{th} polypeptide chain (= "BC1" chain 2)
   Domain P = VH ("Antigen A")
   Domain Q = CH3 (L351D, 447C)
2^{nd} polypeptide chain (= "BC1" chain 2)
   Domain F = VH ("Antigen A")
   Domain G = CH3 (L351D, 447C)
3^{rd} polypeptide chain (= "BC1" chain 3)
   Domain H = VL ("Nivo")
   Domain I = CL (Kappa)
   Domain J = CH2
   Domain K = CH3 (Hole, 349C)
4^{th} polypeptide chain (= "BC1" chain 4)
   Domain L = VH ("Nivo")
   Domain M = CH1.

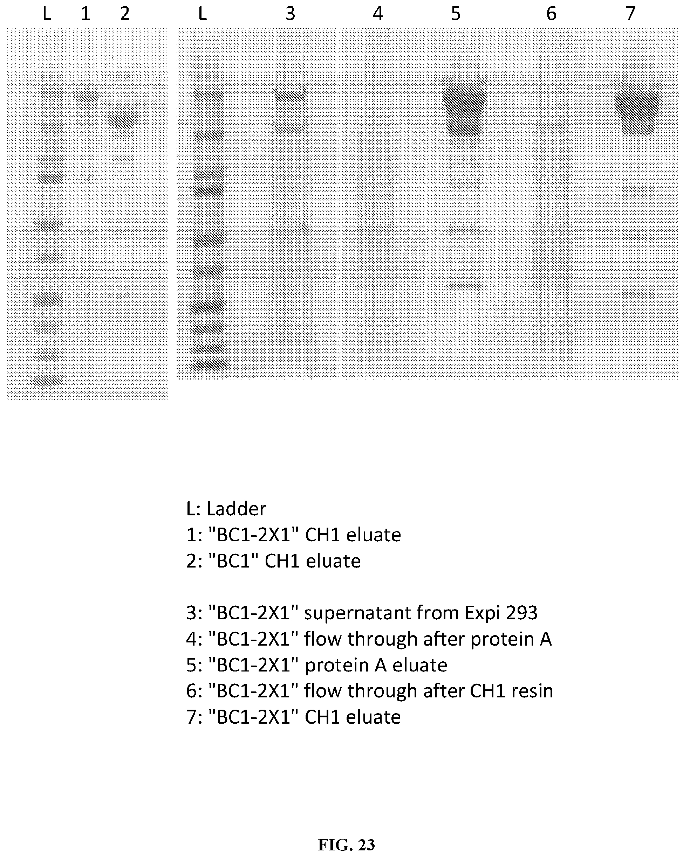

FIG. 23 shows non-reducing SDS-PAGE of protein expressed using the ThermoFisher Expi293 transient transfection system.

Lane 1 shows the eluate of the trivalent 2x1 "BC1-2X1" protein following one-step purification using the CaptureSelect^{™} CH1 affinity resin. Lane 2 shows the lower molecular weight, faster migrating, bivalent "BC1" protein following one-step purification using the CaptureSelect^{™} CH1 affinity resin. Lanes 3-5 demonstrate purification of "BC1-2x1" using protein A. Lanes 6 and 7 show purification of "BC1-2x1" using CH1 affinity resin.

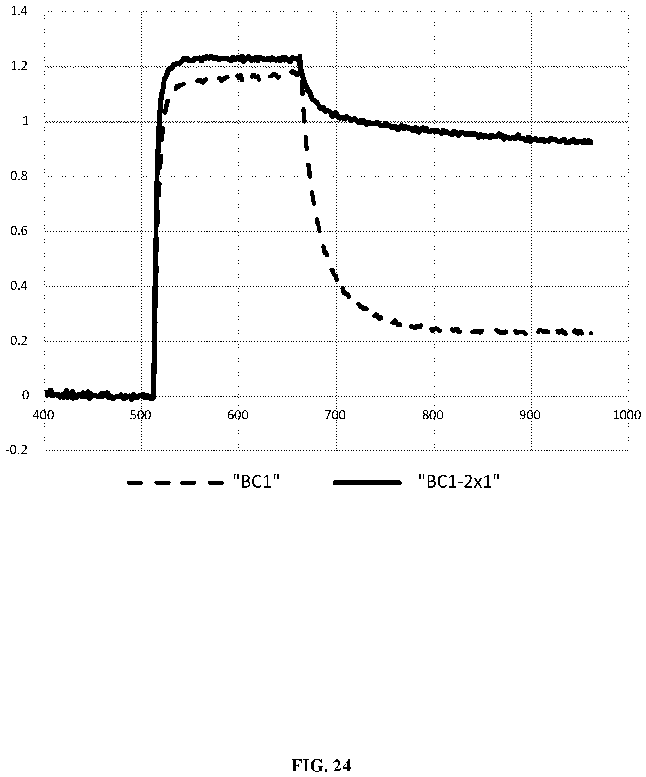

FIG. 24 compares the avidity of the bivalent "BC1" construct to the avidity of the trivalent 2x1 "BC1-2x1" construct using an Octet (Pall ForteBio) analysis. Biotinylated antigen "A" is immobilized on the surface, and the antibody constructs are passed over the surface for binding analysis.

### 4.11.8. Example 8: Trivalent 2x1 Trispecific B-Body construct ("TB111")

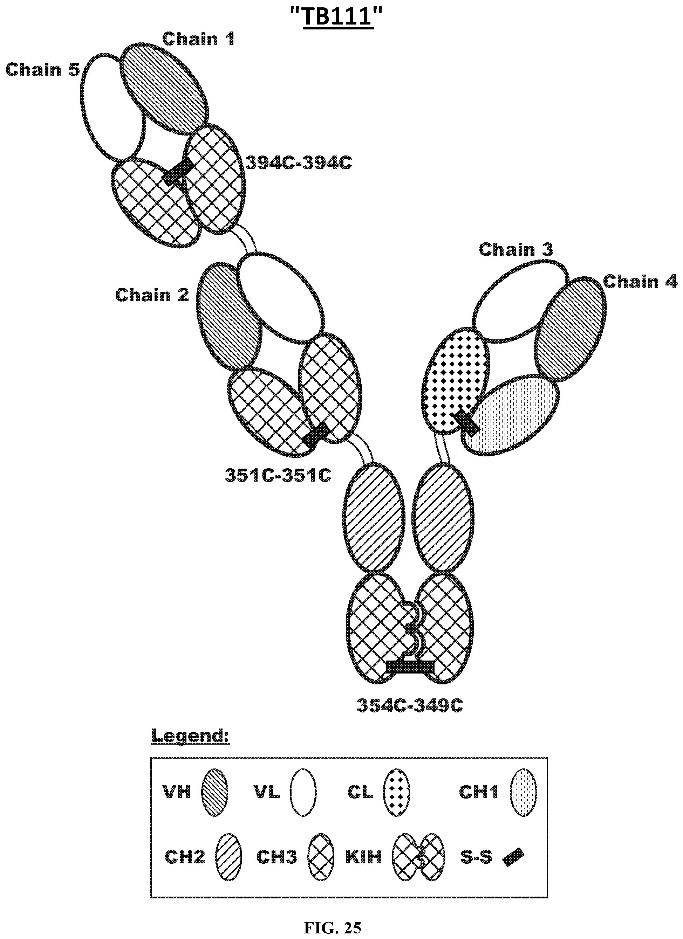

We designed a trivalent 2x1 trispecific molecule, "TB111", having the architecture schematized in **FIG. 25****.** With reference to the domain naming conventions set forth in **FIG. 21****,** TB111 has the following architecture ("Ada" indicates a V region from adalimumab):
polypeptide chain 1

| | |
|---|---|
| Domain N: | VH ("Ada") |
| Domain O: | CH3 (T366K, 394C) |
| Domain A: | VL ("Antigen A") |
| Domain B: | CH3 (T366K, 349C) |
| Domain D: | CH2 |
| Domain E: | CH3 (Knob, 354C) |

polypeptide chain 5

| | |
|---|---|
| Domain P: | VL ("Ada") |
| Domain Q: | CH3 (L351D, 394C) |

polypeptide chain 2

| | |
|---|---|
| Domain F: | VH ("Antigen A") |
| Domain G: | CH3 (L351D, 351C) |

polypeptide chain 3

| | |
|---|---|
| Domain H: | VL ("Nivo") |
| Domain I: | CL (kappa) |
| Domain J: | CH2 |
| Domain K: | CH3 (Hole, 349C) |

polypeptide chain 4 (="BC1" chain 4)

| | |
|---|---|
| Domain L: | VH ("Nivo") |
| Domain M: | CH1 |

This construct did not express.

### 4.11.9. Example 9: Trivalent 1x2 Bispecific Construct ("BC28-1x2")

We constructed a trivalent 1x2 bispecific B-Body having the following domain structure:
1^{st} polypeptide chain (="BC28" chain 1) (SEQ ID NO:24)
   Domain A = VL (Antigen "A")
   Domain B = CH3 (Y349C; 445P, 446G, 447K insertion)
   Domain D = CH2
   Domain E=CH3 (S354C, K366W)
2^{nd} polypeptide chain (="BC28" chain 2) (SEQ ID NO:25)
   Domain F = VH (Antigen "A")
   Domain G = CH3 (S354C; 445P, 446G, 447K insertion)
3^{rd} polypeptide chain (SEQ ID NO:37)
   Domain R = VL (Antigen "A")
   Domain S = CH3 (Y349C; 445P, 446G, 447K insertion)
   Linker = GSGSGS
   Domain H = VL ("Nivo")
   Domain I = CL
   Domain J = CH2
   Domain K = CH3 (Y349C, D356E, L358M, T366S, L368A, Y407V)
4^{th} polypeptide chain (= "BC1" chain 4) (SEQ ID NO:11):
   Domain L = VH ("Nivo")
   Domain M = CH1.
6^{th} polypeptide chain (="BC28" chain 2) (SEQ ID NO:25)
   Domain T = VH (Antigen "A")
   Domain U = CH3 (S354C; 445P, 446G, 447K insertion)

The A:F antigen binding site is specific for "Antigen A", as is the H:L binding antigen binding site. The R:T antigen binding site is specific for PD. The specificity of this construct is thus Antigen "A" x (PD1-Antigen "A").

### 4.11.10. Example 10: Trivalent 1x2 bispecific construct ("CTLA4-4 x Nivo x CTLA4-4")

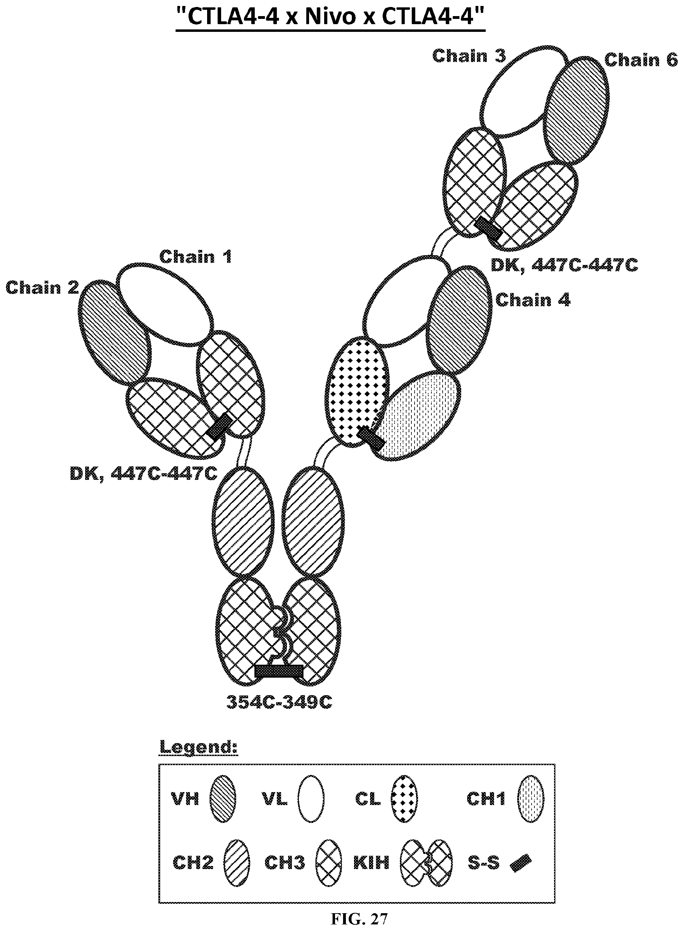

We constructed a trivalent 1x2 bispecific molecule having the general structure schematized in **FIG. 27** ("CTLA4-4 x Nivo x CTLA4-4"). Domain nomenclature is set forth in **FIG. 26****.**

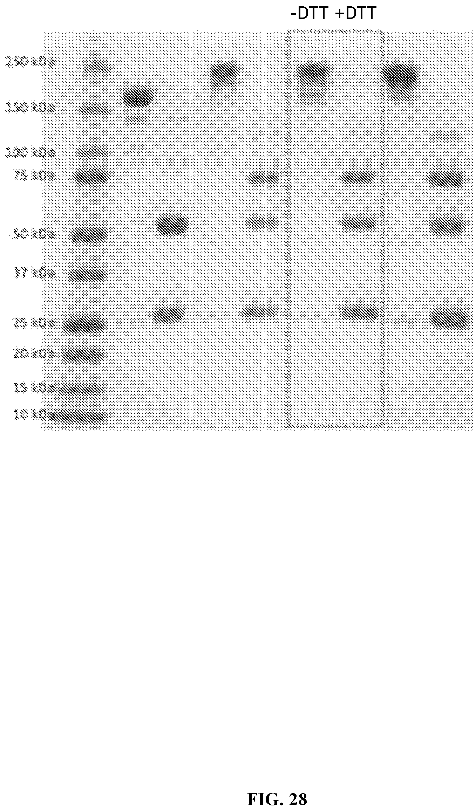

FIG. 28 is an SDS-PAGE gel in which the lanes showing the "CTLA4-4 x Nivo x CTLA4-4" construct under non-reducing and reducing conditions have been boxed.

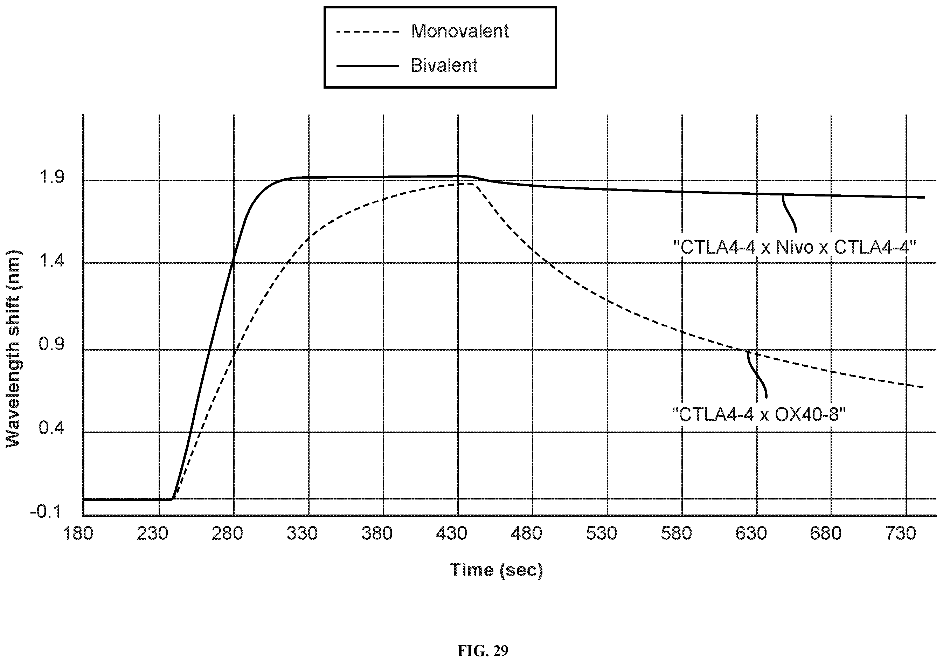

FIG. 29 compares antigen binding of two antibodies: "CTLA4-4 x OX40-8" and "CTLA4-4 x Nivo x CTLA4-4". "CTLA4-4 x OX40-8" binds to CTLA4 monovalently; while "CTLA4-4 x Nivo x CTLA4-4" bind to CTLA4 bivalently.

### 4.11.11. Example 11: Trivalent 1x2 trispecific construct "BC28-1x1x1a"

We constructed a trivalent 1x2 trispecific molecule having the general structure schematized in **FIG. 30****.** With reference to the domain nomenclature set forth in **FIG. 26****,**
1^{st} polypeptide chain (="BC28" chain1) [SEQ ID NO:24]
   Domain A = VL (Antigen "A")
   Domain B = CH3 (Y349C; 445P, 446G, 447K insertion)
   Domain D = CH2
   Domain E = CH3 (S354C, K366W)
2^{nd} polypeptide chain (="BC28" chain 2) (SEQ ID NO:25)
   Domain F = VH (Antigen "A")
   Domain G = CH3 (S354C; 445P, 446G, 447K insertion)
3^{rd} polypeptide chain (SEQ ID NO:45)
   Domain R = VL (CTLA4-4)
   Domain S = CH3 (T366K; 445K, 446S, 447C insertion)
   Linker = GSGSGS
   Domain H = VL ("Nivo")
   Domain I = CL
   Domain J = CH2
   Domain K = CH3 (Y349C, D356E, L358M, T366S, L368A, Y407V)
4^{th} polypeptide chain (="BC1" chain 4) (SEQ ID NO:11)
   Domain L = VH ("Nivo")
   Domain M = CH1.
6^{th} polypeptide chain (=hCTLA4-4 chain2) (SEQ ID NO:53)
   Domain T = VH (CTLA4)
   Domain U = CH3 (L351D, 445G, 446E, 447C insertion)

The antigen binding sites of this trispecific construct were:
Antigen binding site A:F was specific for "Antigen A"
Antigen binding site H:L was specific for PD1 (nivolumab sequence)
Antigen binding site R:T was specific for CTLA4.

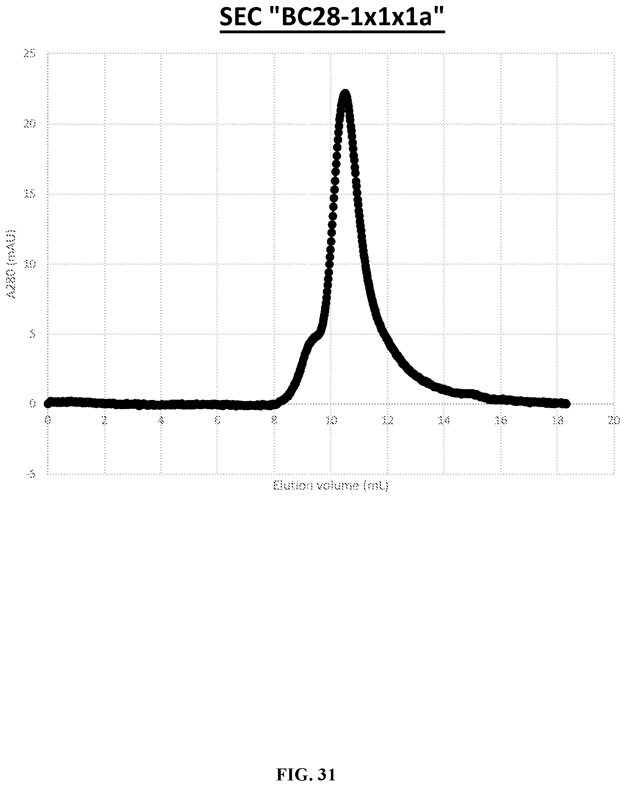

FIG. 31 shows size exclusion chromatography with "BC28-1x1x1a" following transient expression and one-step purification using the CaptureSelect^{™} CH1 affinity resin, demonstrating a single well-defined peak.

### 4.11.12. Example 12: SDS-PAGE analysis of bivalent and trivalent constructs

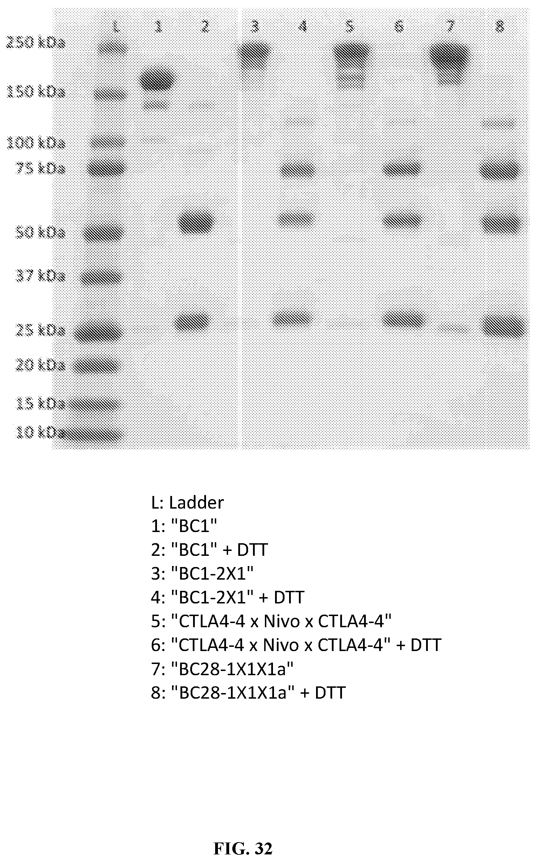

FIG. 32 shows an SDS-PAGE gel with various constructs, each after transient expression and one-step purification using the CaptureSelect^{™} CH1 affinity resin, under non-reducing and reducing conditions.

Lanes 1 (nonreducing conditions) and 2 (reducing conditions, + DTT) are the bivalent 1x1 bispecific construct "BC1". Lanes 3 (nonreducing) and 4 (reducing) are the trivalent bispecific 2x1 construct "BC1-2x1" (see Example 7). Lanes 5 (nonreducing) and 6 (reducing) are the trivalent 1x2 bispecific construct "CTLA4-4 x Nivo x CTLA4-4" (see Example 10). Lanes 7 (nonreducing) and 8 (reducing) are the trivalent 1x2 trispecific "BC28-1x1x1a" construct described in Example 11.

The SDS-PAGE gel demonstrates the complete assembly of each construct, with the predominant band in the non-reducing gel appearing at the expected molecular weight for each construct.

### 4.11.13. Example 13: Binding analysis

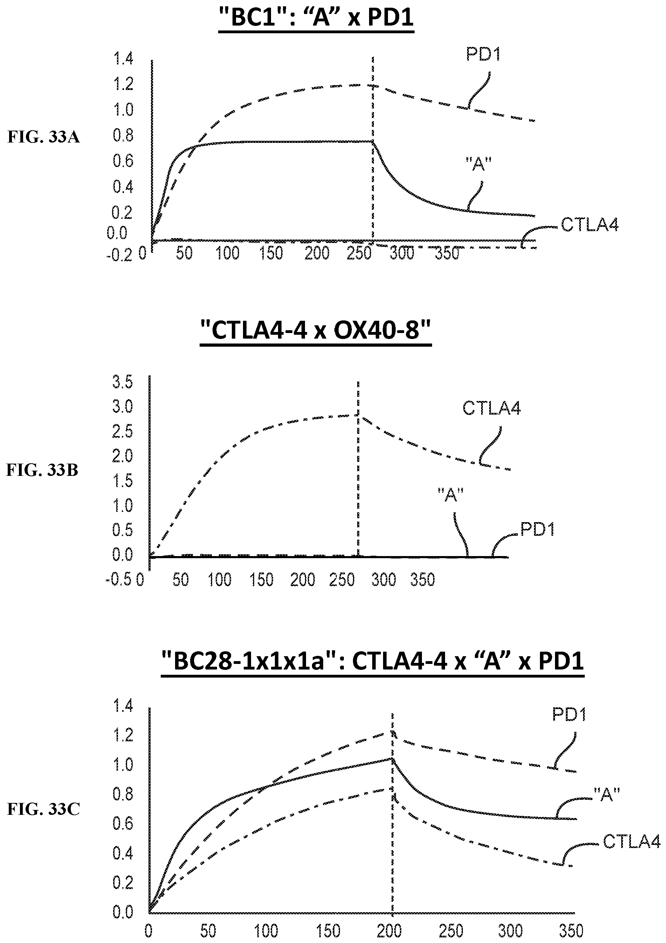

FIG. 33 shows Octet binding analyses to 3 antigens: PD1, Antigen "A", and CTLA-4. In each instance, the antigen is immobilized and the B-Body is the analyte. For reference, 1x1 bispecifics "BC1" and "CTLA4-4 x OX40-8" were also compared to demonstrate 1x1 B-Bodies bind specifically only to antigens for which the antigen binding sites were selected.

FIG. 33A shows binding of "BC1" to PD1 and to Antigen "A", but not CTLA4. FIG. 33B shows binding of a bivalent bispecific 1x1 construct "CTLA4-4 x OX40-8" to CTLA4, but not to Antigen "A" or PD1. FIG. 33C shows the binding of the trivalent trispecific 1x2 construct, "BC28-1x1x1a" to PD1, Antigen "A", and CTLA4.

### 4.11.14. Example 14: Tetravalent constructs

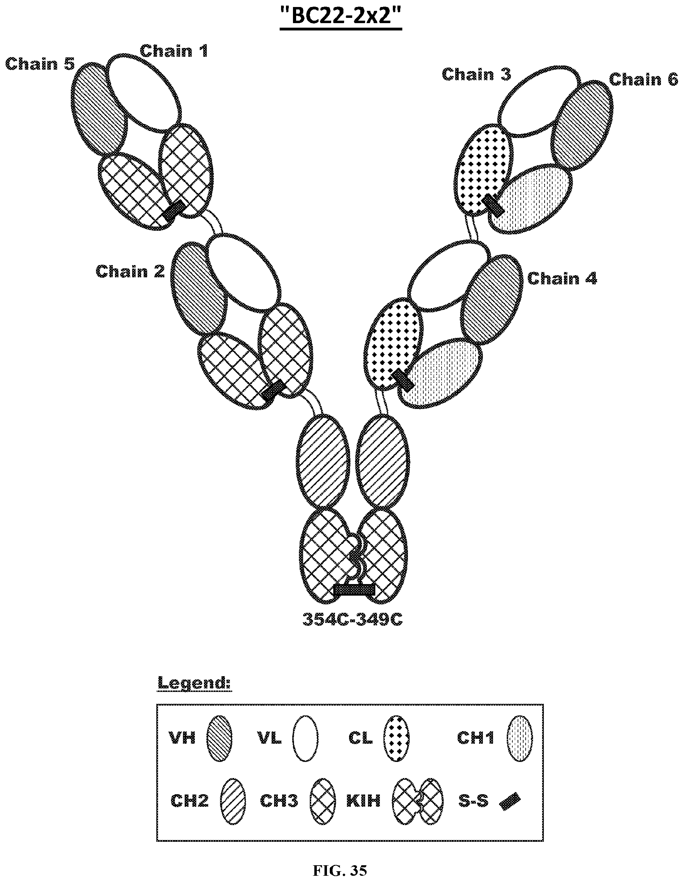

FIG. 35 shows the overall architecture of a 2x2 tetravalent bispecific construct "BC22 -2x2". The 2x2 tetravalent bispecific was constructed with "BC1" scaffold by duplicating each variable domain-constant domain segment. Domain nomenclature is schematized in FIG. 34.

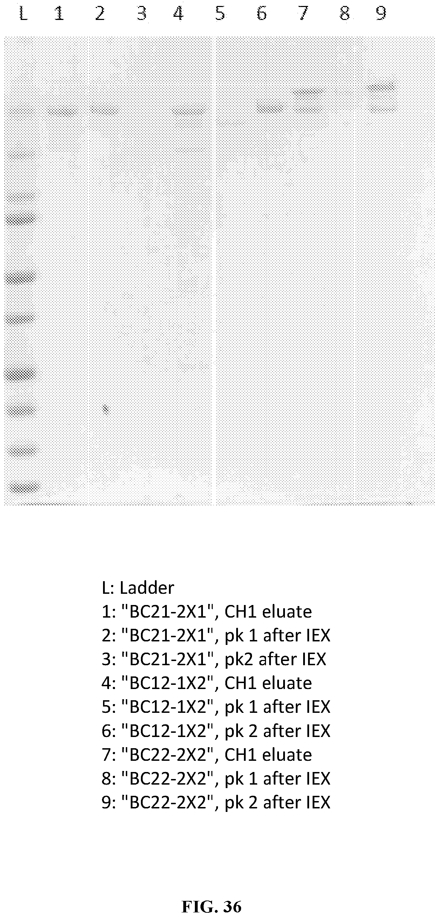

FIG. 36 is an SDS-PAGE gel. Lanes 7-9 show the "BC22-2x2" tetravalent construct respectively following one-step purification using the CaptureSelect^{™} CH1 affinity resin ("CH1 eluate"), and after an additional ion exchange chromatography purification (lane 8, "pk 1 after IEX"; lane 9, "pk 2 after IEX"). Lanes 1-3 are the trivalent 2x1 construct "BC21-2x1" after CH1 affinity purification (lane 1) and, in lanes 2 and 3, subsequent ion exchange chromatography. Lanes 4-6 are the 1x2 trivalent construct "BC12-1x2".

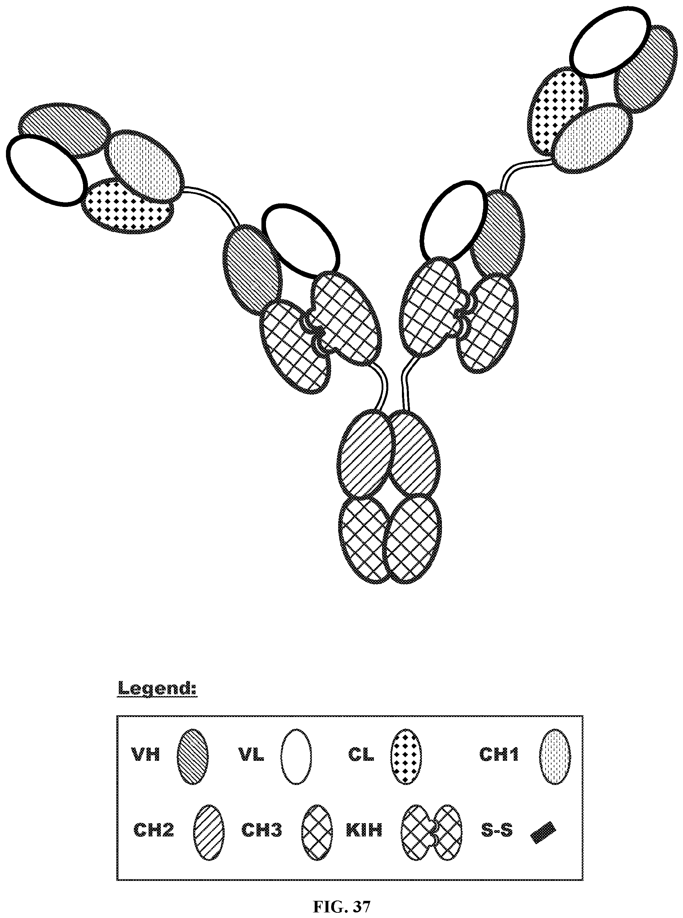

FIG. 37 shows the overall architecture of a 2x2 tetravalent construct.

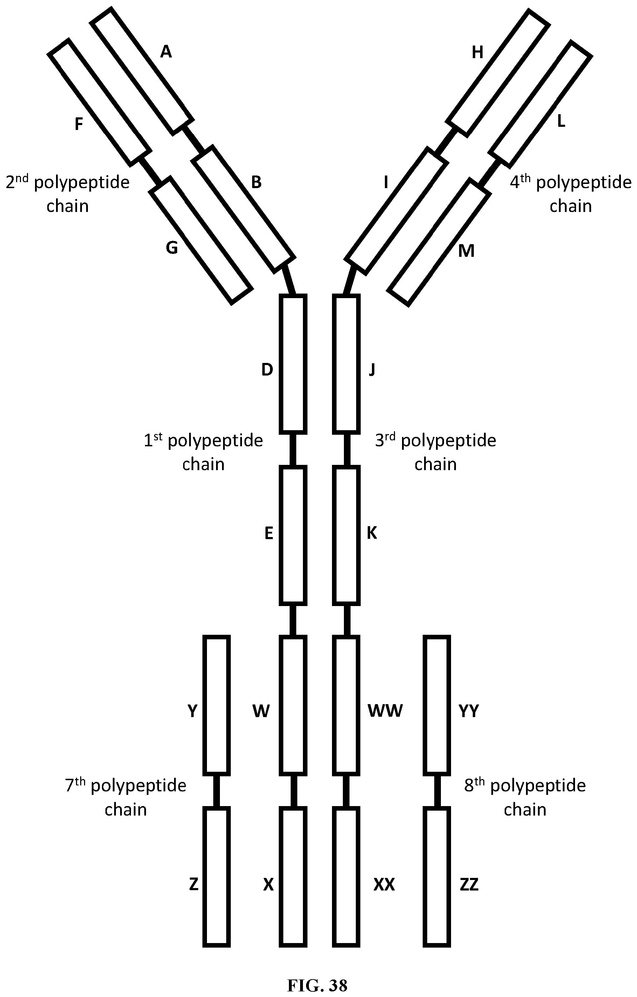

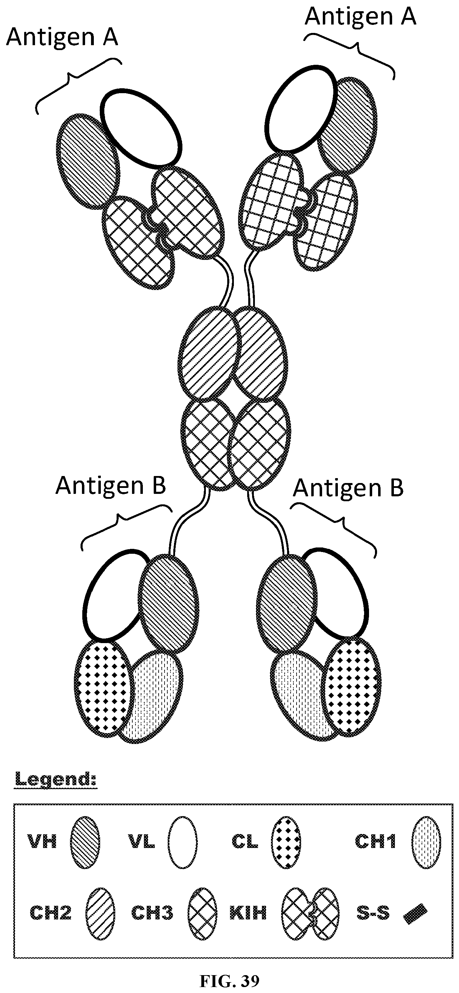

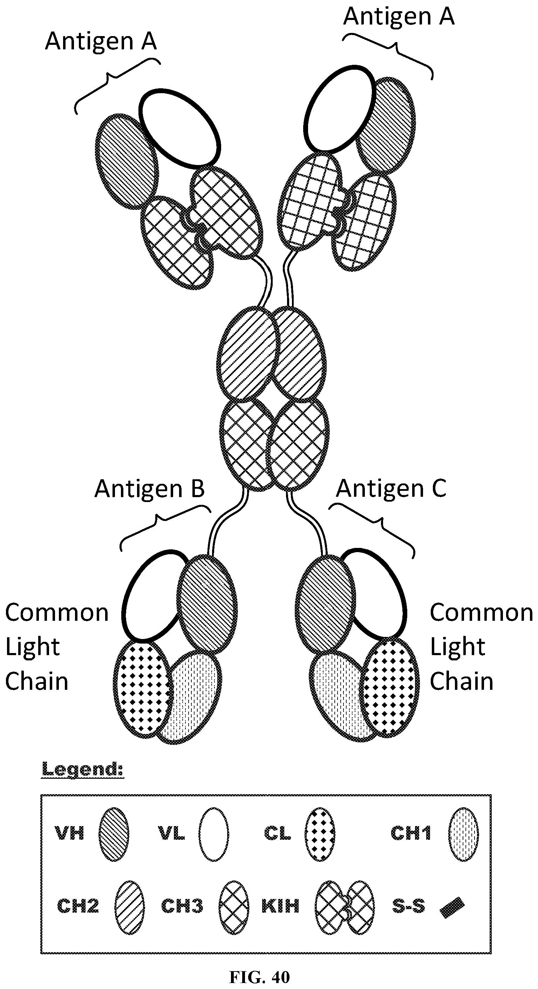

FIGS. 39 and 40 schematize tetravalent constructs having alternative architectures. Domain nomenclature is presented in FIG. 38.

### 4.11.15. Example 15: Bispecific Antigen Engagement by B-Body.

A tetravalent bispecific 2x2 B-Body "B-Body-IgG 2x2" was constructed. In greater detail, using the domain and polypeptide chain references summarized in FIG. 38,
1^{st} polypeptide chain
   Domain A = VL (Certolizumab)
   Domain B = CH3 (IgG1, knob)
   Domain D = CH2 (IgG1)
   Domain E = CH3 (IgG1)
   Domain W = VH (Antigen "A")
   Domain X = CH1 (IgG1)
3^{rd} polypeptide chain (identical to first polypeptide chain)
   Domain H = VL (Certolizumab)
   Domain I = CH3 (IgG1, knob)
   Domain J = CH2 (IgG1)
   Domain K = CH3 (IgG1)
   Domain WW = VH (Antigen "A")
   Domain XX = CH1 (IgG1)
2^{nd} polypeptide chain
   Domain F = VH (Certolizumab)
   Domain G = CH3 (IgG1, hole)
4^{th} polypeptide chain (identical to third polypeptide chain)
   Domain F = VH (Certolizumab)
   Domain G = CH3 (IgG1, hole)
7^{th} polypeptide chain
   Domain Y = VH ("Antigen A")
   Domain Z = CL Kappa
8^{th} polypeptide chain (identical to seventh polypeptide chain)
   Domain YY = VH ("Antigen A")
   Domain ZZ = CL Kappa.

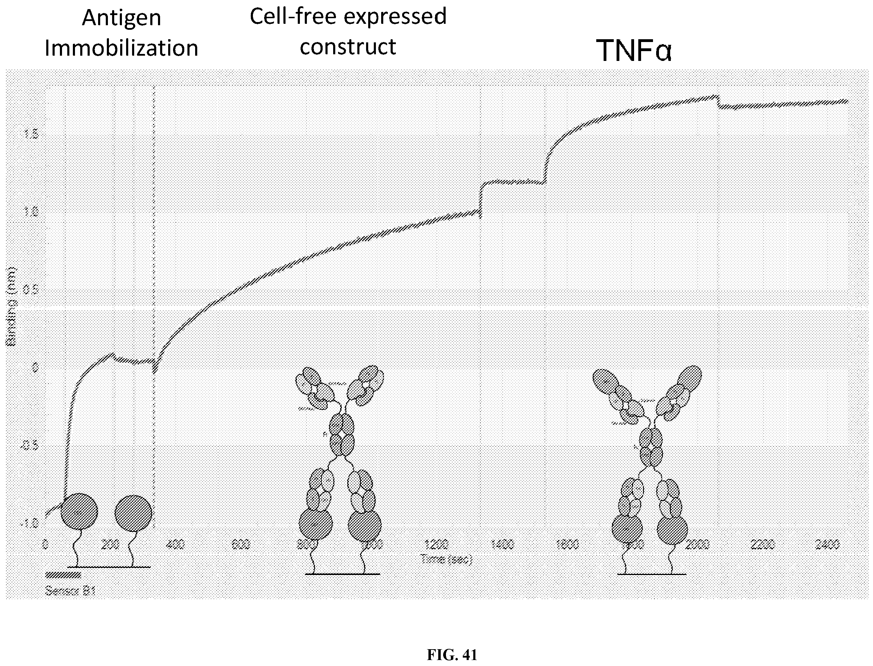

This was cloned and expressed as described in Example 1. Here, the BLI experiment consisted of immobilization of biotinylated antigen "A" on a streptavidin sensor, followed by establishing baseline with 10x kinetic buffer. The sensor was then dipped in cell-free expressed "B-Body-IgG 2x2" followed by establishment of a new baseline. Finally, the sensor was dipped in 100 nM TNFα where a second binding event was observed, confirming the bispecific binding of both antigens by a single "B-Body-IgG 2x2" construct. Results are shown in FIG. 41.

### 4.11.16. Example 16: Antigen-Specific Cell Binding of "BB-IgG 2x2".

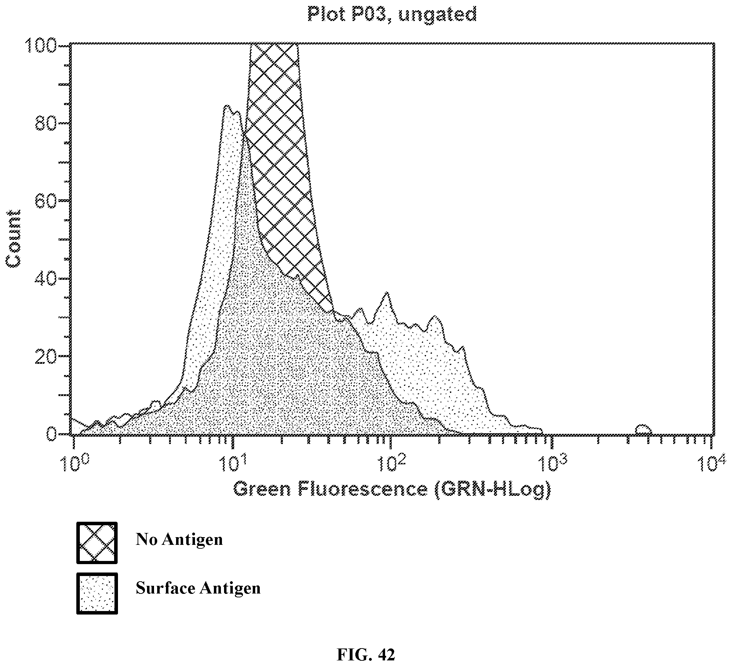

Expi-293 cells were either mock transfected or transiently transfected with Antigen "B" using the Expi-293 Transfection Kit (Life Technologies). Forty-eight hours after transfection, the Expi-293 cells were harvested and fixed in 4% paraformaldehyde for 15 minutes at room temperature. The cells were washed twice in PBS. 200,000 Antigen B or Mock transfected Expi-293 cells were placed in a V-bottom 96 well plate in 100 µL of PBS. The cells were incubated with the "B-Body-IgG 2x2" at a concentration of 3 ug/mL for 1.5 hours at room temperature. The cells were centrifuged at 300xG for 7 minutes, washed in PBS, and incubated with 100 µL of FITC labeled goat-anti human secondary antibody at a concentration of 8 µg/mL for 1 hour at room temperature. The cells were centrifuged at 300xG for 7 minutes, washed in PBS, and cell binding was confirmed by flow cytometry using a Guava easyCyte. Results are shown in FIG. 42.

### 4.11.17. Example 17: SDS-PAGE analysis of bivalent and trivalent constructs

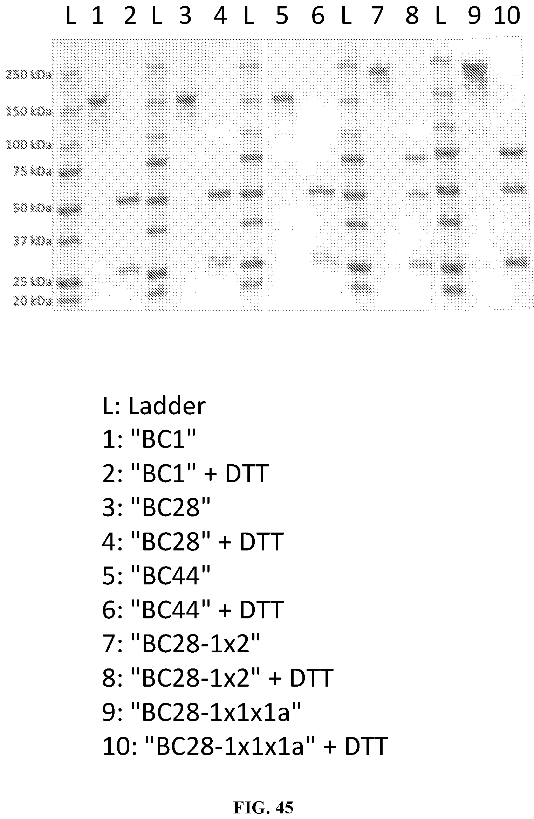

FIG. 45 shows an SDS-PAGE gel with various constructs, each after transient expression and one-step purification using the CaptureSelect^{™} CH1 affinity resin, under non-reducing and reducing conditions.

Lanes 1 (nonreducing conditions) and 2 (reducing conditions, + DTT) are the bivalent 1x1 bispecific construct "BC1". Lanes 3 (nonreducing) and 4 (reducing) are the bivalent 1x1 bispecific construct "BC28" (see Example 4). Lanes 5 (nonreducing) and 6 (reducing) are the bivalent 1x1 bispecific construct "BC44" (see Example 5). Lanes 7 (nonreducing) and 8 (reducing) are the trivalent 1x2 bispecific "BC28-1x2" construct (see Example 9). Lanes 9 (nonreducing) and 10 (reducing) are the trivalent 1x2 trispecific "BC28-1x1x1a" construct described in Example 11.

The SDS-PAGE gel demonstrates the complete assembly of each construct, with the predominant band in the non-reducing gel appearing at the expected molecular weight for each construct.

### 4.11.18. Example 18: Stability analysis of Variable-CH3 junction engineering

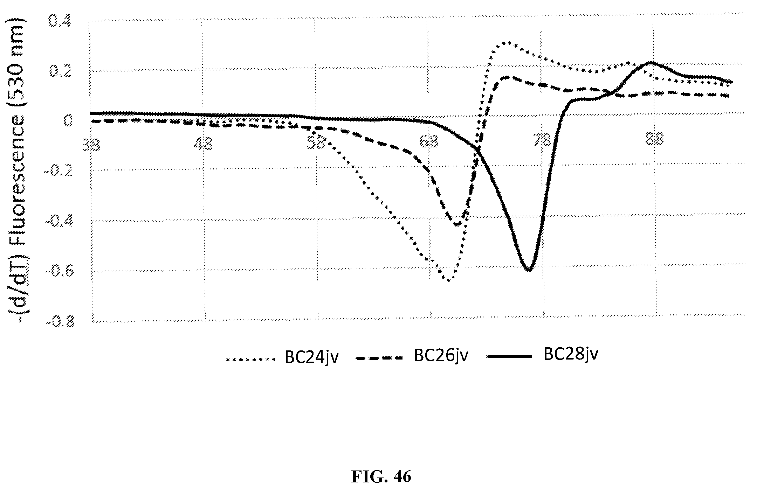

Pairing stability between various junctional variant combinations was assessed. Differential scanning fluorimetry was performed to determine the melting temperature of various junctional variant pairings between VL-CH3 polypeptides from Chain 1 (domains A and B) and VH-CH3 polypeptides from 2 (domains F and G). Junctional variants "BC6jv", "BC28jv", "BC30jv", "BC44jv", and "BC45jv", each having the corresponding junctional sequences of "BC6", "BC28", "BC30", "BC44", and "BC45" found in Table 2 and Table 3 above, demonstrate increased pairing stability with Tm's in the 76-77 degree range (see Table 4). Fig. 46 shows differences in the thermal transitions for "BC24jv", "BC26jv", and "BC28jv", with "BC28jv" demonstrating the greatest stability of the three. The x-axis of the figure is temperature and the y-axis is the change in fluorescence divided by the change in temperature (-dFluor/dTemp). Experiments were performed as described in Niesen et al. (Nature Protocols, (2007) 2, 2212 - 2221).

| Table 4 - Melting Temperatures of Junctional Variant Pairs | | |
|---|---|---|
| JUNCTIONAL VARIANT PAIR | MELTING TEMP #1 (°C) | MELTING TEMP #2 (°C) |
| BC1jv | 69.7 | 55.6 |
| BC5jv | 71.6 | |
| BC6jv | 77 | |
| BC15jv | 68.2 | 54 |
| BC16jv | 65.9 | |
| BC17jv | 68 | |
| BC24jv | 69.7 | |
| BC26jv | 70.3 | |
| BC28jv | 76.7 | |
| BC30jv | 76.8 | |
| BC44jv | 76.2 | |
| BC45jv | 76 | |

### 4.12. Sequences

> Example 1, bivalent monospecific construct CHAIN 1 **[SEQ ID NO:1]** 
(VL)~VEIKRTPREPQVYTLPPCRDELTKNQVSLWCLVKGFYPSDIAVEWESNGQPENNYKT
TPPVLDSDGSFFLYSKLTVDKSRWQQGNVFSCSVMHEALHNHYTQKSLSLSPGKDKTHTCPP
CPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLP
PCRDELTKNQVSLWCLVKGFYPSDIAVEWESNGQPENNYKTTPPVLDSDGSFFLYSKLTVDK
SRWQQGNVFSCSVMHEALHNHYTQKSLSLSPGK
>Example 1, bivalent monospecific construct CHAIN 2 **[SEQ ID NO:2]** 
(VH)~VTVSSASPREPQVYTLPPCRDELTKNQVSLWCLVKGFYPSDIAVEWESNGQPENNYK
TTPPVLDSDGSFFLYSKLTVDKSRWQQGNVFSCSVMHEALHNHYTQKSLSLSPGK
> Example 1, bivalent, bispecific construct CHAIN 1 **[SEQ ID NO:3]** 
(VL)~VEIKRTPREPQVYTLPPCRDELTKNQVSLWCLVKGFYPSDIAVEWESNGQPENNYKT
TPPVLDSDGSFFLYSKLTVDKSRWQQGNVFSCSVMHEALHNHYTQKSLSLSPGKDKTHTCPP
CPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLP
PCRDELTKNQVSLWCLVKGFYPSDIAVEWESNGQPENNYKTTPPVLDSDGSFFLYSKLTVDK
SRWQQGNVFSCSVMHEALHNHYTQKSLSLSPGK
VL-   CH3-          Hinge- CH2-  CH3(knob)
> Example 1, bivalent, bispecific construct CHAIN 2 **[SEQ ID NO:4]** 
(VH)~VTVSSASPREPQVYTLPPCRDELTKNQVSLWCLVKGFYPSDIAVEWESNGQPENNYK
TTPPVLDSDGSFFLYSKLTVDKSRWQQGNVFSCSVMHEALHNHYTQKSLSLSPGK
VH- CH3
> Example 1, bivalent, bispecific construct CHAIN 3_ **[SEQ ID NO:5]** 
(VL)~VEIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGECDKTHTCPP
CPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVCTLP
PSREEMTKNQVSLSCAVKGFYPSDIAVEWESNGQPENNYKTTPPVLDSDGSFFLVSKLTVDK
SRWQQGNVFSCSVMHEALHNHYTQKSLSLSPGK
VL-   CL-          Hinge- CH2-  CH3(hole)
> Example 1, bivalent, bispecific construct CHAIN 4 **[SEQ ID NO:6]** 
(VH)~VTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPPKSC
VH- CH1
> Fc Fragment of Human IgG1 **[SEQ ID NO:7]** 
GQPREPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSP
>BC1 chain 1 **[SEQ ID NO:8]** 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTPREPQ
VYTLPPSRDELTKNQVSLKCLVKGFYPSDIAVEWESNGQPENNYKTTPPVLDSDGSF
FLYSKLTVDKSRWQQGNVFSCSVMHEALHNHYTQKSLSLSKSCDKTHTCPPCPAPEL
LGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQV
YTLPPCRDELTKNQVSLWCLVKGFYPSDIAVEWESNGQPENNYKTTPPVLDSDGSFF
LYSKLTVDKSRWQQGNVFSCSVMHEALHNHYTQKSLSLSPGK
 Domain arrangement:

| | | | | |
|---|---|---|---|---|
| A- | B- | Hinge- | D- | E |
| VL- | CH3- | *Hinge-* | **CH2-** | *CH3 (knob)* |

Mutations in first CH3 (Domain B):
   T366K; 445K, 446S, 447C insertion
Mutations in second CH3 (Domain E):
   S354C, K366W

>BC1 chain 2 **[SEQ ID NO:9]** 
EVQLVESGGGLVQPGGSLRLSCAASGFTFSDYDIHWVRQAPGKGLEWVGDITPYDGT
TNYADSVKGRFTISADTSKNTAYLQMNSLRAEDTAVYYCARLVGEIATGFDYWGQGT
LVTVSSASPREPQVYTDPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSGEC
 Domain arrangement:
F- G
VH- CH3
Mutations in CH3 (Domain G):
   L351D; 445G, 446E, 447C insertion

>BC1 chain 3 **[SEQ ID NO:10]** 
EIVLTQSPATLSLSPGERATLSCRASQSVSSYLAWYQQKPGQAPRLLIYDASNRATG
IPARFSGSGSGTDFTLTISSLEPEDFAVYYCQQSSNWPRTFGQGTKVEIKRTVAAPS
VFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGECDKTHTCPPCPAPEL
LGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQV
CTLPPSREEMTKNQVSLSCAVKGFYPSDIAVEWESNGQPENNYKTTPPVLDSDGSFF
LVSKLTVDKSRWQQGNVFSCSVMHEALHNHYTQKSLSLSPGK
 Domain arrangement:

| | | | | |
|---|---|---|---|---|
| H- | I- | Hinge- | J- | K |
| VL- | CL- | *Hinge-* | **CH2-** | *CH3 (hole)* |

Mutations in CH3 (domain K):
Y349C, D356E, L358M, T366S, L368A, Y407V
>BC1 chain 4 **[SEQ ID NO:11]** 
QVQLVESGGGVVQPGRSLRLDCKASGITFSNSGMHWVRQAPGKGLEWVAVIWYDGSK
RYYADSVKGRFTISRDNSKNTLFLQMNSLRAEDTAVYYCATNDDYWGQGTLVTVSSA
STKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPPKSC
 Domain arrangement:

| | |
|---|---|
| L- | M |
| VH- | CH1 |

> BC1 Domain A **[SEQ ID NO:12]** 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRT
> BC1 Domain B **[SEQ ID NO:13]** 
PREPQVYTLPPSRDELTKNQVSLKCLVKGFYPSDIAVEWESNGQPENNYKTTPPVLD
SDGSFFLYSKLTVDKSRWQQGNVFSCSVMHEALHNHYTQKSLSLSKSC
> BC1 Domain D **[SEQ ID NO:14]** 
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAK
> BC1 Domain E **[SEQ ID NO:15]** 
GQPREPQVYTLPPCRDELTKNQVSLWCLVKGFYPSDIAVEWESNGQPENNYKTTPPV
LDSDGSFFLYSKLTVDKSRWQQGNVFSCSVMHEALHNHYTQKSLSLSPGK
> BC1 Domain F **[SEQ ID NO:16]** 
EVQLVESGGGLVQPGGSLRLSCAASGFTFSDYDIHWVRQAPGKGLEWVGDITPYDGT
TNYADSVKGRFTISADTSKNTAYLQMNSLRAEDTAVYYCARLVGEIATGFDYWGQGT
LVTVSSAS
> BC1 Domain G **[SEQ ID NO:17]** 
PREPQVYTDPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSGEC
> BC1 Domain H **[SEQ ID NO:18]**
> BC1 Domain I **[SEQ ID NO:19]**
> BC1 Domain J **[SEQ ID NO:20]** 
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAK
> BC1 Domain K **[SEQ ID NO:21]** 
GQPREPQVCTLPPSREEMTKNQVSLSCAVKGFYPSDIAVEWESNGQPENNYKTTPPV
LDSDGSFFLVSKLTVDKSRWQQGNVFSCSVMHEALHNHYTQKSLSLSPGK
> BC1 Domain L **[SEQ ID NO:22]**
> BC1 Domain M **[SEQ ID NO:23]** 
ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPPKSC
>BC28 chain 1 **[SEQ ID NO:24]** 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTPREPQ
VCTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGKDKTHTCPPCPAPEL
LGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQV
YTLPPCRDELTKNQVSLWCLVKGFYPSDIAVEWESNGQPENNYKTTPPVLDSDGSFF
LYSKLTVDKSRWQQGNVFSCSVMHEALHNHYTQKSLSLSPGK
Domain arrangement:

| | | | | |
|---|---|---|---|---|
| A- | B- | Hinge- | D- | E |
| VL- | CH3- | *Hinge-* | **CH2-** | *CH3 (knob)* |

Mutations in domain B:
Y349C; 445P, 446G, 447K insertion
Mutations in domain E:
S354C, K366W
>BC28 chain 2 **[SEQ ID NO:25]** 
EVQLVESGGGLVQPGGSLRLSCAASGFTFSDYDIHWVRQAPGKGLEWVGDITPYDGT
TNYADSVKGRFTISADTSKNTAYLQMNSLRAEDTAVYYCARLVGEIATGFDYWGQGT
LVTVSSASPREPQVYTLPPCRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK
Domain arrangement:

| | |
|---|---|
| F- | G |
| VH- | CH3 |

Mutations in domain G:
S354C; 445P, 446G, 447K insertion
>BC28 domain A **[SEQ ID NO:26]** 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRT
>BC28 domain B **[SEQ ID NO:27]** 
PREPQVCTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
>BC28 domain D **[SEQ ID NO:28]** 
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAK
>BC28 domain E **[SEQ ID NO:29]** 
GQPREPQVYTLPPCRDELTKNQVSLWCLVKGFYPSDIAVEWESNGQPENNYKTTPPV
LDSDGSFFLYSKLTVDKSRWQQGNVFSCSVMHEALHNHYTQKSLSLSPGK
>BC28 domain F **[SEQ ID NO:30]** 
EVQLVESGGGLVQPGGSLRLSCAASGFTFSDYDIHWVRQAPGKGLEWVGDITPYDGT
TNYADSVKGRFTISADTSKNTAYLQMNSLRAEDTAVYYCARLVGEIATGFDYWGQGT
LVTVSSAS
>BC28 domain G **[SEQ ID NO:31]** 
PREPQVYTLPPCRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
>BC44 chain 1 **[SEQ ID NO:32]** 
DIQMTQSPSSLSASVGDRVTITCRASQDVNTAVAWYQQKPGKAPKLLIYSASFLYSGVPSRF
SGSRSGTDFTLTISSLQPEDFATYYCQQHYTTPPTFGQGTKVEIKRTVREPQVCTLPPSRDE
LTKNQVSLTCLVKGFYPSDIAVEWESNGQPENNYKTTPPVLDSDGSFFLYSKLTVDKSRWQQ
GNVFSCSVMHEALHNHYTQKSLSLSPGKDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPCRDELTKNQVSLWCLVKGFYPSDIA
VEWESNGQPENNYKTTPPVLDSDGSFFLYSKLTVDKSRWQQGNVFSCSVMHEALHNHYTQKS
LSLSPGK
Domain arrangement:

| | | | | |
|---|---|---|---|---|
| A- | B- | Hinge- | D- | E |
| VL- | CH3- | *Hinge-* | **CH2-** | *CH3 (knob)* |

Mutations in domain B:
P343V; Y349C; 445P, 446G, 447K insertion
Mutations in domain E:
S354C, K366W
>BC44 Domain A **[SEQ ID NO:33]** 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRT
>BC44 Domain B **[SEQ ID NO:34]** 
VREPQVCTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
>BC44 Domain D **[SEQ ID NO:35]** 
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAK
>BC44 Domain E **[SEQ ID NO:36]** 
GQPREPQVYTLPPCRDELTKNQVSLWCLVKGFYPSDIAVEWESNGQPENNYKTTPPV
LDSDGSFFLYSKLTVDKSRWQQGNVFSCSVMHEALHNHYTQKSLSLSPGK
>BC28 bivalent chain 3 equivalent to SEQ ID NO:10
>BC28 bivalent chain 4 equivalent to SEQ ID NO:11
>BC28 1x2 chain 3 **[SEQ ID NO:37]** 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTPREPQ
VCTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGKGSGSGSEIVLTQSP
ATLSLSPGERATLSCRASQSVSSYLAWYQQKPGQAPRLLIYDASNRATGIPARFSGS
GSGTDFTLTISSLEPEDFAVYYCQQSSNWPRTFGQGTKVEIKRTVAAPSVFIFPPSD
EQLKSGTASVVCLLNNFYPREAKVQWKVDNALQSGNSQESVTEQDSKDSTYSLSSTL
TLSKADYEKHKVYACEVTHQGLSSPVTKSFNRGECDKTHTCPPCPAPELLGGPSVFL
FPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVCTLPPSRE
EMTKNQVSLSCAVKGFYPSDIAVEWESNGQPENNYKTTPPVLDSDGSFFLVSKLTVD
KSRWQQGNVFSCSVMHEALHNHYTQKSLSLSPGK
 Domain arrangement:

| | | | | | | | |
|---|---|---|---|---|---|---|---|
| R- | S- | linker- | H-I- | | Hinge- | J- | K- |
| VL- | CH3- | *linker-* | ***VL-*** | **CL-** | *Hinge-* | **CH2-** | *CH3 (hole)* |

Mutations in domain S:
   Y349C; 445P, 446G, 447K insertion
S1x amino acids linker insertion: GSGSGS
Mutations in domain K:
   Y349C, D356E, L358M, T366S, L368A, Y407V

>BC28 1x2 domain R **[SEQ ID NO:38]** 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRT
>BC28 1x2 domain S **[SEQ ID NO:39]** 
PREPQVCTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
>BC28 1x2 linker **[SEQ ID NO:40]**
GSGSGS
>BC28 1x2 domain H **[SEQ ID NO:41]**
>BC28 1x2 domain I **[SEQ ID NO:42]**
>BC28 1x2 domain J **[SEQ ID NO:43]** 
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAK
>BC28 1x2 domain K **[SEQ ID NO:44]** 
GQPREPQVCTLPPSREEMTKNQVSLSCAVKGFYPSDIAVEWESNGQPENNYKTTPPV
LDSDGSFFLVSKLTVDKSRWQQGNVFSCSVMHEALHNHYTQKSLSLSPGK
>BC28-1x1x1a chain 3 **[SEQ ID NO:45]** 
DIQMTQSPSSLSASVGDRVTITCRASQSVSSAVAWYQQKPGKAPKLLIYSASSLYSG
VPSRFSGSRSGTDFTLTISSLQPEDFATYYCQQRDSYLWTFGQGTKVEIKRTPREPQ
VYTLPPSRDELTKNQVSLKCLVKGFYPSDIAVEWESNGQPENNYKTTPPVLDSDGSF
FLYSKLTVDKSRWQQGNVFSCSVMHEALHNHYTQKSLSLSKSCGSGSGSEIVLTQSP
ATLSLSPGERATLSCRASQSVSSYLAWYQQKPGQAPRLLIYDASNRATGIPARFSGS
GSGTDFTLTISSLEPEDFAVYYCQQSSNWPRTFGQGTKVEIKRTVAAPSVFIFPPSD
EQLKSGTASVVCLLNNFYPREAKVQWKVDNALQSGNSQESVTEQDSKDSTYSLSSTL
TLSKADYEKHKVYACEVTHQGLSSPVTKSFNRGECDKTHTCPPCPAPELLGGPSVFL
FPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVCTLPPSRE
EMTKNQVSLSCAVKGFYPSDIAVEWESNGQPENNYKTTPPVLDSDGSFFLVSKLTVD
KSRWQQGNVFSCSVMHEALHNHYTQKSLSLSPGK
Domain arrangement:

| | | | | | | | |
|---|---|---|---|---|---|---|---|
| R- | S- | linker- | H-I- | | Hinge- | J- | K- |
| VL- | CH3- | *linker-* | ***VL-*** | **CL-** | *Hinge-* | **CH2-** | *CH3 (hole)* |

Mutations in domain S:
T366K; 445K, 446S, 447C insertion
Six amino acids linker insertion: GSGSGS
Mutations in domain K:
Y349C, D356E, L358M, T366S, L368A, Y407V
>BC28-1x1x1a domain R **[SEQ ID NO:46]** 
DIQMTQSPSSLSASVGDRVTITCRASQSVSSAVAWYQQKPGKAPKLLIYSASSLYSG
VPSRFSGSRSGTDFTLTISSLQPEDFATYYCQQRDSYLWTFGQGTKVEIKRT
>BC28-1x1x1a domain S **[SEQ ID NO:47]** 
PREPQVYTLPPSRDELTKNQVSLKCLVKGFYPSDIAVEWESNGQPENNYKTTPPVLD
SDGSFFLYSKLTVDKSRWQQGNVFSCSVMHEALHNHYTQKSLSLSKSC
>BC28-1x1x1a linker **[SEQ ID NO:48]**
GSGSGS
>BC28-1x1x1a domain H **[SEQ ID NO:49]**
>BC28-1x1x1a domain I **[SEQ ID NO:50]**
>BC28-1x1x1a domain J **[SEQ ID NO:51]** 
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAK
>BC28-1x1x1a domain K **[SEQ ID NO:52]** 
GQPREPQVCTLPPSREEMTKNQVSLSCAVKGFYPSDIAVEWESNGQPENNYKTTPPV
LDSDGSFFLVSKLTVDKSRWQQGNVFSCSVMHEALHNHYTQKSLSLSPGK
>hCTLA4-4.chain 2 **[SEQ ID NO:53]** 
EVQLVESGGGLVQPGGSLRLSCAASGFTFSTYYIHWVRQAPGKGLEWVAVIYPYTGF
TYYADSVKGRFTISADTSKNTAYLQMNSLRAEDTAVYYCARGEYTVLDYWGQGTLVT
VSSASPREPQVYTDPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSGEC
Domain arrangement:

| | |
|---|---|
| F- G | |
| VH- | CH3 |

Mutations in domain G
L351D, 445G, 446E, 447C insertion
>hCTLA4-4 domain F **[SEQ ID NO:54]** 
EVQLVESGGGLVQPGGSLRLSCAASGFTFSTYYIHWVRQAPGKGLEWVAVIYPYTGF
TYYADSVKGRFTISADTSKNTAYLQMNSLRAEDTAVYYCARGEYTVLDYWGQGTLVT
VSSAS
>hCTLA4-4 domain G **[SEQ ID NO:55]**

PREPQVYTDPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSGEC

### other Sequences:

**>Hinge:** DKTHTCPPCP **[SEQ ID NO:56]**
**>BC1-Polypeptide 1 Domain Junction:** IKRTPREP **[SEQ ID NO:57]**
**>BC15-Polypeptide 1 Domain Junction:** IKRTVREP **[SEQ ID NO:58]**
**>BC16-Polypeptide 1 Domain Junction:** IKRTREP **[SEQ ID NO:59]**
**>BC17-Polypeptide 1 Domain Junction:** IKRTVPREP **[SEQ ID NO:60]**
**>BC26-Polypeptide 1 Domain Junction:** IKRTVAEP **[SEQ ID NO:61]**
**>BC27-Polypeptide 1 Domain Junction:** IKRTVAPREP **[SEQ ID NO:62]**
**>BC1-Polypeptide 2 Domain Junction:** SSASPREP **[SEQ ID NO:63]**
**>BC13-Polypeptide 2 Domain Junction:** SSASTREP **[SEQ ID NO:64]**
**>BC14-Polypeptide 2 Domain Junction:** SSASTPREP **[SEQ ID NO:65]**
**>BC24-Polypeptide 2 Domain Junction:** SSASTKGEP **[SEQ ID NO:66]**
**>BC25-Polypeptide 2 Domain Junction:** SSASTKGREP **[SEQ ID NO:67]**

## Claims

1. An antibody construct, comprising:
a first and a second polypeptide chain, wherein:
(a) the first polypeptide chain comprises a domain A, a domain B, a domain D, and a domain E,
wherein the domains are arranged, from N-terminus to C-terminus, in a A-B-D-E orientation, and
wherein domain A has a VL amino acid sequence, domain B has a CH3 amino acid sequence, domain D has a CH2 amino acid sequence, domain E has a CH3 amino acid sequence;
(b) the second polypeptide chain comprises a domain F and a domain G,
wherein the domains are arranged, from N-terminus to C-terminus, in a F-G orientation, and
wherein domain F has a VH amino acid sequence and domain G has a CH3 amino acid sequence; and
(c) the first and the second polypeptides are associated through an interaction between the A and the F domains and an interaction between the B and the G domains to form the antibody construct;
the antibody construct, further comprising:
a third and a fourth polypeptide chain, wherein:
(d) the third polypeptide chain comprises a domain H, a domain I, a domain J, and a domain K,
wherein the domains are arranged, from N-terminus to C-terminus, in a H-I-J-K orientation, and
wherein domain H has a VL amino acid sequence, domain I has a CL amino acid sequence, domain J has a CH2 amino acid sequence, and K has a CH3 amino acid sequence;
(e) the fourth polypeptide chain comprises a domain L and a domain M,
wherein the domains are arranged, from N-terminus to C-terminus, in a L-M orientation, and
wherein domain L has a VH amino acid sequence and domain M has a CH1 amino acid sequence;
(f) the third and the fourth polypeptides are associated through an interaction between the H and the L domains and an interaction between the I and the M domains; and
(g) the first and the third polypeptides are associated through an interaction between the D and the J domains and an interaction between the E and the K domains to form the antibody construct.

2. The antibody construct of claim 1, wherein the amino acid sequences of the B and the G domains are identical, wherein the sequence is an endogenous CH3 sequence.

3. The antibody construct of claim 1 or 2, wherein the amino acid sequences of the E and K domains are identical, wherein the sequence is an endogenous CH3 sequence.

4. The antibody construct of claim 1 or 2, wherein the amino acid sequences of the E and K domains are different.

5. The antibody construct of any one of claims 1 and 3 to 4, wherein the amino acid sequences of the B and the G domains are different and separately comprise respectively orthogonal modifications in an endogenous CH3 sequence, wherein the B domain interacts with the G domain, and wherein neither the B domain nor the G domain significantly interacts with a CH3 domain lacking the orthogonal modification.

6. The antibody construct of claim 4 or claim 5, wherein the different sequences of the E and K domains separately comprise respectively orthogonal modifications in an endogenous CH3 sequence, wherein the E domain interacts with the K domain, and wherein neither the E domain nor the K domain significantly interacts with a CH3 domain lacking the orthogonal modification.

7. The antibody construct of claim 5 or claim 6, wherein the orthogonal modifications comprise mutations that generate engineered disulfide bridges between (i) domain B and G and/or (ii) domain E and K, optionally wherein the mutations that generate engineered disulfide bridges are a S354C mutation in one of the B domain and G domain, and a 349C in the other domain, G or B, and/or the mutations that generate engineered disulfide bridges are a S354C mutation in one of the E domain and K domain, and a 349C in the other domain, K or E.

8. The antibody construct of claim 5 or claim 6, wherein the orthogonal modifications comprise knob-in-hole mutations, optionally wherein the knob-in hole mutations are a T366W mutation in one of the B domain and G domain, and a T366S, L368A, and aY407V mutation in the other domain, G or B, and/or wherein the knob-in hole mutations are a T366W mutation in one of the E domain and K domain, and a T366S, L368A, and aY407V mutation in the other domain, K or E.

9. The antibody construct of claim 5 or claim 6, wherein the orthogonal modifications comprise charge-pair mutations, optionally wherein the charge-pair mutations are a T366K mutation in one of the B domain and G domain, and a L351D mutation in the other domain, G or B, and/or wherein the charge-pair mutations are a T366K mutation in one of the E domain and K domain, and a L351D mutation in the other domain, K or E.

10. The antibody construct of any one of claims 1 to 9, wherein the interaction between the A domain and the F domain form a first antigen binding site specific for a first antigen, and the interaction between the H domain and the L domain form a second antigen binding site specific for a second antigen.

11. The antibody construct of any one of claims 1 to 10, wherein the sequence that forms the junction between the A domain and the B domain is IKRTPREP or IKRTVREP.

12. The antibody construct of any one of claims 1 to 11, wherein the sequence that forms the junction between the F domain and the G domain is SSASPREP.

13. The antibody construct of any one of claims 1 to 12, wherein at least one CH3 amino acid sequence has a C-terminal tripeptide insertion connecting the CH3 amino acid sequence to a hinge amino acid sequence, wherein the tripeptide insertion is selected from the group consisting of PGK, KSC, and GEC.

14. The antibody construct of any one of claims 1 to 13, wherein the sequences are human sequences.

15. The antibody construct of any one of claims 1 to 14, wherein at least one CH3 amino acid sequence is an IgG sequence, optionally wherein the IgG sequences are IgG1 sequences.

16. The antibody construct of any one of claims 1 to 15, wherein at least one CH3 amino acid sequence has one or more isoallotype mutations, optionally wherein the isoallotype mutations are D356E and L358M.

17. The antibody construct of any one of claims 1 to 16, wherein the CL amino acid sequence is a Cₖₐₚₚₐ sequence or a C_{lambda} sequence.

## Patentansprüche

1. Antikörper-Konstrukt, das Folgendes umfasst:
eine erste und eine zweite Polypeptidkette, wobei:
(a) die erste Polypeptidkette eine Domäne A, eine Domäne B, eine Domäne D und eine Domäne E umfasst,
wobei die Domänen vom N-Terminus zum C-Terminus in der Ausrichtung A-B-D-E angeordnet sind und
wobei die Domäne A eine VL-Aminosäuresequenz aufweist, die Domäne B eine CH3-Aminosäuresequenz aufweist, die Domäne D eine CH2-Aminosäuresequenz aufweist und die Domäne E eine CH3-Aminosäuresequenz aufweist;
(b) die zweite Polypeptidkette eine Domäne F und eine Domäne G umfasst,
wobei die Domänen vom N-Terminus zum C-Terminus in der Ausrichtung F-G angeordnet sind und
wobei die Domäne F eine VH-Aminosäuresequenz aufweist und die Domäne G eine CH3-Aminosäuresequenz aufweist; und
(c) das erste und das zweite Polypeptid durch eine Wechselwirkung zwischen der A- und der F-Domäne und eine Wechselwirkung zwischen der B- und der G-Domäne assoziiert sind, um das Antikörper-Konstrukt zu bilden;
wobei das Antikörper-Konstrukt weiters Folgendes umfasst:
eine dritte und eine vierte Polypeptidkette, wobei:
(d) die dritte Polypeptidkette eine Domäne H, eine Domäne I, eine Domäne J und eine Domäne K umfasst,
wobei die Domänen vom N-Terminus zum C-Terminus in der Ausrichtung H-I-J-K angeordnet sind und
wobei die Domäne H eine VL-Aminosäuresequenz aufweist, die Domäne I eine CL-Aminosäuresequenz aufweist, die Domäne J eine CH2-Aminosäuresequenz aufweist und die Domäne K eine CH3-Aminosäuresequenz aufweist;
(e) die vierte Polypeptidkette eine Domäne L und eine Domäne M umfasst,
wobei die Domänen vom N-Terminus zum C-Terminus in der Ausrichtung L-M angeordnet sind und
wobei die Domäne L eine VH-Aminosäuresequenz aufweist und die Domäne M eine CH1-Aminosäuresequenz aufweist;
(f) das dritte und das vierte Polypeptid durch eine Wechselwirkung zwischen der H- und der L-Domäne und eine Wechselwirkung zwischen der I- und der M-Domäne assoziiert sind; und
(g) das erste und das dritte Polypeptid durch eine Wechselwirkung zwischen der D- und der J-Domäne und eine Wechselwirkung zwischen der E- und der K-Domäne assoziiert sind, um das Antikörper-Konstrukt zu bilden.

2. Antikörper-Konstrukt nach Anspruch 1, wobei die Aminosäuresequenzen der B- und der G-Domäne identisch sind, wobei die Sequenz eine endogene CH3-Sequenz ist.

3. Antikörper-Konstrukt nach Anspruch 1 oder 2, wobei die Aminosäuresequenzen der E- und der K-Domäne identisch sind, wobei die Sequenz eine endogene CH3-Sequenz ist.

4. Antikörper-Konstrukt nach Anspruch 1 oder 2, wobei die Aminosäuresequenzen der E- und der K-Domäne unterschiedlich sind.

5. Antikörper-Konstrukt nach einem der Ansprüche 1 und 3 bis 4, wobei die Aminosäuresequenzen der B- und der G-Domäne unterschiedlich sind und getrennt jeweils orthogonale Modifikationen in einer endogenen CH3-Sequenz umfassen, wobei die B-Domäne mit der G-Domäne wechselwirkt und wobei weder die B-Domäne, noch die G-Domäne signifikant mit einer CH3-Domäne wechselwirken, der die orthogonale Modifikation fehlt.

6. Antikörper-Konstrukt nach Anspruch 4 oder Anspruch 5, wobei die unterschiedlichen Sequenzen der E- und der K-Domäne getrennt jeweils orthogonale Modifikationen in einer endogenen CH3-Sequenz umfassen, wobei die E-Domäne mit der K-Domäne wechselwirkt und wobei weder die E-Domäne, noch die K-Domäne signifikant mit einer CH3-Domäne wechselwirken, der die orthogonale Modifikation fehlt.

7. Antikörper-Konstrukt nach Anspruch 5 oder Anspruch 6, wobei die orthogonalen Modifikationen Mutationen umfassen, die gentechnisch veränderte Disulfidbrücken zwischen (i) den Domänen B und G und/oder (ii) den Domänen E und K erzeugen, wobei die Mutationen, die veränderte Disulfidbrücken erzeugen, gegebenenfalls eine S354C-Mutation in einer aus der B-Domäne und der G-Domäne und eine 349C-Mutation in der jeweils anderen Domäne, G oder B, sind und/oder wobei die Mutationen, die veränderte Disulfidbrücken erzeugen, eine S354C-Mutation in einer aus der E-Domäne und der K-Domäne und eine 349C-Mutation in der jeweils anderen Domäne, K oder E, sind.

8. Antikörper-Konstrukt nach Anspruch 5 oder Anspruch 6, wobei die orthogonalen Modifikationen Knob-into-hole-Mutationen umfassen, wobei die Knob-into-hole-Mutationen gegebenenfalls eine T366W-Mutation in einer aus der B-Domäne und der G-Domäne und eine T366S-, eine L368A- und eine Y407V-Mutation in der jeweils anderen Domäne, G oder B, sind und/oder wobei die Knob-into-hole-Mutationen eine T366W-Mutation in einer aus der E-Domäne und der K-Domäne und eine T366S-, eine L368A- und eine Y407V-Mutation in der jeweils anderen Domäne, K oder E, sind.

9. Antikörper-Konstrukt nach Anspruch 5 oder Anspruch 6, wobei die orthogonalen Modifikationen Ladungspaar-Mutationen umfassen, wobei die Ladungspaar-Mutationen gegebenenfalls eine T366K-Mutation in einer aus der B-Domäne und der G-Domäne und eine L351D-Mutation in der jeweils anderen Domäne, G oder B, sind und/oder wobei die Ladungspaar-Mutationen eine T366K-Mutation in einer aus der E-Domäne und der K-Domäne und eine L351D-Mutation in der jeweils anderen Domäne, K oder E, sind.

10. Antikörper-Konstrukt nach einem der Ansprüche 1 bis 9, wobei die Wechselwirkung zwischen der A-Domäne und der F-Domäne eine erste Antigenbindungsstelle bildet, die für ein erstes Antigen spezifisch ist, und die Wechselwirkung zwischen der H-Domäne und der L-Domäne eine zweite Antigenbindungsstelle bildet, die für ein zweites Antigen spezifisch ist.

11. Antikörper-Konstrukt nach einem der Ansprüche 1 bis 10, wobei die Sequenz, welche die Verbindung zwischen der A-Domäne und der B-Domäne bildet, IKRTPREP oder IKRTVREP ist.

12. Antikörper-Konstrukt nach einem der Ansprüche 1 bis 11, wobei die Sequenz, welche die Verbindung zwischen der F-Domäne und der G-Domäne bildet, SSASPREP ist.

13. Antikörper-Konstrukt nach einem der Ansprüche 1 bis 12, wobei zumindest eine CH3-Aminosäuresequenz eine C-terminale Tripeptid-Insertion umfasst, welche die CH3-Aminosäuresequenz mit einer Gelenks-Aminosäuresequenz verbindet, wobei die Tripeptid-Insertion aus der aus PGK, KSC und GEC bestehenden Gruppe ausgewählt ist.

14. Antikörper-Konstrukt nach einem der Ansprüche 1 bis 13, wobei die Sequenzen menschliche Sequenzen sind.

15. Antikörper-Konstrukt nach einem der Ansprüche 1 bis 14, wobei zumindest eine CH3-Aminosäuresequenz eine IgG-Sequenz ist, wobei die IgG-Sequenzen gegebenenfalls IgG1-Sequenzen sind.

16. Antikörper-Konstrukt nach einem der Ansprüche 1 bis 15, wobei zumindest eine CH3-Aminosäuresequenz eine oder mehrere Isoallotyp-Mutationen aufweist, wobei die Isoallotyp-Mutationen gegebenenfalls D356E und L358M sind.

17. Antikörper-Konstrukt nach einem der Ansprüche 1 bis 16, wobei die CL-Aminosäuresequenz eine Cₖₐₚₚₐ-Sequenz oder eine C_{lambda}-Sequenz ist.

## Revendications

1. Construction d'anticorps, comprenant :
une première et une deuxième chaîne polypeptidique, dans laquelle :
(a) la première chaîne polypeptidique comprend un domaine A, un domaine B, un domaine D, et un domaine E,
dans laquelle les domaines sont disposés, de l'extrémité N-terminale à l'extrémité C-terminale, selon une orientation A-B-D-E, et
dans laquelle le domaine A a une séquence d'acides aminés VL, le domaine B a une séquence d'acides aminés CH3, le domaine D a une séquence d'acides aminés CH2, le domaine E a une séquence d'acides aminés CH3 ;
(b) la deuxième chaîne polypeptidique comprend un domaine F et un domaine G,
dans laquelle les domaines sont disposés, de l'extrémité N-terminale à l'extrémité C-terminale, selon une orientation F-G, et
dans laquelle le domaine F a une séquence d'acides aminés VH et le domaine G a une séquence d'acides aminés CH3 ; et
(c) les premier et deuxième polypeptides sont associés par une interaction entre les domaines A et F et une interaction entre les domaines B et G pour former la construction d'anticorps ;
la construction d'anticorps, comprenant en outre :
une troisième et une quatrième chaîne polypeptidique, dans laquelle :
(d) la troisième chaîne polypeptidique comprend un domaine H, un domaine I, un domaine J, et un domaine K,
dans laquelle les domaines sont disposés, de l'extrémité N-terminale à l'extrémité C-terminale, selon une orientation H-I-J-K, et
dans laquelle le domaine H a une séquence d'acides aminés VL, le domaine I a une séquence d'acides aminés CL, le domaine J a une séquence d'acides aminés CH2, et K a une séquence d'acides aminés CH3 ;
(e) la quatrième chaîne polypeptidique comprend un domaine L et un domaine M,
dans laquelle les domaines sont disposés, de l'extrémité N-terminale à l'extrémité C-terminale, selon une orientation L-M, et
dans laquelle le domaine L a une séquence d'acides aminés VH et le domaine M a une séquence d'acides aminés CH1 ;
(f) les troisième et quatrième polypeptides sont associés par une interaction entre les domaines H et L et une interaction entre les domaines I et M ; et
(g) les premier et troisième polypeptides sont associés par une interaction entre les domaines D et J et une interaction entre les domaines E et K pour former la construction d'anticorps.

2. Construction d'anticorps selon la revendication 1, dans laquelle les séquences d'acides aminés des domaines B et G sont identiques, dans laquelle la séquence est une séquence CH3 endogène.

3. Construction d'anticorps selon la revendication 1 ou 2, dans laquelle les séquences d'acides aminés des domaines E et K sont identiques, dans laquelle la séquence est une séquence CH3 endogène.

4. Construction d'anticorps selon l'une quelconque des revendications 1 à 2, dans laquelle les séquences d'acides aminés des domaines E et K sont différentes.

5. Construction d'anticorps selon l'une quelconque des revendications 1 et 3 à 4, dans laquelle les séquences d'acides aminés des domaines B et G sont différentes et comprennent séparément des modifications respectivement orthogonales dans une séquence CH3 endogène, dans laquelle le domaine B interagit avec le domaine G, et dans laquelle ni le domaine B ni le domaine G n'interagit de manière significative avec un domaine CH3 dépourvu de la modification orthogonale.

6. Construction d'anticorps selon la revendication 4 ou la revendication 5, dans laquelle les différentes séquences des domaines E et K comprennent séparément des modifications respectivement orthogonales dans une séquence CH3 endogène, dans laquelle le domaine E interagit avec le domaine K, et dans laquelle ni le domaine E ni le domaine K n'interagit de manière significative avec un domaine CH3 dépourvu de la modification orthogonale.

7. Construction d'anticorps selon la revendication 5 ou la revendication 6, dans laquelle les modifications orthogonales comprennent des mutations qui génèrent des ponts disulfure modifiés entre (i) les domaines B et G et/ou (ii) les domaines E et K, facultativement dans laquelle les mutations qui génèrent des ponts disulfure modifiés sont une mutation S354C dans l'un des domaines B et G, et une mutation 349C dans l'autre domaine, G ou B, et/ou les mutations qui génèrent des ponts disulfures modifiés sont une mutation S354C dans l'un des domaines E et K, et une mutation 349C dans l'autre domaine, K ou E.

8. Construction d'anticorps selon la revendication 5 ou la revendication 6, dans laquelle les modifications orthogonales comprennent des mutations de type nœud dans la cavité, facultativement dans laquelle les mutations de type nœud dans la cavité sont une mutation T366W dans l'un des domaines B et G, et une mutation T366S, L368A et Y407V dans l'autre domaine, G ou B, et/ou dans laquelle les mutations de type nœud dans la cavité sont une mutation T366W dans l'un des domaines E et K, et une mutation T366S, L368A et Y407V dans l'autre domaine, K ou E.

9. Construction d'anticorps selon la revendication 5 ou la revendication 6, dans laquelle les modifications orthogonales comprennent des mutations par paire de charges, facultativement dans laquelle les mutations par paire de charges sont une mutation T366K dans l'un des domaines B et G, et une mutation L351D dans l'autre domaine, G ou B, et/ou dans laquelle les mutations par paire de charges sont une mutation T366K dans l'un des domaines E et K, et une mutation L351D dans l'autre domaine, K ou E.

10. Construction d'anticorps selon l'une quelconque des revendications 1 à 9, dans laquelle l'interaction entre le domaine A et le domaine F forme un premier site de liaison à l'antigène spécifique à un premier antigène, et l'interaction entre le domaine H et le domaine L forme un second site de liaison à l'antigène spécifique à un second antigène.

11. Construction d'anticorps selon l'une quelconque des revendications 1 à 10, dans laquelle la séquence qui forme la jonction entre le domaine A et le domaine B est IKRTPREP ou IKRTVREP.

12. Construction d'anticorps selon l'une quelconque des revendications 1 à 11, dans laquelle la séquence qui forme la jonction entre le domaine F et le domaine G est SSASPREP.

13. Construction d'anticorps selon l'une quelconque des revendications 1 à 12, dans laquelle au moins une séquence d'acides aminés CH3 a une insertion tripeptidique C-terminale reliant la séquence d'acides aminés CH3 à une séquence d'acides aminés charnière, dans laquelle l'insertion tripeptidique est choisie parmi le groupe constitué de PGK, KSC, et GEC.

14. Construction d'anticorps selon l'une quelconque des revendications 1 à 13, dans laquelle les séquences sont des séquences humaines.

15. Construction d'anticorps selon l'une quelconque des revendications 1 à 14, dans laquelle au moins une séquence d'acides aminés CH3 est une séquence IgG, facultativement dans laquelle les séquences IgG sont des séquences IgG1.

16. Construction d'anticorps selon l'une quelconque des revendications 1 à 15, dans laquelle au moins une séquence d'acides aminés CH3 a une ou plusieurs mutations d'isoallotype, facultativement dans laquelle les mutations d'isoallotype sont D356E et L358M.

17. Construction d'anticorps selon l'une quelconque des revendications 1 à 16, dans laquelle la séquence d'acides aminés CL est une séquence Cₖₐₚₚₐ ou une séquence C_{lambda}.
